# EUROPEAN PATENT APPLICATION

(11) **EP 2 025 678 A1**
(43) Date of publication of application: **18.02.2009**
(21) Application number: 07016154.2
(22) Date of filing: 17.08.2007
(51) Int. Cl.: C07D 487/04, A61K 31/519, A61P 35/00

(54) **Pyrazolo[3,4-d]pyrimidine compounds and their use as modulators of protein kinase**

(71) Applicant: Oncalis AG, 8952 Schlieren (CH)
(72) Inventor: Lüthi, Urs, 8330 Pfäffikon (CH); Berset, Catherine Elisabeth, 5210 Windisch (CH); Schmid, Gerhard, 8803 Rüschlikon (CH); Capraro, Hans-Georg, 4310 Rheinfelden (CH); Traxler, Peter Martin, 4124 Schönenbuch (CH)
(74) Representative: Grimm, Siegfried

(57) **Abstract**

The invention relates to pyrazolo[3,4-d]pyrimidine compounds of formual I and its salts 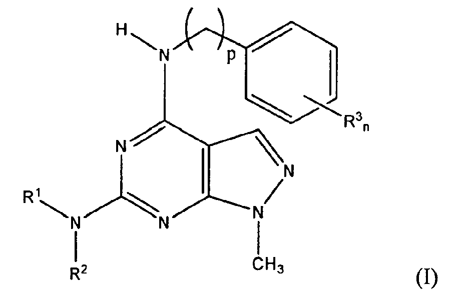 wherein the substitents are as defined in the specification, their use for the treatment of protein kinase modulation responsive diseases or in the manufacture of pharmaceutical preparations useful in the treatment of said diseases, pharmaceutical preparations, especially useful against said diseases, comprising said compounds and a pharmaceutically acceptable carrier, said compounds for use in the treatment of the animal or human body, especially against said diseases, methods of treatment of the animal or human body comprising administering said compounds to an animal or human, and processes for the manufacture of said compounds.

## Description

The invention relates to pyrazolo[3,4-d]pyrimidine compounds, their use for the treatment of protein kinase modulation responsive diseases or in the manufacture of pharmaceutical preparations useful in the treatment of said diseases, pharmaceutical preparations, especially useful against said diseases, comprising said compounds and a pharmaceutically acceptable carrier, said compounds for use in the treatment of the animal or human body, especially against said diseases, methods of treatment of the animal or human body comprising administering said compounds to an animal or human, and processes for the manufacture of said compounds, where in each case where compounds are mentioned they can be present as such and/or in the form of (preferably pharmaceutically acceptable) salts.

### Background of the invention

By the term "protein kinases", a class of enzymatically active proteins is defined where receptor-type kinases and nonreceptor-type kinases can be distinguished, as well as tyrosine and serine/threonine kinases. Regarding their localization, nuclear, cytoplasmic and membrane-associated kinases can be distinguished. Many membrane-associated tyrosine kinases are at the same time receptors for growth factors.

Regarding their catalytic activity, protein kinases (PKs) are enzymes which catalyze the phosphorylation of specific serine, threonine or tyrosine residues in cellular proteins. This post-translational modification of substrate proteins usually works as molecular switch, representing a step in regulating cell proliferation, activation and/or differentiation. Aberrant or excessive or more generally inappropriate PK activity has been observed in several disease states including benign and malignant proliferative disorders. In many cases, it has been possible to treat diseases in vitro and in many cases in vivo, such as proliferative disorders, by making use of PK inhibitors.

Over the past years, basic roles for Eph receptor tyrosine kinases and their ligands, the ephrins, have been understood. Several different Eph receptors are catalogued and grouped into EphA or EphB subclasses, based on their affinity for ligands. At least eight ephrins were identified which are membrane proteins, either of the glycerophosphatidylinositol (GPI)- linked (ephrinA) or transmembrane (ephrinB) type. Signaling between Eph receptors and their ligands appears to be restricted to sites of direct cell-cell contact. The result of contact is the induction of reciprocal bidirectional events between cells. The expression of ephrins and their receptors at certain locations is considered to have impact on tissue patterning and the organizing of spatially very restricted cell loci. Included among the specific effects is the modification of cell migration, adhesion and somite formation.

EphB4 (also named HTK) and its ligand, ephrinB2 (HTKL), play important roles in establishing and determining vascular networks. On the venous epithelium, EphB4 is expressed specifically, while, during early stages of vascular development, ephrinB2 is specifically and reciprocally expressed on arterial endothelial cells. Dysfunctional genes lead to embryonic lethality in mice, and the embryos show identical defects in forming capillary connections in case of either defect ephrinB2 and EphB4. Both are expressed at the first site of hemato- poiesis and vascular development during embryogenesis. An essential role for proper hematopoietic, endothelial, hemangioblast and primitive mesoderm development has been established. EphB4 deficiency results in an alteration in the mesodermal differentiation outcome of embryonic stem cells. Ectopic expression of EphB4 in mammary tissue results in disordered architecture, abnormal tissue function and a predisposition to malignancy (see e.g. N. Munarini et al., J. Cell. Sci. V\5, 25-37 (2002)). From these and other data, it has been concluded that inadequate EphB4 expression may be involved in the formation of malignancies and thus that inhibition of EphB4 can be expected to be a tool to combat malignancies, e.g. cancer and the like.

VEGFRs (vascular endothelial growth factor receptors) are known to be involved in the control of the onset of angiogenesis. As especially solid tumors depend on good blood supply, inhibition of VEGFRs and thus angiogenesis is under clinical investigation in the treatment of such tumors, showing promising results. VEGF is also a major player in leukemias and lymphomas and highly expressed in a variety of solid malignant tumors, correlating well with malignant disease progression. Examples of tumor diseases with VEGFR-2 (KDR) expression are lung carcinomas, breast carcinomas, Non Hodgkin' s lymphomas, ovarian carcinoma, pancreatic cancer, malignant pleural mesothelioma and melanoma. In addition to its angiogenic activity, the ligand of VEGFR, VEGF, may promote tumor growth by direct pro-survival effects in tumor cells. Various other diseases are associated with deregulated angiogenesis, e.g. as mentioned below.

This leads to the problem of the present invention: In view of the large number of protein kinase inhibitors and the multitude of proliferative and other protein kinase-related diseases, as well as in view of the development of resistance against certain therapeutics, there is an ever-existing need to provide new classes of compounds that are useful as protein kinase inhibitors and thus in the treatment of these protein tyrosine kinase, such as serine/threonine and/or preferably PTK (protein tyrosine kinase) related diseases. What is required are new classes of pharmaceutically advantageous protein kinase inhibitors, especially PTK inhibiting compounds, especially with advantageous properties, such as high affinity and/or selectivity for limited groups of or singular protein kinases, activity also where resistance against different classes of compounds has been developed, a useful affinity profile against certain groups of kinases or the like.

WO 2005/082865 describes certain bicyclic pyrimidine compounds and their use as CCR4 function controlling agents (e.g. treatment of inflammatory diseases, allergies). The specifically described compounds structurally differ from the compounds of the present invention; further no specific indication to the use as kinase inhibitors is provided in this document.

WO 2007/062805 describes certain bicyclic pyrimidine compounds and their use as kinase inhibitors. Allthough selected compounds of this document show good results in specific test assays, they also show disadvantages. It is therefore a need to provide further compounds with improved properties.

### General description of the invention

It has been found now surprisingly that a number of protein kinases which can be involved in signal transmission mediated by trophic factors and in the manifestation of diseases that involve the activity of protein kinases, e.g. in proliferative (e.g. tumor) growth, especially as representative examples for protein tyrosine kinases abl kinase, especially v-abl or c-abl kinase, kinases from the family of the src kinases, especially c-src kinase, RET-receptor kinase or Ephrin receptor kinases, e.g. EphB2 kinase, EphB4 kinase or related kinases, and/or b-raf (V599E), further EGF receptor kinase or other kinases of the EGF family, for example HER- 1 or c-erbB2 kinase (HER-2) and/or VEG F-receptor kinase (e.g. KDR and Flt-1), yet further Flt-3, lck, fyn, c-erbB3 kinase, c-erbB4 kinase; members of the family of the PDGF-receptor tyrosine protein kinases, for example PDGF-receptor kinase, CSF-1 receptor kinase, Kit-receptor kinase (c-Kit), FGF-receptor kinase, e.g. FGF-R1, FGF-R2, FGF-R3, FGF-R4, c- Raf, casein kinases (CK-1, CK-2, G-CK), Pak, ALK, ZAP70, Jak1, Jak2, AxI, Cdk1 , cdk4, cdk5, Met, FAK, Pyk2, Syk, Tie-2, insulin receptor kinase (Ins-R), the receptor kinase of the insulin-like growth factor (IGF-1 kinase), and/or further serine/threonine kinases, for example protein kinase C (PK-C), PK-B, EK-B or cdc kinases, such as CDK1 , as well as (e.g. constitutively activated) mutated forms of any one or more of these (e.g. Bcr-Abl, RET/MEN2A, RET/MEN2B, RET/PTC 1-9 or b-raf (V599E))can be inhibited by a compound according to the invention. All these and other protein kinases play a part in growth regulation and transformation in mammalian cells, including human cells.

In view of these activities, the compounds of the invention can be used for the treatment of protein kinase modulation responsive diseases, such as diseases related to especially aberrant (e.g. unregulated, deregulated or constitutive or the like) or excessive activity of such types of kinases, especially those mentioned.

### Detailed description of the invention

The invention, in a first embodiment, relates to a compound of the formula I 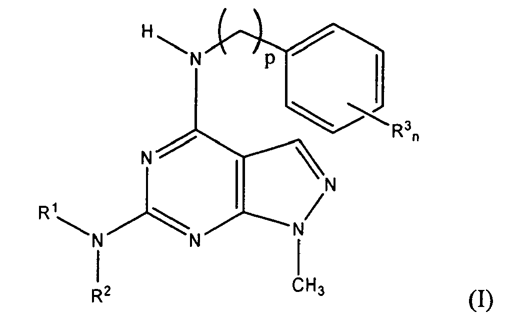 wherein
- R1: represents unsubstituted or substituted alkyl, unsubstituted or substituted cycloalkyl, unsubstituted or substituted aryl, unsubstituted or substituted heterocyclyl and
- R2: represents hydrogen or a substituent as defined for R1; or
- R1: and R2 represents together with the nitrogen to which theay are bound unsubstituted or substituted heterocyclyl
and
- R3: represents unsubstituted or substituted lower alkyl, unsubstituted or substituted cycloalkyl, unsubstituted or substituted lower alkoxy, halo, cyano
- n: represents 0, 1, 2 or 3
- p: represents 0 or 1. or a (preferably pharmaceutically acceptable) salt thereof.

In formula I, the following significances are preferred independently, collectively or in any combination or sub-combination thereof.
- n: preferably represents 1, 2, 3.
- n: particular preferably represents 1.
- p: preferably represents 0.
- R1: preferably represents unsubstituted or substituted lower alkyl, the substituents being selected from the group consisting of lower alkoxy, lower alkylamino, lower dialkylamino, halo, cyano, cycloalkyl, unsubstituted heterocyclyl; unsubstituted or substituted cycloalkyl the substituents being selected from the group consisting of lower alkoxy, lower alkylamino, lower dialkylamino, halo, cyano,; unsubstituted or substituted phenyl, the substituents being selected from the group consisiting of lower alkyl, lower alkoxy, lower alkylamino, lower dialkylamino, halo, cyano, cylcoalkyl; unsubstituted lower alkenyl; unsubstituted or substituted heterocyclyl, the substituents being selected from the group consisting of lower alkoxy, lower alkylamino, lower dialkylamino, halo, cyano, cycloalkyl, unsubstituted heterocyclyl.
- R1: preferably represents unsubstituted or substituted methyl, ethyl, n-propyl, iso-propyl, n-butyl, iso- butyl, sec-butyl, tert-butyl, the substituents being selected from the group consisting of lower methoxy, ethoxy, methylamino, ethylamino, dimethylamino, diethylamin, fluoro, chloro, cyano, cyclopropyl, cyclopentyl, cyclohexyl, pyridino, piperidino, piperazino, N-methylpiperazino, morpholino, pyrrolo, pyrrolidino, furano, tetrahydrofurano; cyclopropyl, cyclopentyl, cyclohexyl; unsubstituted or one- or twofold substituted phenyl, the substituents being selected from the group consisting of methyl, ethyl, n-propyl, iso-propyl, n-butyl, iso-butyl, sec-butyl, tert-butyl, methoxy, ethoxy, dimethylamino fluoro, chloro, cyano, cyclopropyl, -pentyl, -hexyl.
- R2: preferably represents hydrogen.
- R1: and R2 together with the nitrogen to which they are bound preferably represent unsubstituted or substituted monocyclic heterocyclyl having 5 to 7 ring atoms, the substituents being selected from the group consisting of unsubstituted or substituted lower alkyl, the substituents being selected from the group of hydroxy, halogen or aryl; unsubstituted or substituted cycloalkyl, the substituents being selected from the group of hydroxy, halogen. lower alkyl or aryl; unsubstituted heterocyclyl; substituted or unsubstituted heterocyclylcarbonyl, the substituents being selected from the group of hydroxy, halogen, lower alkyl or aryl.
- R1: and R2 together with the nitrogen to which they are bound preferably represent an unsubstituted or substituted heterocycle, the hetereocycle being selected from the group consisting of pyridyl, piperidyl, pyrazinyl, piperazinyl, morpholinyl, pyrrolyl, pyrrolidinyl, pyrazolyl, imidazolyl, triazolyl, the substituents being selected from the group consisting of unsubstituted lower alkyl, cycloalkyl, lower hydroxyalkyl, phenyl, benzyl, pyridyl, piperidyl, pyrazinyl, piperazinyl, morpholinyl, pyrrolyl, pyrrolidinyl, pyrazolyl, imidazolyl, triazolyl, furanyl, furanylcarbonyl.
- R3: preferably represents unsubstituted lower alkyl, unsubstituted lower alkoxy, halo, cyano.
- R3: particular preferably represents methyl, ethyl, iso- propyl, n-porpyl, tert.-butyl, iso-butyl, n-butyl, methoxy, ethoxy, iso-propoxy, n-porpoxy, tert.- butoxy, iso-butoxy, n-butoxy, fluoro, chloro.

In a further preferred embodiment, at least one R3 is in the ortho-position.

In view of the close relationship between the compounds in free form and in the form of their salts, including those salts that can be used as intermediates, for example in the purification or identification of the compounds or salts thereof, any reference to "compounds"(including also starting materials and "intermediates") hereinbefore and hereinafter, especially to the compound(s) of the formula I, is to be understood as referring also to one or more salts thereof or a mixture of a free compound and one or more salts thereof, each of which is intended to include also any solvate, metabolic precursor such as ester or amide of the compound of formula I, or salt of any one or more of these, as appropriate and expedient and if not explicitly mentioned otherwise. Different crystal forms and solvates may be obtainable and then are also included.

Where the plural form is used for compounds, salts, pharmaceutical preparations, diseases, disorders and the like, this is intended to mean to include also a single compound, salt, pharmaceutical preparation, disease or the like, where "a" or "an" is used, this means to refer to the indefinite article or preferably to "one".

In some cases, a compound of the present invention may comprise one or more chiral centers in substitutents or show other asymmetry (leading to enantiomers) or may otherwise be able to exist in the form of more than one stereoisomer, e.g. due more than one chiral centers or more than one other type of asymmetry or due to rings or double bonds that allow for Z/E (or cis-trans) isomerism (diastereomers). The present inventions includes both mixtures of two or more such isomers, such as mixtures of enantiomers, especially racemates, as well as preferably purified isomers, especially purified enantiomers or enantiomerically enriched mixtures.

The general terms or symbols used hereinbefore and hereinafter preferably have, within the context of this disclosure, the following meanings, unless otherwise indicated:
The term "lower" or **C1-C7-alkyl** defines a moiety with 1-7, especially 1-4, carbon atoms, said moiety being branched (one or more times) or straight-chained. Lower or C1-C7 alkyl, for example, is n-pentyl, n-hexyl or n-heptyl or preferably C1-C4-alkyl, especially as methyl, ethyl, n-propyl, iso-propyl, n-butyl, iso-butyl, sec-butyl, tert-butyl. In a similar manner, this term is used in connection with "alkoxy", "alkylthio", "alkylcarbonyl", "alkylamino" and the like. In case of lower alkenyl or lower alkynyl, lower means preferably "C2-C7" -, more preferably "C2-C4-"

**Halo** or halogen is preferably fluoro, chloro, bromo or iodo, most preferably fluoro, chloro or bromo, even preferably, fluoro or chloro.

**Unsubstituted or substituted alkyl** is preferably C1- to C20-alkyl, more preferably lower alkyl, e.g. methyl, ethyl or propyl, that can be linear or branched one or more times (provided the number of carbon atoms allows this) and that is unsubstituted or substituted by one or more, preferably up to three, substitutents independently selected from the group consisting of unsubstituted or substituted heterocyclyl as described below, especially piperidino, piperazino, morpholino, pyrrolidino, pyrrolo, furano the substituents being selected from the group consisting of lower alkyl, lower alkoxy halo, cyano, unsubstituted heterocyclyl (as described below), unsubstituted or substituted cycloalkyl as described below, unsubstituted or substituted aryl as defined below, especially phenyl or naphthyl; lower alkenyl, lower alkynyl, halo, hydroxy, lower alkoxy, lower-alkoxy-lower alkoxy, (lower-alkoxy)-lower alkoxy-lower alkoxy, phenoxy, naphthyloxy, phenyl- or naphthyl-lower alkoxy, such as benzyloxy; amino-lower alkoxy, lower-alkanoyloxy, benzoyloxy, naphthoyloxy, nitro, cyano, carboxy, lower alkoxy carbonyl, e.g. methoxy carbonyl, n-propoxy carbonyl, iso-propoxy carbonyl or tert-butoxycarbonyl; phenyl- or naphthyl-lower alkoxycarbonyl, such as benzyloxycarbonyl; lower alkanoyl, such as acetyl, benzoyl, naphthoyl, carbamoyl, N-mono- or N,N-disubstituted carbamoyl, such as N-mono- or N,N-di-substituted carbamoyl wherein the substitutents are selected from lower alkyl and hydroxy-lower alkyl; amidino, guanidino, ureido, mercapto, lower alkylthio, phenyl- or naphthylthio, phenyl- or naphthyl-lower alkylthio, lower alkyl-phenylthio, lower alkyl-naphthylthio, halogen-lower alkylmercapto, lower alkylsulfinyl, phenyl- or naphthyl-sulfinyl, phenyl- or naphthyl-lower alkylsulfinyl, lower alkyl-phenylsulfinyl, lower alkyl-napthylsulfinyl, sulfo, lower alkanesulfonyl, phenyl- or naphthyl-sulfonyl, phenyl- or naphthyl-lower alkylsulfonyl, alkylphenylsulfonyl, halogen-lower alkylsulfonyl, such as trifluoromethanesulfonyl; sulfon-amido, benzosulfonamido, amino, N-mono- or N,N-di-[lower alkyl, phenyl and/or phenyl-lower alkyl)-amino, such as N,N-dimethylamino, N,N-diethylamino, 3-[N-(N, N-dimethylamino)- propylamino, 2-[N-(N, N-dimethylamino)-ethylamino or N-(N,N-dimethylamino)-methylamino; where each phenyl or naphthyl (also in phenoxy or naphthoxy) mentioned above as sub- stituent or part of a substituent of substituted alkyl is itself unsubstituted or substituted by one or more, e.g. up to three, preferably 1 or 2, substituents independently selected from halo, especially fluoro, chloro, bromo or iodo, halo-lower alkyl, such as trifluoromethyl, hydroxy, lower alkoxy, amino, N-mono- or N,N-di-(lower alkyl, phenyl, naphthyl, phenyl-lower alkyl and/or naphthyl-lower alkyl)-amino, nitro, carboxy, lower-alkoxycarbonyl carbamoyl, cyano and/or sulfamoyl.

**Unsubstituted or substituted aryl** is preferably an unsaturated carbocyclic system of not more than 20 carbon atoms, especially not more than 16 carbon atoms, in particular phenyl, is preferably mono-, bi- or tri-cyclic, which is unsubstituted or substituted preferably by one or more, preferably up to three, e.g. one or two substituents independently selected from the group consisting of halo, hydroxy, nitro, lower alkyl (e.g. methyl), phenyl, naphthyl, phenyl- or naphthyl-lower alkyl, (e.g. benzyl); hydroxy-lower alkyl, such as hydroxymethyl; lower-alkoxy-lower alkyl, (lower- alkoxy)-lower alkoxy-lower alkyl, lower alkanoyl-lower alkyl, halo-lower alkyl, such as trifluoromethyl, cyano-lower alkyl; phenoxy- or naphtyloxy-lower alkyl, phenyl- or naphthyl-lower alkoxy-lower alkyl, such as benzyloxylower alkyl; lower alkoxy-carbonyloxy-lower alkyl, such as tert- butoxycarbonyloxy-lower alkyl; phenyl- or naphthyl-lower alkoxycarbonyloxy-lower alkyl, such as benzyloxycarbonyloxy-lower alkyl; lower alkenyl, lower alkynyl, lower alkanoyl, such as acetyl; lower alkoxy, such as methoxy, lower-alkoxy-lower alkoxy, (lower-alkoxy)-lower alkoxy-lower alkoxy, phenoxy, naphthyloxy, phenyl- or naphthyl- lower alkoxy, such as benzyloxy; amino-lower alkoxy, lower-alkanoyloxy, benzoyloxy, naphthoyloxy, amino, mono-, di- or tri-substituted (in the latter case quaternary and positively charged) amino wherein the amino substituents are independently selected from lower alkyl, lower alkanoyl, phenyl, naphthyl, phenyl- and naphthyl-lower alkyl; cyano, carboxy, lower alkoxy carbonyl, e.g. methoxy carbonyl, n-propoxy carbonyl, iso-propoxy car- bonyl or tert-butoxycarbonyl; phenyl- or naphthyl-lower alkoxycarbonyl, such as benzyloxy-carbonyl; benzoyl, naphthoyl, carbamoyl, N-mono- or N,N-disubstituted carbamoyl, such as N-mono- or N,N-di-substituted carbamoyl wherein the substitutents are selected from lower alkyl and hydroxy-lower alkyl; amidino, guanidino, ureido, mercapto, lower alkylthio, phenyl- or naphthylthio, phenyl- or naphthyl-lower alkylthio, lower alkyl-phenylthio, lower alkyl-naphthylthio, halogen-lower alkylmercapto, lower alkylsulfinyl, phenyl- or naphthyl-sulfinyl, phenyl- or naphthyl-lower alkylsulfinyl, lower alkyl-phenylsulfinyl, lower alkyl-napthylsulfinyl, sulfo, lower alkanesulfonyl, phenyl- or naphthyl-sulfonyl, phenyl- or naphthyl-lower alkylsulfonyl, alkylphenylsulfonyl, halogen-lower alkylsulfonyl, such as trifluoromethanesulfonyl; sulfon-amido, benzosulfonamido, pyrrolidino, piperidino, piperidino substituted by amino or N-mono- or N,N-di-[lower alkyl, phenyl and/or phenyl-lower alkyl)-amino, unsubstituted or N-lower alkyl substituted piperidinyl bound via a ring carbon atom, such as 1-isopropyl-piperidin-4-yl, piperazino, lower alkylpiperazino, such as 4-(methyl, ethyl or isopropyl)-piperazino, morpholino or thiomorpholino, furano; where each phenyl or naphthyl (also in phenoxy or naphthoxy) mentioned above as substitutent or part of a substituent of substituted aryl is itself unsubstituted or substituted by one or more, e.g. up to three, preferably 1 or 2, substituents independently selected from halo, especially fluoro, chloro, bromo or iodo, halo-lower alkyl, such as trifluoromethyl, hydroxy, lower alkoxy, amino, N-mono- or N,N-di-(lower alkyl, phenyl, naphthyl, phenyl-lower alkyl and/or naphthyl-lower alkyl)amino, nitro, carboxy, lower-alkoxycarbonyl carbamoyl, cyano and/or sulfamoyl. Unsubstituted or substituted aryl, especially as R1 and/ or R2 in formula I is preferably phenyl that is unsubstituted or substituted by halo, hydroxyl, more preferably by lower alkoxy, nitro, amino, N-mono-, N,N-di- or N,N,N-tri-(lower alkyl, phenyl and/or phenyl-lower alkyl)-amino (the latter corresponding to a quaternary amino = quaternary ammonio), pyrrolidino, piperidino, piperidino substituted by amino or N-mono- or N,N-di-[lower alkyl, phenyl and/or phenyl-lower alkyl)-amino, unsubstituted or N-lower alkyl substituted piperidinyl bound via a ring carbon atom, such as 1-isopropyl-piperidin-4-yl, piperazino, lower alkylpiperazino, such as 4-(methyl, ethyl or isopropyl)-piperazino, morpholino or thiomorpholino.

**Unsubstituted or substituted heterocyclyl** is preferably a heterocyclic radical that is unsaturated, saturated or partially saturated and is preferably a monocyclic or in a broader aspect of the invention bicyclic or tricyclic ring; and has 3 to 24, more preferably 4 to 16, most preferably 4 to 10 ring atoms; wherein one or more, preferably one to four, especially one or two carbon ring atoms are replaced by a heteroatom selected from the group consisting of nitrogen, oxygen and sulfur, the bonding ring preferably having 4 to 12, especially 5 to 7 ring atoms; which heterocyclic radical (heterocyclyl) is unsubstituted or substituted by one or more, especially 1 to 3, substituents independently selected from the group consisting of the substituents defined above under "substituted aryl". In particular heterocyclyl is a heterocyclyl radical selected from the group consisting of oxiranyl, azirinyl, aziridinyl, 1 ,2-oxathiolanyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidaz- olidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpho- linyl, thiomorpholinyl, (S-oxo or S,S-dioxo)-thiomorpholinyl, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydro-quinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, beta- carbolinyl, phenanthridinyl, acridinyl, perimidinyl, phenanthrolinyl, furazanyl, phenazinyl, phenothiazinyl, phenoxazinyl, chromenyl, isochromanyl and chromanyl. Each of these radicals being preferably unsubstituted or substituted by one to two radicals selected from the group consisting of lower alkyl, especially methyl or tert-butyl, lower alkoxy, especially methoxy, and halo, especially bromo or chloro.

In **unsubstituted or substituted cycloalkyl,** cycloalkyl is preferably a saturated mono- or bicyclic hydrocarbon group with 3 to 16, more preferably 3 to 9 ring carbon atoms, e.g. cyclopropyl, cyclobutyl, cyclopentyl, cyclohexyl, cycloheptyl or cyclooctyl, and is substituted by one or more, preferably one to three, substitutents independently selected from those described for substituted aryl or is (preferably) unsubstituted.

**Salts** are especially the pharmaceutically acceptable salts of compounds of formula I. They can be formed where salt forming groups, such as basic or acidic groups, are present that can exist in dissociated form at least partially, e.g. in a pH range from 4 to 10 in aqueous environment, or can be isolated especially in solid form, or where charged groups (e.g. quaternary ammonium) are present - in the latter case acylate salts are formed with anions of organic or inorganic acids (e.g. as defined in the next paragraph).

Such salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, lactic acid, fumaric acid, succinic acid, citric acid, amino acids, such as glutamic acid or as- partic acid, maleic acid, hydroxymaleic acid, methylmaleic acid, benzoic acid, methane- or ethane-sulfonic acid, ethane- 1,2-disulfonic acid, benzenesulfonic acid, 2-naphthalenesulfonic acid, 1 ,5-naphthalene-disulfonic acid, N-cyclohexylsulfamic acid, N-methyl-, N-ethyl- or N- propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethyl- amine or tri(2-hydroxyethyl)amine, or heterocyclic bases, for example N-ethyl-piperidine or N.N'-dimethylpiperazine.

When a basic group and an acid group are present in the same molecule, a compound of formula I may also form internal salts. For isolation or purification purposes it is also possible to use pharmaceutically unacceptable salts, for example picrates or perchlorates. For therapeutic use, only pharmaceutically acceptable salts or free compounds are employed (where applicable comprised in pharmaceutical preparations), and these are therefore preferred.

### Process of Manufacture

In a second aspect, the invention relates to the anufacture a compound of the formula I.

A compound of the formula I is prepared analogously to methods that, for other compounds, are in principle known in the art, so that for the novel compounds of the formula I the process is novel as analogy process, preferably by reacting a compound of the formula II 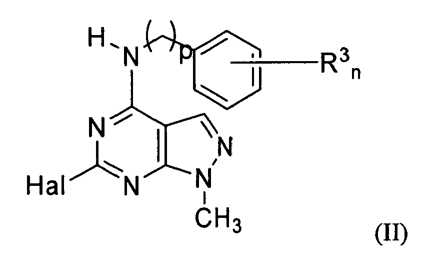 wherein R3, n, p are as defined for a compound of the formula I, Hal is halo, especially bromo or chloro, with a compound of the formula (III)

**(R¹)(R²)NH** **(III)**

wherein R1 and R2 are as defined for a compound of the formula I;
optionally removing any protecting groups present;
optionally transforming a compound of the formula I into a different compound of the formula I, transforming a salt of a compound of the formula I into the free compound or a different salt, transforming a free compound of the formula I into a salt thereof, and/or
optionally separating a mixture of isomers of a compound of the formula I into the individual isomers.

The reaction can preferably take place in a solvent or solvent mixture, e.g. in an ether, such as thf, at elevated temperatures, e.g. in the range from 30 °C to the reflux temperature.

The properties, introduction, types and removal of protecting groups (if present at all) are as described below in the standard reference works mentioned under "General Process Conditions".

### Optional Reactions and Conversions

A compound of the formula I may be converted into a different compound of the formula I by applying known methods to the obtained compounds.

For example, in a compound of the formula I, wherein a substituent is halo-aryl, such as bromo-aryl, e.g. bromophenyl, the halogen may be replaced with a substituent bound via a nitrogen atom, for example with morpholino, by reaction with a corresponding primary or secondary amine, such as morpholine, in the presence of a strong base, such as an alkaline metal alkoxide, e.g. potassium tert-butoxide, and an appropriate coupling catalyst, e.g. 2-(dimethyl- amino-)-2-biphenylyl-palladium(11)chloride dinorbornylphosphin complex, in an appropriate solvent or solvent mixture, e.g. an ether, such as tetrahydrofurane. The halogen may also be replaced with an appropriate boronic acid to form diaryl derivatives under Suzuki reaction conditions in presence of an catalysator, e.g. tetrakistriphenylpalladium(0) in an appropriate solvent e.g. dimethlyformamide, or solvent mixture.

Yet another example of a conversion of a compound of the formula I can be given where a nitro substitutent is present in substituted aryl; such a nitro substituent can be reduced to a corresponding amino substituent, for example by catalytic hydrogenation, e.g. in the presence of Raney-Ni, in an appropriate solvent or solvent mixture, e.g. an alcohol, such as methanol or ethanol, e.g. at temperatures from 0 to 50 °C.

An amino substituent in a compound of the formula I (e.g. amino as substituent of aryl R1 in formula I) can be converted into a di- or tri-alkylated amino (in the latter case quarter- nary) substituent by reaction with a corresponding alkyl halogenide, e.g. methyl iodide, preferably in the presence of a tertiary nitrogen base, such as triethylamine, in an appropriate solvent or solvent mixture, e.g. an N,N-di-(lower alkyl)-lower alkanoylamide, such as N, N- dimethylformamide, preferably at temperatures from 20 to 80 °C. Similary the amino substituent can react with an acid chloride or can be coupled in the presence of appropriate coupling reagents, e.g. 1-Hydroxybenzotriazole (HOBT), to to form a corresponding amide. The amino substituent can be converted into sulfonamides by reacting the free amine with sulfonylchlorides, e.g. methylsulfonylchloride, or can be converted into ureas by reacting the free amine with appropriate isocyanates.

Salts of compounds of formula I having at least one salt-forming group may be prepared in a manner known per se. For example, a salt of a compound of formula I having acid groups may be formed by treating the compound with a metal compound, such as an alkali metal salt of a suitable organic carboxylic acid, e.g. the sodium salt of 2-ethylhexanoic acid, with an organic alkali metal or alkaline earth metal compound, such as the corresponding hydroxide, carbonate or hydrogen carbonate, such as sodium or potassium hydroxide, carbonate or hydrogen carbonate, with a corresponding calcium compound or with ammonia or a suitable organic amine, stoichiometric amounts or only a small excess of the salt-forming agent preferably being used. An acid addition salt of compounds of formula I can be obtained in customary manner, e.g. by treating a compound of the formula I with an acid or a suitable anion exchange reagent. Internal salts of compounds of formula I containing acid and basic salt- forming groups, e.g. a free carboxy group and a free amino group, may be formed, e.g. by the neutralization of salts, such as acid addition salts, to the isoelectric point, e.g. with weak bases, or by treatment with ion exchangers.

A salt of a compound of the formula I can be converted in customary manner into the free compound; a metal or ammonium salt can be converted, for example, by treatment with a suitable acid, and an acid addition salt, for example, by treatment with a suitable basic agent. In both cases, suitable ion exchangers may be used. Stereoisomeric mixtures, e.g. mixtures of diastereomers, can be separated into their corresponding isomers in a manner known per se by means of appropriate separation methods. Diastereomeric mixtures for example may be separated into their individual diastereomers by means of fractionated crystallization, chromatography, solvent distribution, and similar procedures. This separation may take place either at the level of one of the starting compounds or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

Intermediates and final products can be worked up and/or purified according to standard methods, e.g. using chromatographic methods, distribution methods, (re-) crystallization, and the like.

### Starting Materials

The starting materials may be prepared as follows:
A compound of the formula II is prepared analogously to methods that, for other compounds, are in principle known in the art. For the novel compounds of the formula II the process is novel as analogy process. A compound of formula II may be obtained by reacting a compound of formula IV 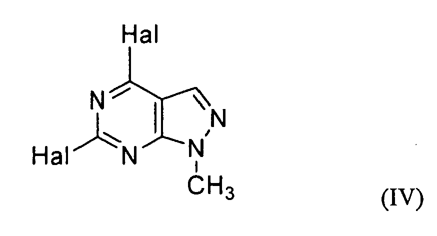 wherein each Hal represents independently halo, especially bromo or chloro, with a compound of the formula (V) 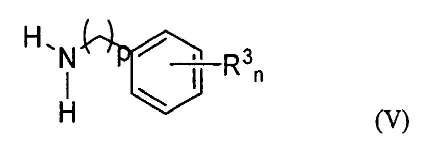 wherein R3, n, p are as defined for a compound of the formula I,
   optionally removing any protecting groups present,
   optionally transforming a thus obtained compound of the formula II into a different compound of the formula II, transforming a salt of a compound of the formula II into the free compound or a different salt, transforming a free compound of the formula II into a salt thereof, and/or separating a mixture of isomers of a compound of the formula II into the individual isomers.

The starting materials, compounds of formula III, IV, V are known and commercially available or obtainable according to known procedures. An example for manufacturing a compound of formula IV, wherein both Hal represent Cl, is provided later in this specification.

### General process conditions

The following applies in general to all processes mentioned hereinbefore and hereinafter, while reaction conditions specifically mentioned above or below are preferred:
In any of the reactions mentioned hereinbefore and hereinafter, **protecting groups** may be used where appropriate or desired, even if this is not mentioned specifically, to protect functional groups that are not intended to take part in a given reaction, and they can be introduced and/or removed at appropriate or desired stages. Reactions comprising the use of protecting groups are therefore included as possible wherever reactions without specific mentioning of protection and/or deprotection are described in this specification. Within the scope of this disclosure only a readily removable group that is not a constituent of the particular desired end product of formula I is designated a "protecting group", unless the context indicates otherwise. The protection of functional groups by such protecting groups, the protecting groups themselves, and the reactions appropriate for their removal are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" {Methods of Organic Chemistry), Houben Weyl, 4th edition, Volume 15/1, Georg Thieme Veriag, Stuttgart 1974, in H.-D. Jakubke and H. Jeschkeit, "Aminosauren, Peptide, Proteine" {Amino acids, Peptides, Proteins), Veriag Chemie, Weinheim, Deerfield Beach, and Basel 1982, and in Jochen Lehmann, "Chemie der Kohlenhydrate: Monosaccharide und Derivate" {Chemistry of Carbohydrates: Monosaccharides and Derivatives), Georg Thieme Veriag, Stuttgart 1974. A characteristic of protecting groups is that they can be removed readily (i.e. without the occurrence of undesired secondary reactions) for example by solvolysis, reduction, photolysis or alternatively under physiological conditions (e.g. by enzymatic cleavage). AII the above-mentioned process steps can be carried out under reaction conditions that are known per se, preferably those mentioned specifically, in the absence or, customarily, in the presence of solvents or diluents, preferably solvents or diluents that are inert towards the reagents used and dissolve them, in the absence or presence of catalysts, condensation or neutralizing agents, for example ion exchangers, such as cation exchangers, e.g. in the H<+> form, depending on the nature of the reaction and/or of the reactants at reduced, normal or elevated temperature, for example in a temperature range of from about -100 °C to about 190°C, preferably from approximately -80°C to approximately 150°C, for example at from -80 to -60°C, at room temperature, at from -20 to 40 °C or at reflux temperature, under atmospheric pressure or in a closed vessel, where appropriate under pressure, and/or in an inert atmosphere, for example under an argon or nitrogen atmosphere.

The **solvents** from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetonitrile, halogenated hydrocarbons, e.g. as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopen- tane, or mixtures of these, for example aqueous solutions, unless otherwise indicated in the description of the processes. Such solvent mixtures may also be used in working up, for example by chromatography or partitioning.

The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in sjtu. In the process of the present invention those starting materials are preferably used which result in compounds of formula I described as being preferred. The invention also relates to novel intermediates and/or starting materials. Special preference is given to reaction conditions that are identical or analogous to those mentioned in the Examples.

### Uses of compounds / applicability

The invention relates in a further aspect of the invention to the use of a compound of the formula I or a pharmaceutically acceptable salt thereof, for the treatment of protein kinase modulation responsive diseases, especially in an animal or preferably a human, especially a disease responsive to the inhibition of one or more protein tyrosine kinases (PTKs) mentioned under " General Description of the Invention" , more especially one or more PTKs selected from abl kinase, especially v-abl or c-abl kinase, kinases from the family of the src kinases, especially c-src kinase, b-raf (V599E) and/or especially RET-receptor kinase or Ephrin receptor kinases, e.g. EphB2 kinase, EphB4 kinase or related kinases, or mutated (e.g. constitutively active or otherwise partially or totally deregulated) forms thereof - often here good inhibition values are found in comparison to EGF receptor kinase or other kinases of the EGF family, for example HER-1 or c-erbB2 kinase (HER-2) and/or VEG F-receptor kinase (e.g. KDR and Flt-1), however, also these PTK may be usefully inhibited by one or more compounds of the formula I.

The compounds of formula I have valuable pharmacological properties and are useful in the treatment of protein kinase, especially protein tyrosine kinase (especially one or more of the protein kinases mentioned above under "General Description of the invention" , most especially abl kinase, especially v-abl or c-abl kinase, kinases from the family of the src kinases, especially c-src kinase, RET-receptor kinase or Ephrin receptor kinases, e.g. EphB2 kinase, EphB4 kinase or related kinases, and/or b-raf (V599E), as well as (e.g. constitutively activated) mutated forms of any one or more of these) modulation responsive diseases, where modulation preferably means inhibition and responsive means that the progress of a disease and/or its symptoms is slowed, stopped or even inverted up to and including a complete or at least temporary cure. The term "treatment" includes especially prophylaxis including preventative treatment, e.g. in patients where mutations or changes have been found that indicate that they are or may be prone to the development of a disease, or preferably therapeutic (including but not limited to palliative, curative, symptom-alleviating, symptom-reducing, disease- or symptom-suppressing, progression-delaying, kinase-regu- lating and/or kinase-inhibiting) treatment of said diseases, especially of any one or more of the diseases mentioned below.

The term "curative" as used herein preferably means efficacy in treating ongoing episodes involving (specially deregulated) receptor tyrosine kinase activity. The term "prophylactic" preferably means the prevention of the onset or recurrence of diseases involving deregulated receptor tyrosine kinase activity.

The term "delay of progression" as used herein especially means administration of the active compound to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop, or where e.g. metastasation can be expected without treatment.

An animal is preferably a warm-blooded animal, more preferably a mammal. A human (which generally also falls under the general term "animal") is especially a patient or a person that (e.g. due to some mutation or other features) is prone to a risk for a disease as defined above or below.

Where subsequently or above the term "use" is mentioned (as verb or noun) (relating to the use of a compound of the formula I or a pharmaceutically acceptable salt thereof), this (if not indicated differently or suggested differently by the context) includes any one or more of the following embodiments of the invention, respectively (if not stated otherwise): the use in the treatment of a protein (especially tyrosine) kinase modulation (especially inhibition) responsive disease, the use for the manufacture of pharmaceutical compositions for use in the treatment of a protein kinase modulation (especially inhibition) responsive disease, methods of use of one or more compounds of the formula I in the treatment of a protein kinase modulation (especially inhibition) responsive and/or proliferative disease, pharmaceutical preparations comprising one or more compounds of the formula I for the treatment of said protein kinase modulation (especially inhibition) responsive disease, and one or more compounds of the formula I in the treatment of said protein kinase modulation (especially inhibition) responsive disease, as appropriate and expedient, if not stated otherwise. In particular, diseases to be treated and are thus preferred for "use" of a compound of formula I are selected from (especially tyrosine) protein kinase modulation (especially inhibition) responsive (meaning also "supported" , not only "dependent" , including also situations where a disease is responding to modulation, especially inhibition, of a protein kinase, that is, the activity of the protein kinase supports or even causes disease manifestation) diseases mentioned below, especially proliferative diseases mentioned below.

Where a protein kinase is mentioned, this relates to any type of protein kinase, especially one of those defined above under "General Description of the Invention" , more especially serine/threonine and/or preferably protein tyrosine kinases, most preferably one or more tyrosine protein kinases, especially selected from the group consisting of v-abl or c-abl kinase, kinases from the family of the src kinases, especially c-src kinase, b-raf (V599E) and/or preferably RET-receptor kinase or Ephrin receptor kinases, e.g. EphB2 kinase, EphB4 kinase or related kinases, including one or more altered or mutated or allelic forms of any one or more of these (e.g. those that result in conversion of the respective proto-oncogene into an oncogene, such as constitutively activated mutants, e.g. Bcr-Abl). Especially an abnormally highly-expressed, constitutively activated or normal but in the given context of other regulatory mechanism in a patient relatively overactive, and/or mutated form is encompassed.

The usefulness of the compounds of the present invention in the modulation, especially as inhibitors, of protein kinases can especially and paradigmatically be demonstrated by the test systems described herein for the protein kinases mentioned as preferred above. An "inhibitor" is a test compound of the formula I if not mentioned otherwise. The efficacy of compounds of the formula I as inhibitors or Eph B4 receptor (EphB4) kinases can be demonstrated as described in the examples section of this specification. The results indicate an advantageous selectivity profile of some preferred compounds of the formula I, where selectivity does not necessarily mean that only one kinase is inhibited to an advantageous extent, but also that selectively two or more kinases may be inhibited stronger in comparison to other kinases.

In a preferred sense of the invention, a protein kinase modulation responsive disease is a disorder that responds in a for the treated individual beneficial way to modulation, especially inhibition, of the activity of a protein (preferably tyrosine) kinase, especially one characterized as being preferred above, where a compound of the formula I can be used, is one or more of a proliferative disease (meaning one dependent on (especially inadequate) activity of a protein kinase) including a hyperproliferative condition, such as one or more of leukemia, hyperplasia, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty. Further, a compound of the formula I may be used for the treatment of thrombosis and/or scleroderma.

Preferred is the use of a compound of the formula I in the therapy (including prophylaxis) of a proliferative disorder (especially which is responsive to modulation, especially inhibition, of the activity of a protein (preferably tyrosine) kinase, especially as mentioned as preferred herein) selected from tumor or cancer diseases, especially against preferably a benign or especially malignant tumor or cancer disease, more preferably solid tumors, e.g. carcinoma of the brain, kidney, liver, adrenal gland, bladder, breast, stomach (especially gastric tumors), ovaries, colon, rectum, prostate, pancreas, lung (e.g. small or large cell lung carcinomas), vagina, thyroid, sarcoma, glioblastomas, multiple myeloma or gastrointestinal cancer, especially colon carcinoma or colorectal adenoma, or a tumor of the neck and head, e.g. squameous carcinoma of the head and neck, including neoplasias, especially of epithelial character, e.g. in the case of mammary carcinoma; an epidermal hyperproliferation (other than cancer), especially psoriasis; prostate hyperplasia; or a leukemia.

A compound of formula I or its use makes it possible to bring about the regression of tumors and/or to prevent the formation of tumor metastases and the growth of (also micro)- metastases.

It is also possible to use the compounds of the formula I in the treatment of diseases of the immune system insofar as several or, especially, individual protein (preferably tyrosine) kinases, especially those mentioned as preferred, are involved. Furthermore, the compounds of the formula I can be used also in the treatment of diseases of the central or peripheral nervous system, in which signal transmission by at least one protein (preferably tyrosine) kinase, especially selected from those protein tyrosine kinases mentioned as preferred, is involved.

Angiogenesis is regarded as an absolute prerequisite for those tumors which grow beyond a maximum diameter of about 1-2 mm; up to this limit, oxygen and nutrients may be supplied to the tumor cells by diffusion. Every tumor, regardless of its origin and its cause, is thus dependent on angiogenesis for its growth after it has reached a certain size. Three principal mechanisms play an important role in the activity of angiogenesis inhibitors against tumors: 1 ) Inhibition of the growth of vessels, especially capillaries, into avascular resting tumors, with the result that there is no net tumor growth owing to the balance that is achieved between apoptosis and proliferation; 2) Prevention of the migration of tumor cells owing to the absence of blood flow to and from tumors; and 3) Inhibition of endothelial cell proliferation, thus avoiding the paracrine growth-stimulating effect exerted on the surrounding tissue by the endothelial cells normally lining the vessels. Compounds of the formula I, in regard of their ability to inhibit KDR and Ephrin receptor kinase, e.g. EphB4 kinase, and possibly other protein kinases, and thus to modulate angiogenesis, are especially appropriate for the use against diseases or disorders related to the inadequate activity of the corresponding receptor (preferably tyrosine) kinase, especially an overexpression thereof. Among these diseases, especially (e.g. ischemic) retinopathies, (e.g. age related) macula degeneration, psoriasis, obesity, haemangioblastoma, haem- angioma, inflammatory diseases, such as rheumatoid or rheumatic inflammatory diseases, especially arthritis, such as rheumatoid arthritis, or other chronic inflammatory disorders, such as chronic asthma, arterial or post-transplantational atherosclerosis, endometriosis, and especially neoplastic diseases, for example so-called solid tumors (especially cancers of the gastrointestinal tract, the pancreas, breast, stomach, cervix, bladder, kidney, prostate, ovaries, endometrium, lung, brain, melanoma, Kaposi' s sarcoma, squamous cell carcinoma of head and neck, malignant pleural mesotherioma, lymphoma or multiple myeloma) and further liquid tumors (e.g. leukemias) are especially important.

The compounds of the formula I are especially of use to prevent or treat diseases that are triggered by persistent angiogenesis, such as restenosis, e.g. stent-induced restenosis; Crohn' s disease; Hodgkin' s disease; eye diseases, such as diabetic retinopathy and neovascular glaucoma; renal diseases, such as glomerulonephritis; diabetic nephropathy; inflammatory bowel disease; malignant nephrosclerosis; thrombotic microangiopathic syndromes; (e.g. chronic) transplant rejections and glomerulopathy; fibrotic diseases, such as cirrhosis of the liver; mesangial cell-proliferative diseases; injuries of the nerve tissue; and for inhibiting the re-occlusion of vessels after balloon catheter treatment, for use in vascular prosthetics or after inserting mechanical devices for holding vessels open, such as, e.g., stents, as immunosuppressants, as an aid in scar-free wound healing, and for treating age spots and contact dermatitis.

Preferably, the invention relates to the use of compounds of the formula I or pharmaceutically acceptable salts thereof, in the treatment of solid tumors as mentioned herein and/or of liquid tumors, e.g. leukemias, as mentioned herein.

Due to their protein kinase, such as Eph receptor kinase, modulating properties, the compounds of the formula I can also be used for stimulating or promoting neural regeneration (neuronal regeneration; neuroregeneration), such as axon regeneration, or inhibiting or reversing neural degeneration (neuronal degeneration; neurodegeneration). These uses represent further aspects of the instant invention.

The compounds of the formula I are, therefore, also useful in the treatment of protein kinase, such as Eph receptor kinase, modulation responsive conditions, diseases or disorders, where the stimulation or the promotion of neural regeneration (neuronal regeneration; neuroregeneration), such as axon regeneration, or the inhibition or the reversal of neural degeneration (neuronal degeneration; neurodegeneration) is desired, e. g. in the treatment of spinal cord injury, hypoxic conditions, traumatic brain injury, infarct, stroke, multiple sclerosis or other neurodegenerative conditions, diseases or disorders. These uses and methods of treatment represent further aspects of the instant invention.

### Pharmaceutical Compositions

The invention relates in a further aspect to pharmaceutical compositions comprising a compound of formula I to their use in the therapeutic (in a broader aspect of the invention also prophylactic) treatment or a method of treatment of a disease or disorder that depends on inadequate protein (especially tyrosine) kinase activity, especially the preferred disorders or diseases mentioned above, to the compounds for said use and to pharmaceutical preparations and their manufacture, especially for said uses. More generally, pharmaceutical preparations are useful in case of compounds of the formula I.

The pharmacologically acceptable compounds of the present invention may be present in or employed, for example, for the preparation of pharmaceutical compositions that comprise an effective amount of a compound of the formula I, or a pharmaceutically acceptable salt thereof, as active ingredient together or in admixture with one or more inorganic or organic, solid or liquid, pharmaceutically acceptable carriers (carrier materials).

The invention relates also to a pharmaceutical composition that is suitable for administration to a warm-blooded animal, especially a human (or to cells or cell lines derived from a warmblooded animal, especially a human, e.g. lymphocytes), for the treatment (this, in a broader aspect of the invention, also including prevention of (= prophylaxis against)) a disease that responds to inhibition of protein (especially tyrosine) kinase activity, comprising an amount of a compound of formula I or a pharmaceutically acceptable salt thereof, preferably which is effective for said inhibition, together with at least one pharmaceutically acceptable carrier.

The pharmaceutical compositions according to the invention are those for enteral, such as nasal, rectal or oral, or parenteral, such as intramuscular or intravenous, administration to warm-blooded animals (especially a human), that comprise an effective dose of the pharmacologically active ingredient, alone or together with a significant amount of a pharmaceutically acceptable carrier. The dose of the active ingredient depends on the species of warmblooded animal, the body weight, the age and the individual condition, individual pharmacokinetic data, the disease to be treated and the mode of administration.

The invention relates also to method of treatment for a disease that responds to inhibition of a disease that depends on inadequate activity of a protein (especially tyrosine) kinase; which comprises administering a prophylactically or especially therapeutically effective amount of a compound of formula I, or a pharmaceutically acceptable salt thereof, , especially to a warmblooded animal, for example a human, that, on account of one of the mentioned diseases, requires such treatment. The dose of a compound of the formula I or a pharmaceutically acceptable salt thereof to be administered to warm-blooded animals, for example humans of approximately 70 kg body weight, preferably is from approximately 3 mg to approximately 10 g, more preferably from approximately 10 mg to approximately 1.5 g, most preferably from about 100 mg to about 1000 mg /person/day, divided preferably into 1-3 single doses which may, for example, be of the same size. Usually, children receive half of the adult dose.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragees, tablets or capsules.

The pharmaceutical compositions of the present invention are prepared in a manner known per se, for example by means of conventional dissolving, lyophilizing, mixing, granulating or confectioning processes.

Solutions of the active ingredient, and also suspensions, and especially isotonic aqueous solutions or suspensions, are preferably used, it being possible, for example in the case of lyophilized compositions that comprise the active ingredient alone or together with a carrier, for example mannitol, for such solutions or suspensions to be produced prior to use. The pharmaceutical compositions may be sterilized and/or may comprise excipients, for example preservatives, stabilizers, wetting and/or emulsifying agents, solubilizers, salts for regulating the osmotic pressure and/or buffers, and are prepared in a manner known per se, for example by means of conventional dissolving or lyophilizing processes. The said solutions or suspensions may comprise viscosity-increasing substances, such as sodium carboxymethyl- cellulose, carboxymethylcellulose, dextran, polyvinylpyrrolidone or gelatin.

Suspensions in oil comprise as the oil component the vegetable, synthetic or semi-synthetic oils customary for injection purposes. There may be mentioned as such especially liquid fatty acid esters that contain as the acid component a long-chained fatty acid having from 8- 22, especially from 12-22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brasidic acid or linoleic acid, if desired with the addition of antioxidants, for example vitamin E, [beta]-carotene or 3,5-di-tert-butyl-4-hydroxytoluene. The alcohol component of those fatty acid esters has a maximum of 6 carbon atoms and is a mono- or poly-hydroxy, for example a mono-, di- or tri-hydroxy, alcohol, for example methanol, ethanol, propanol, butanol or pentanol or the isomers thereof, but especially glycol and glycerol. The following examples of fatty acid esters are therefore to be mentioned: ethyl oleate, isopropyl myristate, isopropyl palmitate, "Labrafil M 2375" (polyoxyethylene glycerol trioleate, Gattefosse, Paris), "Miglyol 812" (triglyceride of saturated fatty acids with a chain length of C8 to C12, H[upsilon]ls AG, Germany), but especially vegetable oils, such as cottonseed oil, almond oil, olive oil, castor oil, sesame oil, soybean oil and groundnut oil.

The injection or infusion compositions are prepared in customary manner under sterile conditions; the same applies also to introducing the compositions into ampoules or vials and sealing the containers.

Pharmaceutical compositions for oral administration can be obtained by combining the active ingredient with solid carriers, if desired granulating a resulting mixture, and processing the mixture, if desired or necessary, after the addition of appropriate excipients, into tablets, dragee cores or capsules. It is also possible for them to be incorporated into plastics carriers that allow the active ingredients to diffuse or be released in measured amounts.

Suitable carriers are especially fillers, such as sugars, for example lactose, saccharose, mannitol or sorbitol, cellulose preparations and/or calcium phosphates, for example tri- calcium phosphate or calcium hydrogen phosphate, and binders, such as starch pastes using for example corn, wheat, rice or potato starch, gelatin, tragacanth, methylcellulose, hydroxypropylmethyl cellulose, sodium carboxymethyl cellulose and/or polyvinylpyrrolidone, and/or, if desired, disintegrators, such as the above-mentioned starches, and/or carboxymethyl starch, crosslinked polyvinylpyrrolidone, agar, alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Dragee cores are provided with suitable, optionally enteric, coatings, there being used, inter alia, concentrated sugar solutions which may comprise gum arabic, talc, polyvinylpyrrolidone, polyethylene glycol and/or titanium dioxide, or coating solutions in suitable organic solvents, or, for the preparation of enteric coatings, solutions of suitable cellulose preparations, such as ethylcellulose phthalate or hydroxypropylmethylcellulose phthalate. Capsules are dry-filled capsules made of gelatin and soft sealed capsules made of gelatin and a plasticizer, such as glycerol or sorbitol. The dry-filled capsules may comprise the active ingredient in the form of granules, for example with fillers, such as lactose, bin-ders, such as starches, and/or glidants, such as talc or magnesium stearate, and if desired with stabilizers. In soft capsules the active ingredient is preferably dissolved or suspended in suitable oily excipients, such as fatty oils, paraffin oil or liquid polyethylene glycols, it being possible also for stabilizers and/or antibacterial agents to be added. Dyes or pigments may be added to the tablets or dragee coatings or the capsule casings, for example for identification purposes or to indicate different doses of active ingredient.

### Combinations with other active agents

A compound of the formula I may also be used to advantage in combination with other biologically active agents, preferentially with other antiproliferative agents. Such antiproliferative agents include, but are not limited to aromatase inhibitors; antiestrogens; topoisomerase I inhibitors; topoisomerase Il inhibitors; microtubule active agents; alkylating agents; histone deacetylase inhibitors; compounds which induce cell differentiation processes; cyclooxy- genase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopep- tidase inhibitors; bisphosphonates; biological response modifiers; antiproliferative antibodies; heparanase inhibitors; inhibitors of Ras oncogenic isoforms; telomerase inhibitors; proteasome inhibitors; agents used in the treatment of hematologic malign-nancies; compounds which target, decrease or inhibit the activity of Flt-3; Hsp90 inhibitors; and temozolomide (TEMODAL(R)).

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN. Formestane can be administered, e.g., in the form as it is marketed, e.g. under the trademark LENTARON. Fadrozole can be administered, e.g., in the form as it is marketed, e.g. under the trademark AFEMA. Anastrozole can be administered, e.g., in the form as it is marketed, e.g. under the trademark ARIMIDEX. Letrozole can be administered, e.g., in the form as it is marketed, e.g. under the trademark FEMARA or FEMAR. Aminoglutethimide can be administered, e.g., in the form as it is marketed, e.g. under the trademark ORIMETEN. A combination of the invention comprising a chemotherapeutic agent which is an aromatase inhibitor is particularly useful for the treatment of hormone receptor positive tumors, e.g. breast tumors.

The term "anti-estrogen" as used herein relates to a compound which antagonizes the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX. Raloxifene hydrochloride can be administered, e.g., in the form as it is marketed, e.g. under the trademark EVISTA. Fulvestrant can be formulated as disclosed in US 4,659,516 or it can be administered, e.g., in the form as it is marketed, e.g. under the trademark FASLODEX. A combination of the invention comprising a chemotherapeutic agent which is an antiestrogen is particularly useful for the treatment of estrogen receptor positive tumors, e.g. breast tumors.

The term "anti-androgen" as used herein relates to any substance which is capable of inhibiting the biological effects of androgenic hormones and includes, but is not limited to, bica- lutamide (CASODEX), which can be formulated, e.g. as disclosed in US 4,636,505.

The term "gonadorelin agonist" as used herein includes, but is not limited to abarelix, go- serelin and goserelin acetate. Goserelin is disclosed in US 4,100,274 and can be administered, e.g., in the form as it is marketed, e.g. under the trademark ZOLADEX. Abarelix can be formulated, e.g. as disclosed in US 5,843,901.

The term "topoisomerase I inhibitor" as used herein includes, but is not limited to topotecan, gimatecan, irinotecan, camptothecian and its analogues, 9-nitrocamptothecin and the ma- cromolecular camptothecin conjugate PNU-166148 (compound A1 in WO99/ 17804). Irinotecan can be administered, e.g. in the form as it is marketed, e.g. under the trademark CAMPTOSAR. Topotecan can be administered, e.g., in the form as it is marketed, e.g. under the trademark HYCAMTIN.

The term "topoisomerase Il inhibitor" as used herein includes, but is not limited to the anthracyclines such as doxorubicin (including liposomal formulation, e.g. CAELYX), dauno- rubicin, epirubicin, idarubicin and nemorubicin, the anthraquinones mitoxantrone and lo- soxantrone, and the podophillo toxines etoposide and teniposide. Etoposide can be administered, e.g. in the form as it is marketed, e.g. under the trademark ETOPOPHOS. Teniposide can be administered, e.g. in the form as it is marketed, e.g. under the trademark VM 26-BRISTOL. Doxorubicin can be administered, e.g. in the form as it is marketed, e.g. under the trademark ADRIBLASTIN or ADRIAMYCIN. Epirubicin can be administered, e.g. in the form as it is marketed, e.g. under the trademark FARMORUBICIN. ldarubicin can be administered, e.g. in the form as it is marketed, e.g. under the trademark ZAVEDOS. Mitoxan- trone can be administered, e.g. in the form as it is marketed, e.g. under the trademark NOVANTRON.

The term "microtubule active agent" relates to microtubule stabilizing, microtubule destabilizing agents and microtublin polymerization inhibitors including, but not limited to taxanes, e.g. paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g. epothilone B or a derivative thereof. Paclitaxel may be administered e.g. in the form as it is marketed, e.g. TAXOL. Docetaxel can be administered, e.g., in the form as it is marketed, e.g. under the trademark TAXOTERE. Vinblastine sulfate can be administered, e.g., in the form as it is marketed, e.g. under the trademark VINBLASTIN R.P.. Vincristine sulfate can be administered, e.g., in the form as it is marketed, e.g. under the trademark FARMISTIN. Discodermolide can be obtained, e.g., as disclosed in US 5,010,099. Also included are Epothilone derivatives which are disclosed in WO 98/10121, US 6,194,181 , WO 98/25929, WO 98/08849, WO 99/43653, WO 98/22461 and WO 00/31247. Especially preferred are Epothilone A and/or B.

The term "alkylating agent" as used herein includes, but is not limited to, cyclophosphamide, ifosfamide, melphalan or nitrosourea (BCNU or Gliadel). Cyclophosphamide can be administered, e.g., in the form as it is marketed, e.g. under the trademark CYCLOSTIN. Ifosfamide can be administered, e.g., in the form as it is marketed, e.g. under the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4-[[(2-hydroxyethyl)[2-(1H- indol-3-yl)ethyl]-amino]methyl]phenyl]-2E-2-propenamide, N-hydroxy-3-[4-[[[2-(2-methyl-1H- indol-3-yl)-ethyl]-amino]methyl]phenyl]-2E-2-propenamide and pharmaceutically acceptable salts thereof. It further especially includes Suberoylanilide hydroxamic acid (SAHA).

The term "antineoplastic antimetabolite" includes, but is not limited to, 5-fluorouracil (5-FU); capecitabine; gemcitabine; DNA demethylating agents, such as 5-azacytidine and deci- tabine; methotrexate; edatrexate; and folic acid antagonists such as pemetrexed. Capecita-bine can be administered, e.g., in the form as it is marketed, e.g. under the trademark XELODA. Gemcitabine can be administered, e.g., in the form as it is marketed, e.g. under the trademark GEMZAR. Also included is the monoclonal antibody trastuzumab which can be administered, e.g., in the form as it is marketed, e.g. under the trademark HERCEPTIN.

The term "platin compound" as used herein includes, but is not limited to, carboplatin, cis-platin, cisplatinum and oxaliplatin. Carboplatin can be administered, e.g., in the form as it is marketed, e.g. under the trademark CARBOPLAT. Oxaliplatin can be administered, e.g., in the form as it is marketed, e.g. under the trademark ELOXATIN.

The term "compounds targeting/decreasing a protein or lipid kinase activity and further anti-angiogenic compounds" as used herein includes, but is not limited to: protein tyrosine kinase and/or serine and/or threonine kinase inhibitors or lipid kinase inhibitors, e.g.: a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g. a N-phenyl-2-pyri- midine-amine derivative, e.g. imatinib, SU101, SU6668, and GFB-111 ; b) compounds targeting, decreasing or inhibiting the activity of the fibroblast growth factor- receptors (FGFR); c) compounds targeting, decreasing or inhibiting the activity of the insulin-like growth factor I receptor (IGF-IR), especially compounds which inhibit the IGF-IR, such as those compounds disclosed in WO 02/092599; d) compounds targeting, decreasing or inhibiting the activity of the Trk receptor tyrosine kinase family; e) compounds targeting, decreasing or inhibiting the activity of the AxI receptor tyrosine kinase family; f) compounds targeting, decreasing or inhibiting the activity of the c-Met receptor; g) compounds targeting, decreasing or inhibiting the activity of the c-Kit receptor tyrosine kinases - (part of the PDGFR family), such as compounds which target, decrease or inhibit the activity of the c-Kit receptor tyrosine kinase family, especially compounds which inhibit the c-Kit receptor, e.g. imatinib; h) compounds targeting, decreasing or inhibiting the activity of members of the c-Abl family and their gene-fusion products (e.g. BCR-AbI kinase), such as compounds which target decrease or inhibit the activity of c-Abl family members and their gene fusion products, e.g. a N-phenyl-2-pyrimidine-amine derivative, e.g. imatinib; PD180970; AG957; NSC 680410; or PD173955 from ParkeDavis; i) compounds targeting, decreasing or inhibiting the activity of members of the protein kinase C (PKC) and Raf family of serine/threonine kinases, members of the MEK, SRC, JAK, FAK, PDK and Ras/MAPK family members, or P1(3) kinase family, or of the Pl(3)- kinase-related kinase family, and/or members of the cyclin-dependent kinase family (CDK) and are especially those staurosporine derivatives disclosed in US 5,093,330, e.g. midostaurin; examples of further compounds include e.g. UCN-01 , safingol, BAY 43-9006, Bryostatin 1, Perifosine; llmofosine; RO 318220 and RO 320432; GO 6976; lsis 3521; LY333531/LY379196; isochinoline compounds such as those disclosed in WO 00/09495; FTIs; PD184352 or QAN697 (a P13K inhibitor); j) compounds targeting, decreasing or inhibiting the activity of a protein-tyrosine kinase, such as imatinib mesylate (GLIVEC/GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzene malonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydro- xyphenyl)methyl]amino}-benzoic acid adamantyl ester; NSC 680410, adaphostin); and k) compounds targeting, decreasing or inhibiting the activity of the epidermal growth factor family of receptor tyrosine kinases (EGFR, ErbB2, ErbB3, ErbB4 as homo- or heterodimers), such as compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180); e.g. trastuzumab (HerpetinR), cetuximab, Iressa, erlotinib (Tarceva(TM)), CI-1033, EKB-569, GW- 2016, E1.1 , E2.4, E2.5, E6.2, E6.4, E2.11, E6.3 or E7.6.3, and 7H-pyrrolo-[2,3-d]pyrimidine derivatives which are disclosed in WO 03/013541. Further anti-angiogenic compounds include compounds having another mechanism for their activity, e.g. unrelated to protein or lipid kinase inhibition e.g. thalidomide (THALOMID) and TNP-470.

Compounds which target, decrease or inhibit the activity of a protein or lipid phosphatase are e.g. inhibitors of phosphatase 1, phosphatase 2A, PTEN or CDC25, e.g. okadaic acid or a derivative thereof.

Compounds which induce cell differentiation processes are e.g. retinoic acid, [alpha]- [gamma]- or [delta]-toco- pherol or [alpha]- [gamma]- or [delta]-tocotrienol.

The term "cyclooxygenase inhibitor" as used herein includes, but is not limited to, e.g. Cox- 2 inhibitors, 5-alkyl substituted 2-arylaminophenylacetic acid and derivatives, such as cele- coxib (CELEBREX), rofecoxib (VIOXX), etoricoxib, valdecoxib or a 5-alkyl-2-arylaminophe- nylacetic acid, e.g. 5-methyl-2-(2' - chloro-6' -fluoroanilino)phenyl acetic acid, lumiracoxib.

The term "mTOR inhibitors" relates to compounds which inhibit the mammalian target of rapamycin (mTOR) and which possess antiproliferative activity such as sirolimus (Rapamune(R)), everolimus (Certican(TM)), CCI-779 and ABT578.

The term "bisphosphonates" as used herein includes, but is not limited to, etridonic, clo- dronic, tiludronic, pamidronic, alendronic, ibandronic, risedronic and zoledronic acid. "Etridonic acid" can be administered, e.g., in the form as it is marketed, e.g. under the trademark DIDRONEL. "Clodronic acid" can be administered, e.g., in the form as it is marketed, e.g. under the trademark BONEFOS. "Tiludronic acid" can be administered, e.g., in the form as it is marketed, e.g. under the trademark SKELID. "Pamidronic acid" can be administered, e.g. in the form as it is marketed, e.g. under the trademark AREDIA(TM). "Alendronic acid" can be administered, e.g., in the form as it is marketed, e.g. under the trademark FOSAMAX. "Ibandronic acid" can be administered, e.g., in the form as it is marketed, e.g. under the trademark BONDRANAT. "Risedronic acid" can be administered, e.g., in the form as it is marketed, e.g. under the trademark ACTONEL. "Zoledronic acid" can be administered, e.g. in the form as it is marketed, e.g. under the trademark ZOMETA.

The term "heparanase inhibitor" as used herein refers to compounds which target, decrease or inhibit heparin sulphate degradation. The term includes, but is not limited to, PI-88. The term "biological response modifier" as used herein refers to a lymphokine or interferons, e.g. interferon [gamma].

The term "inhibitor of Ras oncogenic isoforms" , e.g. H-Ras, K-Ras, or N-Ras, as used herein refers to compounds which target, decrease or inhibit the oncogenic activity of Ras e.g. a "farnesyl transferase inhibitor" , e.g. L-744832, DK8G557 or R115777 (Zarnestra).

The term "telomerase inhibitor" as used herein refers to compounds which target, decrease or inhibit the activity of telomerase. Compounds which target, decrease or inhibit the activity of telomerase are especially compounds which inhibit the telomerase receptor, e.g. telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase are e.g. bengamide or a derivative thereof.

The term "proteasome inhibitor" as used herein refers to compounds which target, decrease or inhibit the activity of the proteasome. Compounds which target, decrease or inhibit the activity of the proteasome include e.g. PS-341 and MLN 341.

The term "matrix metalloproteinase inhibitor" or ("MMP inhibitor" ) as used herein includes, but is not limited to collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS- 279251, BAY 12-9566, TAA211 , MMI270B or AAJ996.

The term "agents used in the treatment of hematologic malignancies" as used herein includes, but is not limited to FMS-like tyrosine kinase inhibitors e.g. compounds targeting, decreasing or inhibiting the activity of Flt-3; interferon, 1-b-D-arabinofuransylcytosine (ara-c) and bisulfan; and ALK inhibitors e.g. compounds which target, decrease or inhibit anaplastic lymphoma kinase.

The term "compounds which target, decrease or inhibit the activity of Flt-3" are especially compounds, proteins or antibodies which inhibit Flt-3, e.g. PKC412, midostaurin, a stauro- sporine derivative, SU11248 and MLN518. The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteasome pathway. Compounds targeting, decreasing or inhibiting the intrinsic ATPase activity of HSP90 are especially compounds, proteins or antibodies which inhibit the ATPase activity of HSP90 e.g.,17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to trastu- zumab (Herceptin(TM)), Trastuzumab-DM1 , bevacizumab (Avastin(TM)), rituximab (Rituxan(R)), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), compounds of formula I can be used in combination with standard leukemia therapies, especially in combination with therapies used for the treatment of AML. In particular, compounds of formula I can be administered in combination with e.g. famesyl transferase inhibitors and/or other drugs useful for the treatment of AML, such as Daunorubicin, Adriamycin, Ara-C, VP-16, Teniposide, Mitoxantrone, Idarubi- cin, Carboplatinum and PKC412.

The structure of the active agents identified by code nos., generic or trade names may be taken from the actual edition of the standard compendium "The Merck Index" or from databases, e.g. Patents International (e.g. IMS World Publications).

The above-mentioned compounds, which can be used in combination with a compound of the formula I, can be prepared and administered as described in the art such as in the documents cited above.

A compound of the formula I may also be used to advantage in combination with known therapeutic processes, e.g., the administration of hormones or especially radiation.

The invention also relates to the use of a compound of the formula I, or a (preferably pharmaceutically acceptable) salt thereof, as a radiosensitizer, especially for the treatment of tumors which exhibit poor sensitivity to radiotherapy. By "combination", there is meant either a fixed combination in one dosage unit form, or a kit of parts for the combined administration where a compound of the formula I and a combination partner may be administered independently at the same time or separately within time intervals that especially allow that the combination partners show a cooperative, e.g. synergistic, effect, or any combination thereof.

The invention also relates to the use of a compound of the formula I, or a (preferably pharmaceutically acceptable) salt thereof, in the manufacture of pharmaceutical preparations useful in the treatment of the diseases identified herein, pharmaceutical preparations, especially useful against said diseases, comprising a compound of the formula I, or a pharmaceutically acceptable salt thereof and at least one pharmaceutically acceptable carrier, a compound of the formula I, or a pharmaceutically acceptable salt thereof, for use in the treatment of the animal or human body, especially against a disease mentioned above as particular examples, to a method of treatment of the animal or human body comprising administering a compound of the formula I, or a pharmaceutically acceptable salt thereof, to an animal or human, especially to a patient in need of such treatment in an amount effective for the treatment of said disease, and to a process for the manufacture of a compound of the formula I, or a (preferably pharmaceutically acceptable) salt thereof.

### Preferred embodiments according to the invention:

In the preferred embodiments as well as in preceding and following embodiments of more general scope, also in the claims, any one or more or all general expressions can be replaced by the corresponding more specific definitions provided above and below, thus yielding stronger preferred embodiments of the invention.

The invention relates especially to a compound of the formula I, or a salt thereof, as defined in the claims, more preferably the dependent compound / salt claims.

The invention also especially relates to a pharmaceutical preparation comprising a compound of the formula I or a pharmaceutically acceptable salt thereof, according to the main compound/salt claim or a dependent compound/salt claim and at least one pharmaceutically acceptable carrier.

The invention also preferably relates to a compound of the formula I or a salt thereof, according to the main compound/salt claim or a dependent compound/salt claim for use in the diagnostic or therapeutic treatment of the animal or human body, especially in the treatment as defined herein or in the claims.

The invention also relates to other embodiments regarding the uses and methods mentioned in the claims, where dependent claims describe preferred embodiments.

All the claims are therefore included by reference herein.

The invention relates especially to a compound of the formula I given in the Examples, or a pharmaceutically acceptable salt thereof, or its use according to the invention, as well as the processes and novel starting materials and intermediates mentioned in the Examples.

### Examples

The following examples illustrate the invention without limiting the scope thereof.

Temperatures are measured in degrees Celsius. Unless otherwise indicated, the reactions take place at room temperature

The analytical conditions are as follow:
Column: Column Engineering Inc., Intersil, 3[mu]m ODS-3 50x20 mm
LC-MS machine: Shimadzu Analytical HPLC SCL10Avp, autosampler Gilson 215 with ELSD (evaporative light scattering detector)
Sedex75, mass-spectrometer PE SCIEX 150, Canada.
Detection by UV absorption at 215 and 254nm. The column temperature is 35 °C and the retention times (tR) are given in minutes. Flow rate: 0.45 mL/min. Gradient:

| | |
|---|---|
| Solvent system: | Solvent A: water(0.05 % TFA), |
| | solvent B: acetonitrile (0.05 % TFA) |

| Eluation gradient: | |
|---|---|
| | 0.01 min - controller start |
| | 4.00 min - pump B% - 95.0 |
| | 5.50 min - pump B% - 95.0 |
| | 5.90 min - pump B% - 5.0 |
| | 6.00 min - controller stop |
| Total run time: | 6.00 min |

### Abbreviations:

- t_{Ret}: retention time
- RT: room temperature
- TFA: trifluoro acetic acid
- THF: tetrahydrofurane
- POCl3: phosphoroxychloride
- PCl5: phosphorpentachloride
- h: hours
- min: minutes
- mL: milliliter
- TLC: thin layer chromatography
- UV: Ultraviolet

### Example 1: 1-methyl-N6-phenyl-N4-o-tolyl-1H-pyrazolo[3,4-d]pyrimidine-4,6-diamine (42F11)

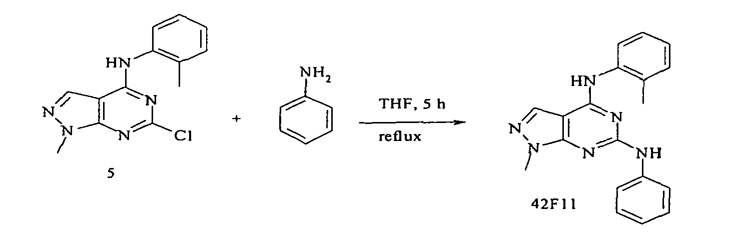

1.37 g (5 mmol) of 6-chloro-1-methyl-N-o-tolyl-1H-pyrazolo[3,4-d]pyrimidin-4-amine (**5**; for preparation see Example 1.4) are dissolved in 15 mL of THF; 0.9 g (10 mmol) of aniline are added and the reaction mixture is stirred under reflux for 5 h. The solvent is removed under vacuum and the crude product dissolved in chloroform. The organic phase is washed with water, dried with sodium sulfate and evaporated. The residue is subjected to purification by column chromatography on silica-gel using hexane ÷ EtOAc = 5÷1 to 2÷1 as the mobile phase (TLC control). The title compound was obtained after evaporation of the target fraction followed by crystallization from hexane. LC-MS: t_{Ret}= 3.30 min; M+1 = 331.4.

### Example 1.1.: 5-amino-1-methyl-1H-pyrazole-4-carboxamide (2)

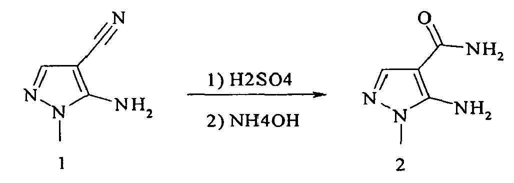

12.2 g (100 mmol) of 5-amino-1-methyl-1H-pyrazole-4-carbonitrile, **1** (Commercial supplier: BIONET-INTER) are dissolved in 50 mL of conc. sulfuric acid and stirred at RT for 3 h. The reaction mixture is cooled to 0-5° C and neutralized at that temperature by addition of aqueous ammonia. The water phase is extracted with chloroform, the solvent is evaporated and the residue crystallized from water to yield the title compound.

### Example 1.2.: 1-methyl-1H-pyrazolo[3,4-d]pyrimidine-4,6(5H,7H)-dione (3)

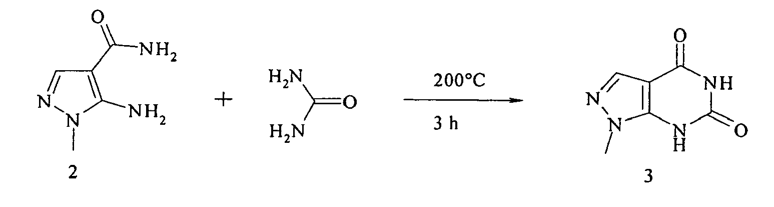

7 g (50 mmol) of 5-amino-1-methyl-1H-pyrazole-4-carboxamide (**2**) is mixed with an excess of urea (50 g) and the mixture is heated at 200°C for 3 h. The reaction cake is cooled to RT and the residue is dissolved in 500 mL of chloroform. The organic phase is washed with 5% aqueous acetic acid. The organic phase is separated and the solvent is evaporated. The title compound is purified by crystallization from water.

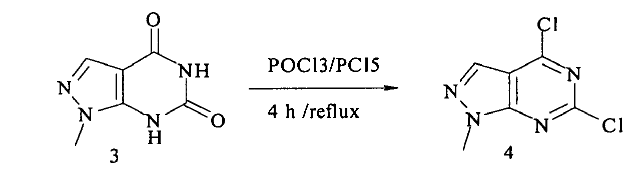

### Example 1.3.: 4,6-dichloro-1-methyl-1H-pyrazolo[3,4-d]pyrimidine (4)

6,6 g (39.75 mmol) of 1-methyl-1H-pyrazolo[3,4-d]pyrimidine-4,6(5H,7H)-dione (**3**) are heated at reflux in a mixture of 50 mL of POCl3 and 5 g of PCl5 for 4 h. The excess of POCl3 is evaporated under vacuum and to the residue a saturated ice-cold solution of sodium bicarbonate is added for neutralization. The aqueous phase is extracted with chloroform, dried with sodium sulfate, and the solvent evaporated. The residue is crystallized from hexane to give the title compound.

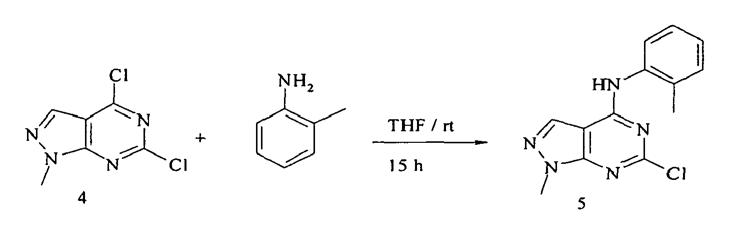

### Example 1.4.: 6-chloro-1-methyl-N-o-tolyl-1H-pyrazolo[3,4-d]pyrimidin-4-amine (5)

2.03 g (10 mmol) of 4,6-dichloro-1-methyl-1H-pyrazolo[3,4-d]pyrimidine (**4**) and 2.15 g (20 mmol) of 2-methylaniline are dissolved in 50 mL of THF. The reaction mixture is stirred at RT for 15 h. The solvent is evaporated under vacuum; the residue is dissolved in chloroform, washed with water, dried with sodium sulfate and evaporated. The residue is subjected to purification by column chromatography on silica-gel using hexane ÷ EtOAc = 8÷1 to 4÷1 as the mobile phase (TLC control). The title compound was obtained after evaporation of the target fraction followed by crystallization of thus obtained solid from hexane.

Starting from the intermediate 6-chloro-1-methyl-N-o-tolyl-1H-pyrazolo[3,4-d]pyrimidin-4-amine (**5**; see Example 1.4) and different amine building blocks, the compounds listed in the Table 1 are prepared according to the general synthetic protocol (see Example 1):

| Ex. | Amin | Product | LC-MS |
|---|---|---|---|
| 2 | 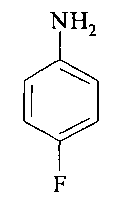 | 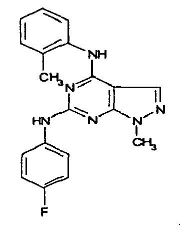 | t_{Ret}= 3.41 min; M+1 = 349.2 |
| 3 | 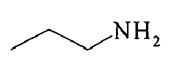 | 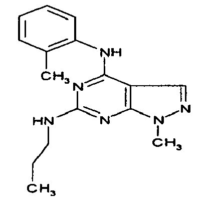 | t_{Ret=} 2.49 min; M+1 = 297.3 |
| 4 | 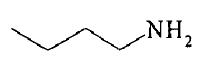 | 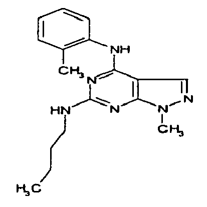 | t_{Ret}= 2.67 min; M+1 = 311.3 |
| 5 | 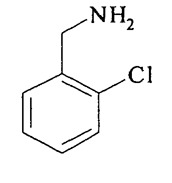 | 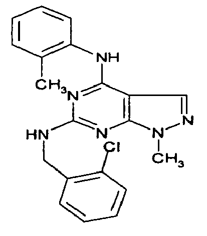 | M+1 = 378, 380 |
| 6 | 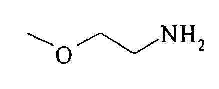 | 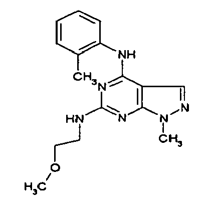 | t_{Ret=} 2.24 min; M+1 = 313.3 |
| 7 | 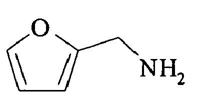 | 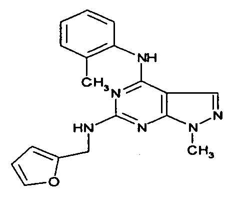 | t_{Ret}= 2.64 min; M+1 = 335.5 |
| 8 | 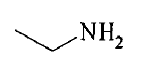 | 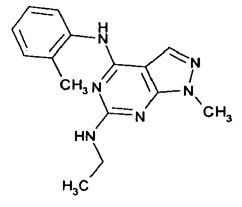 | t_{Ret}= 2.13 min; M+1 = 283.8 |
| 9 | 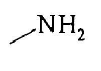 | 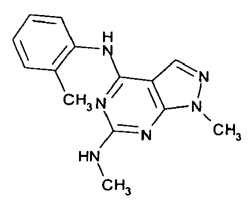 | t_{Ret}= 1.95 min; M+1 = 269.5 |

The following compounds are obtainable according to the general procedure as outlined herein using the appropriate starting materials.

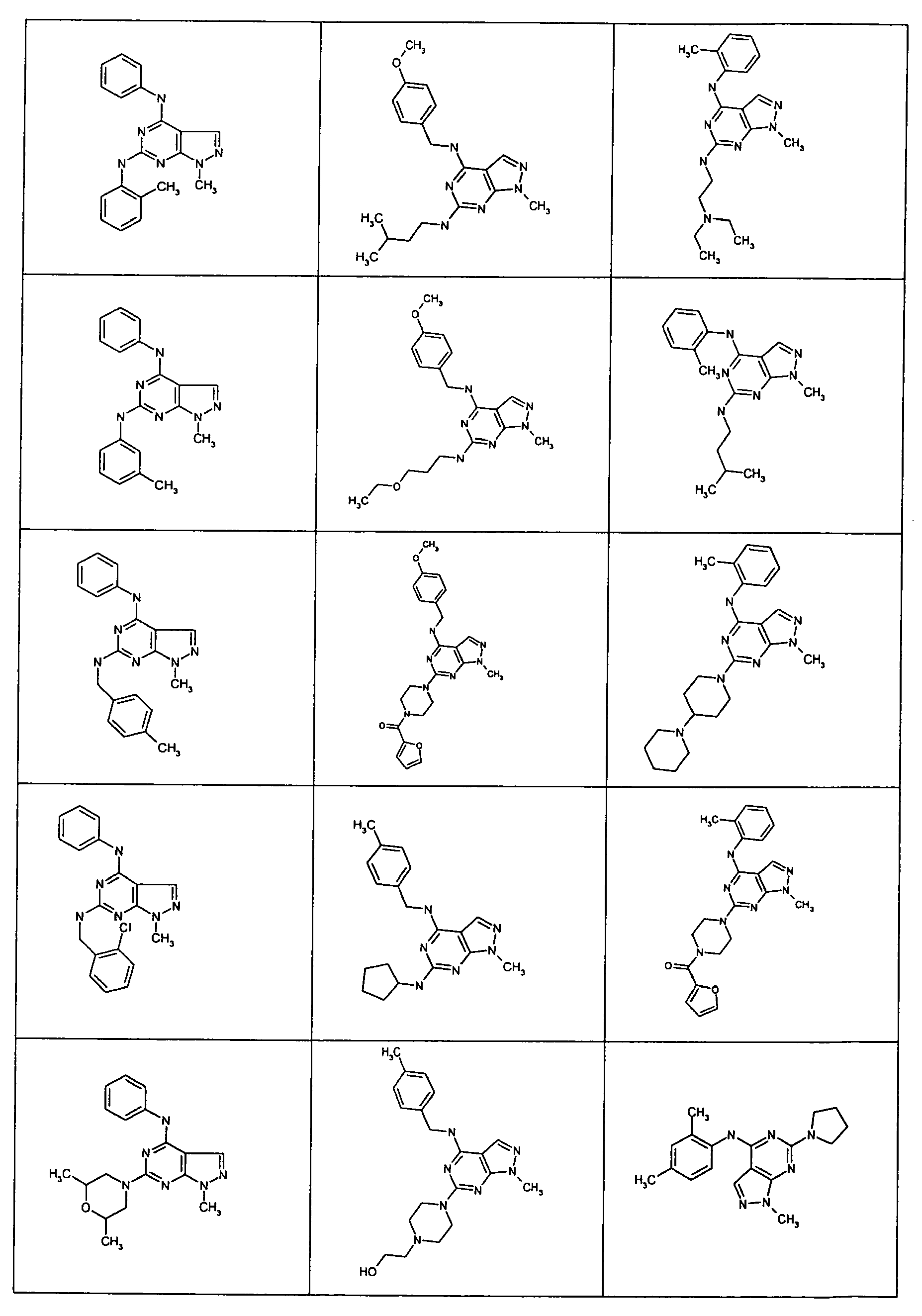

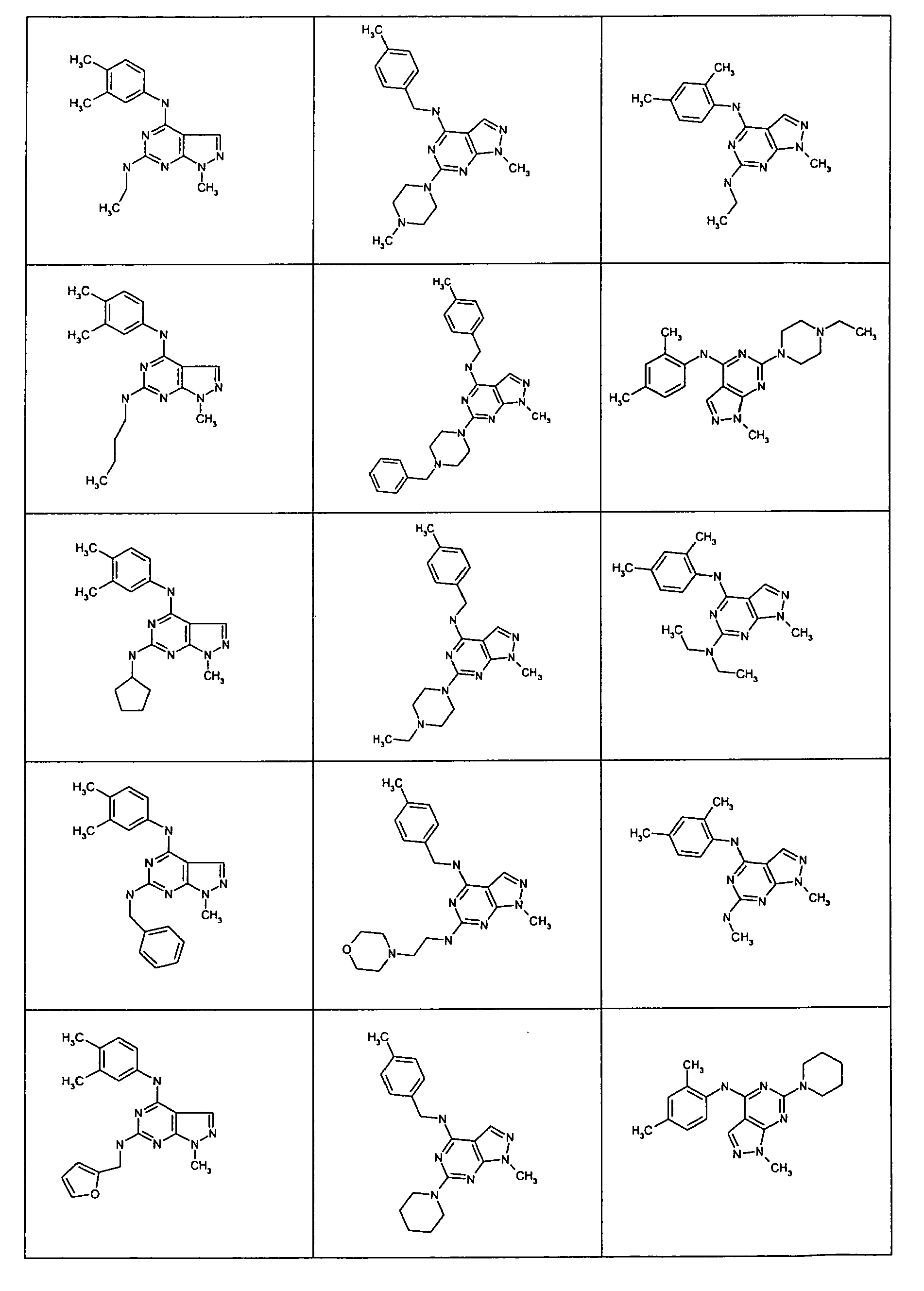

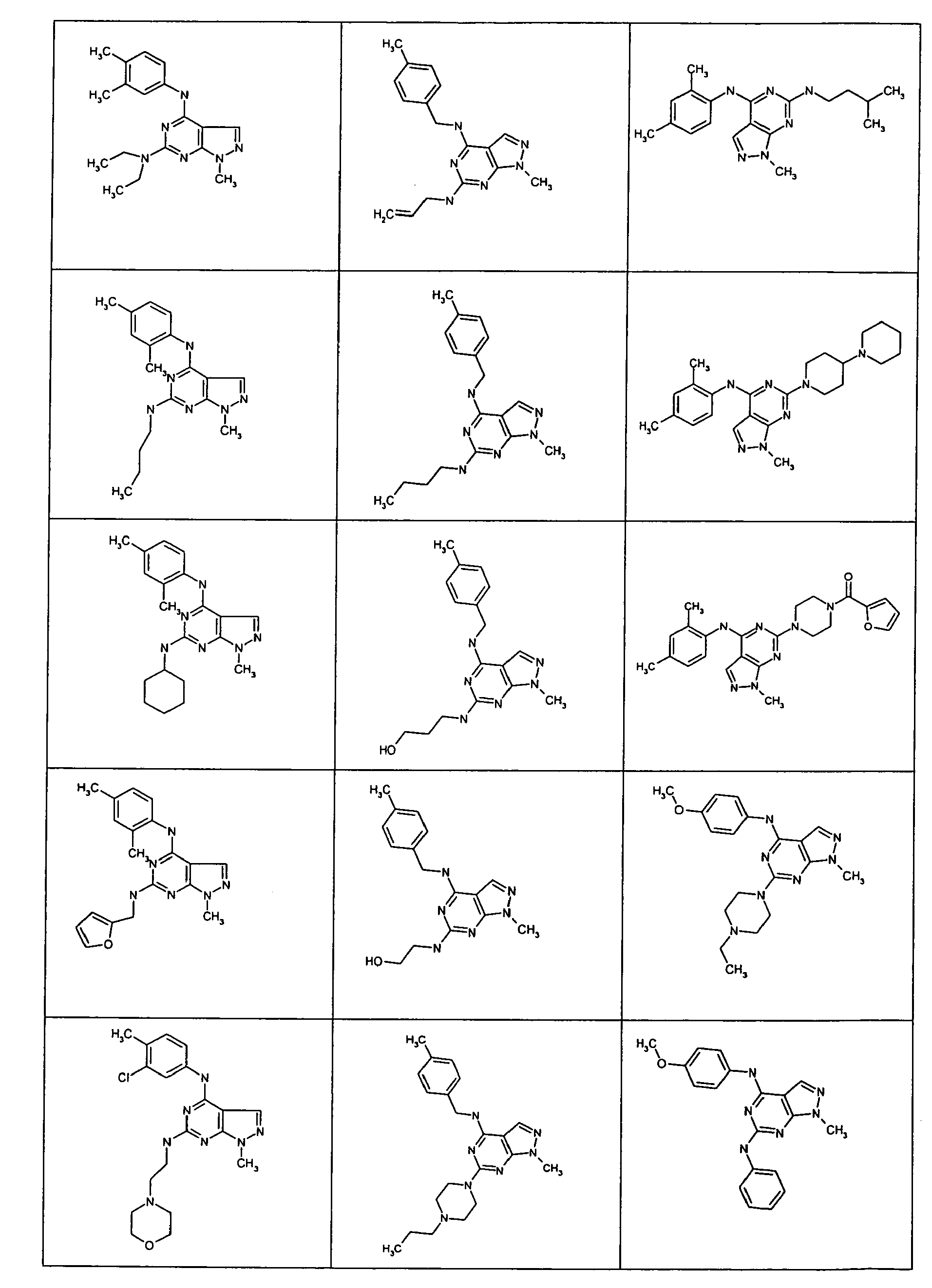

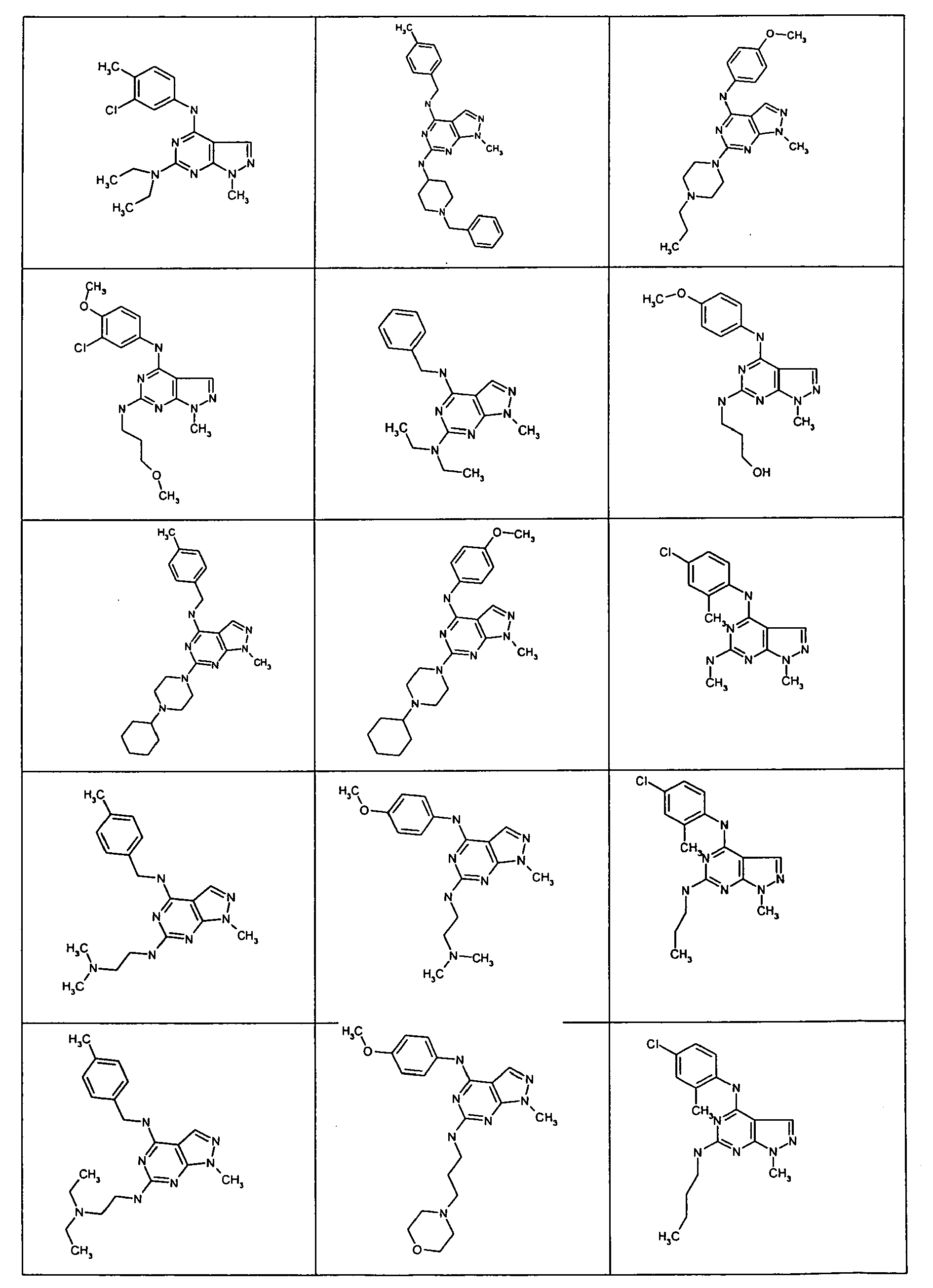

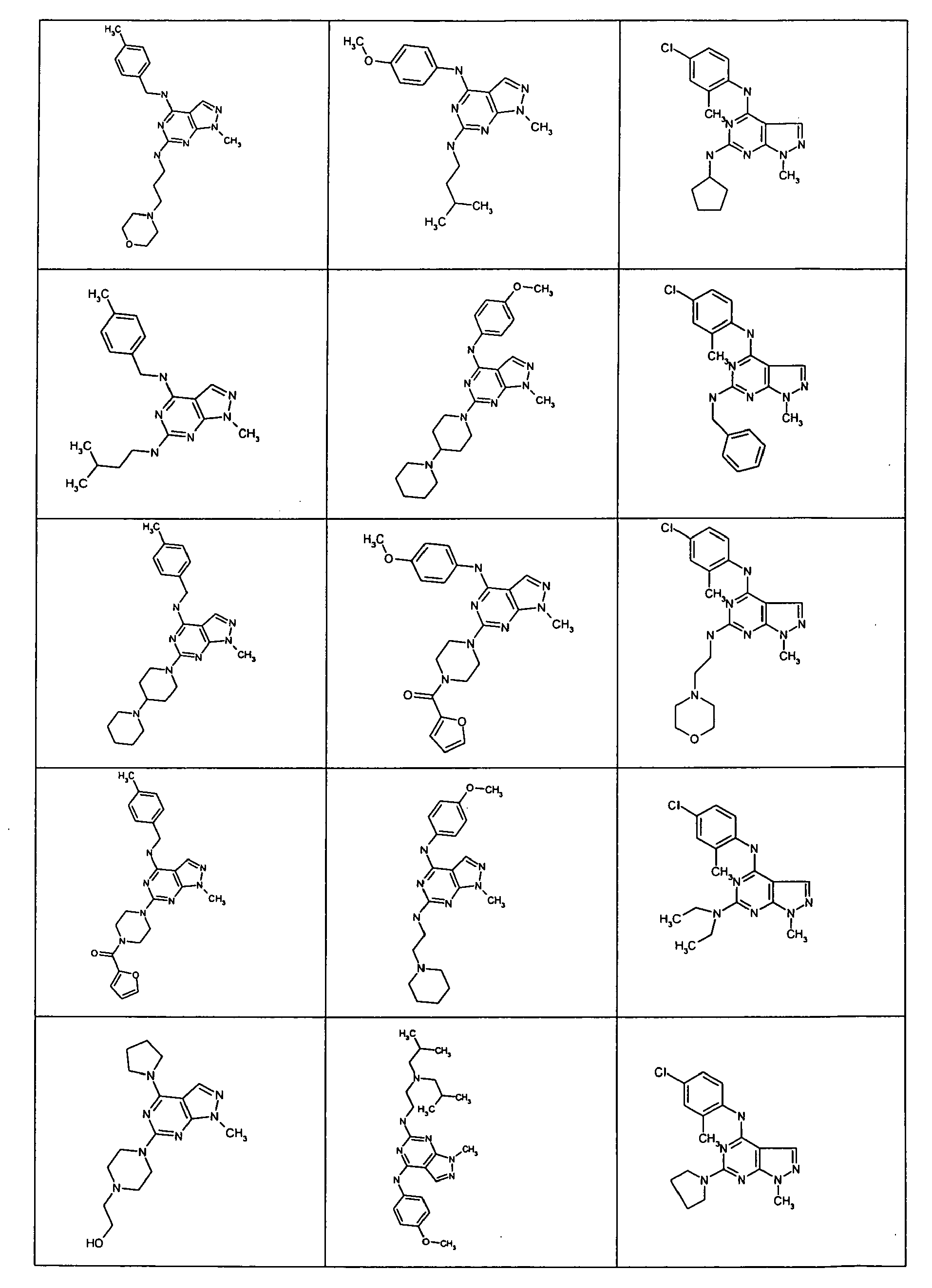

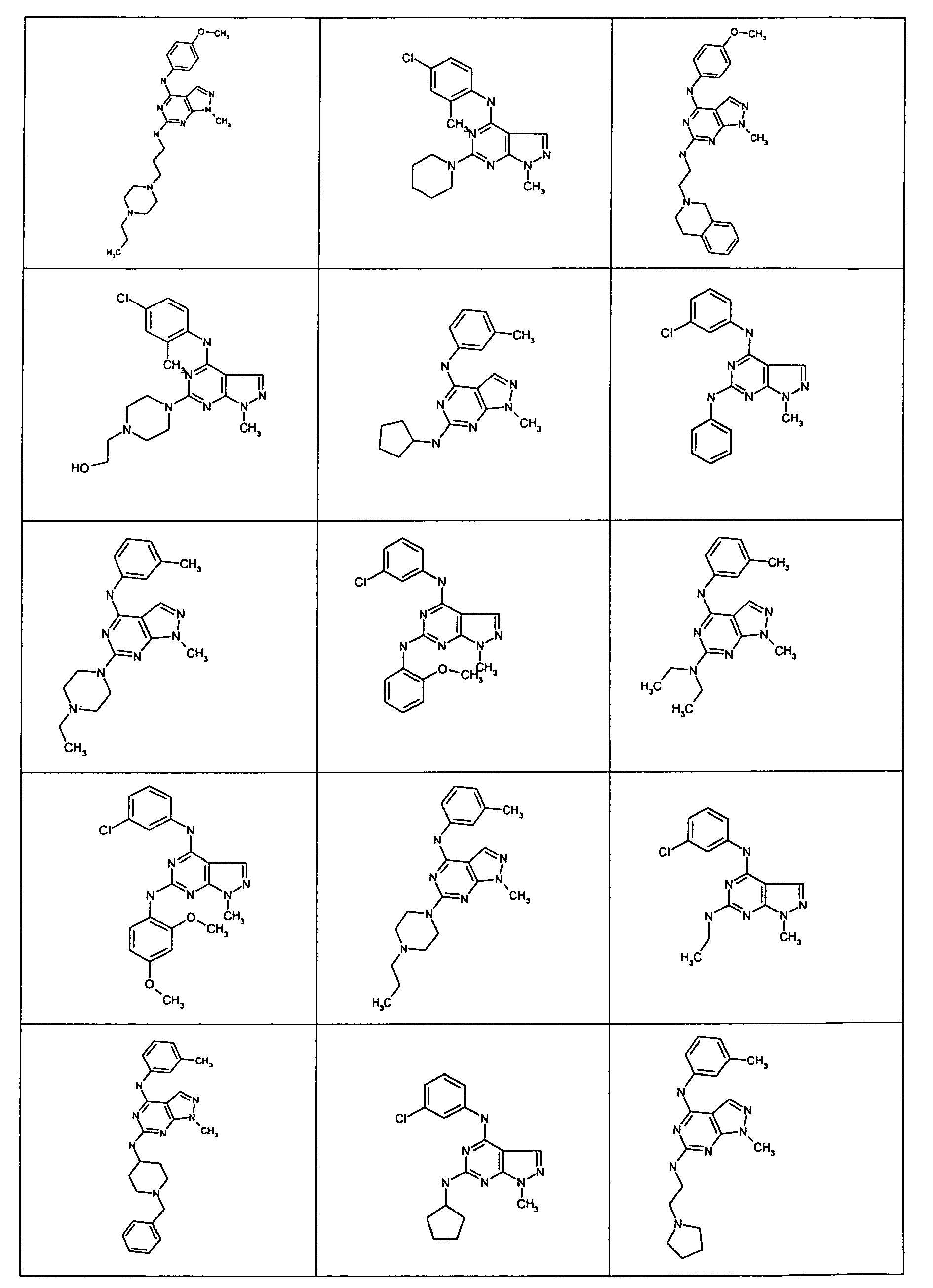

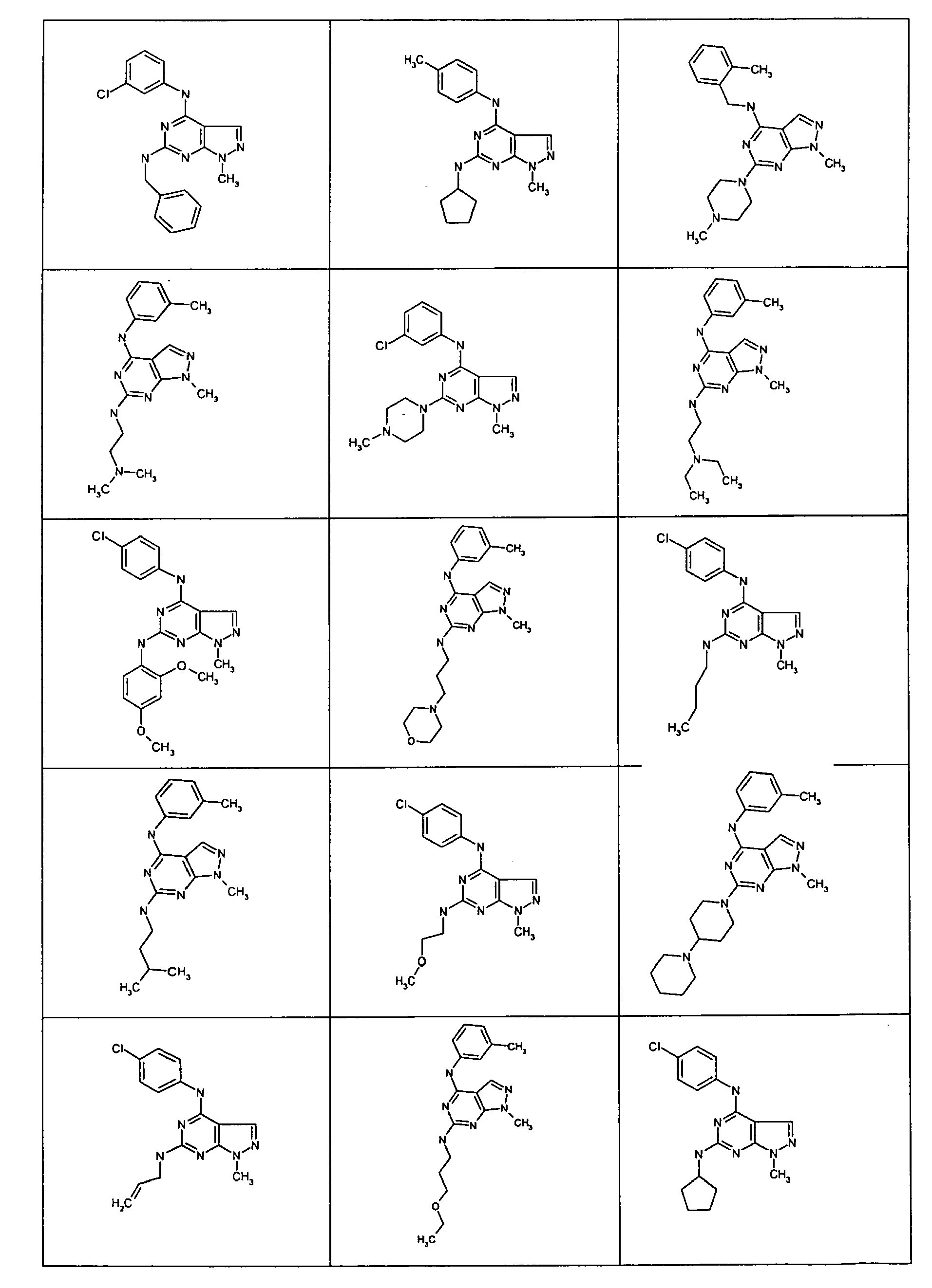

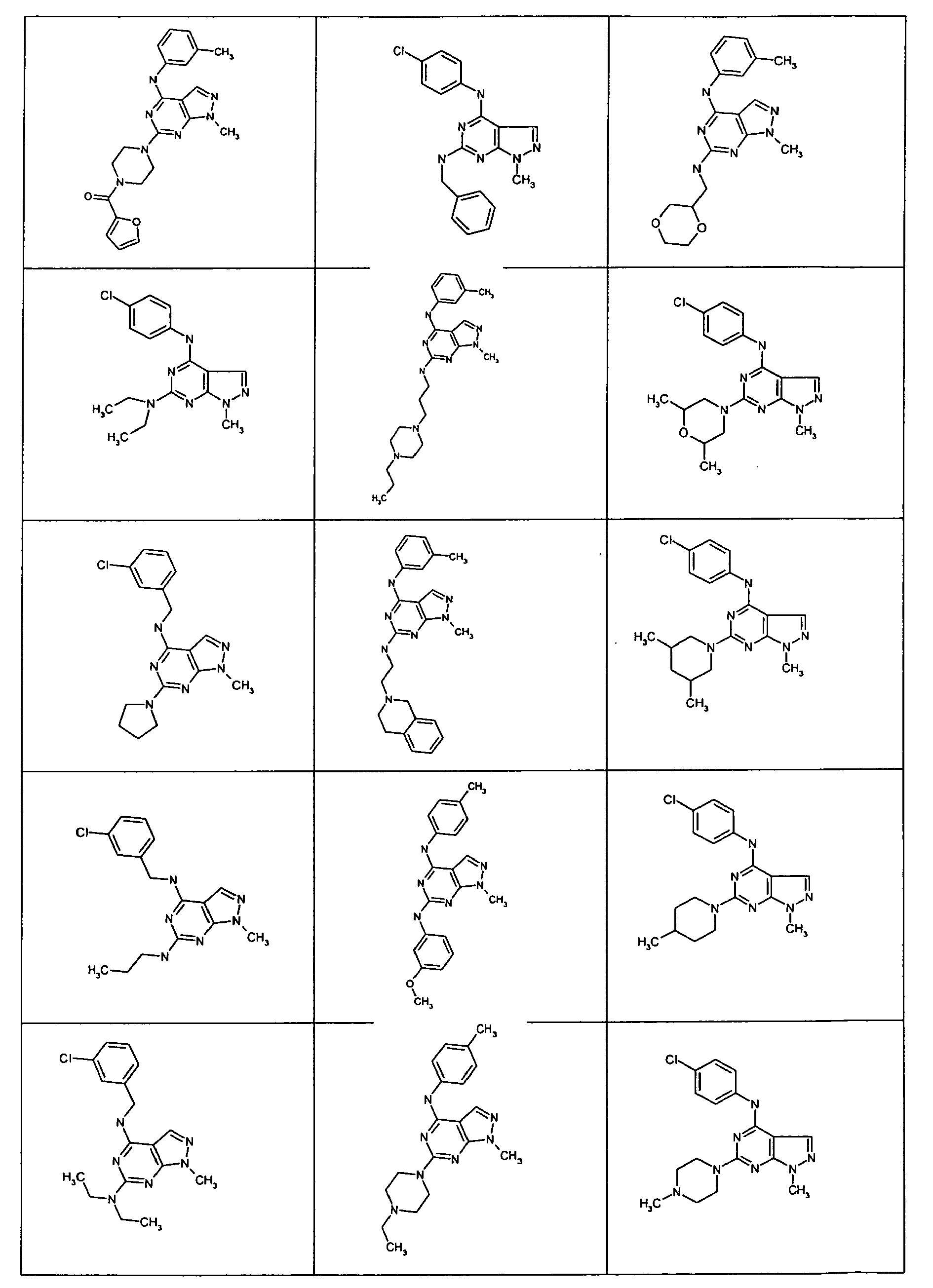

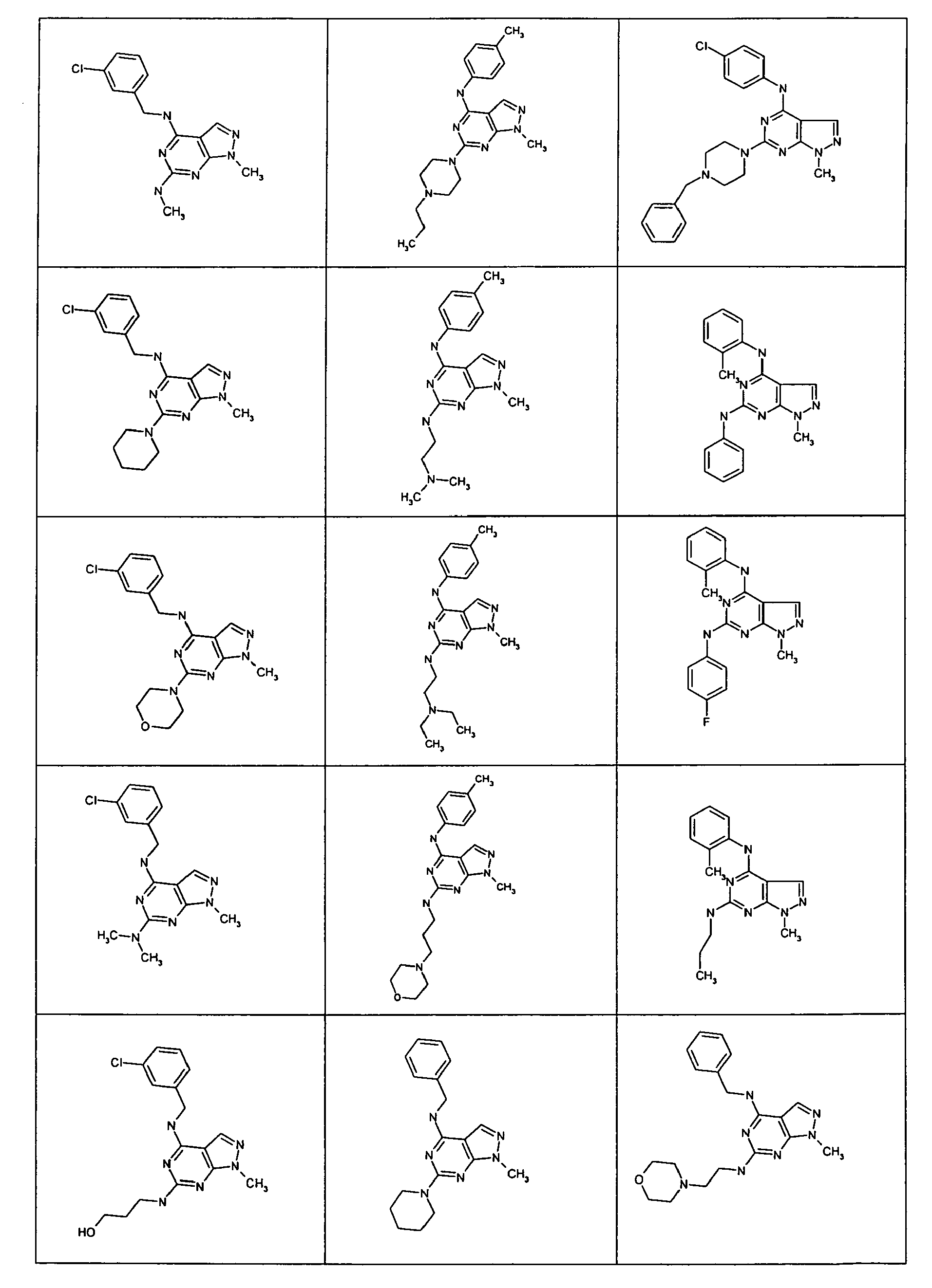

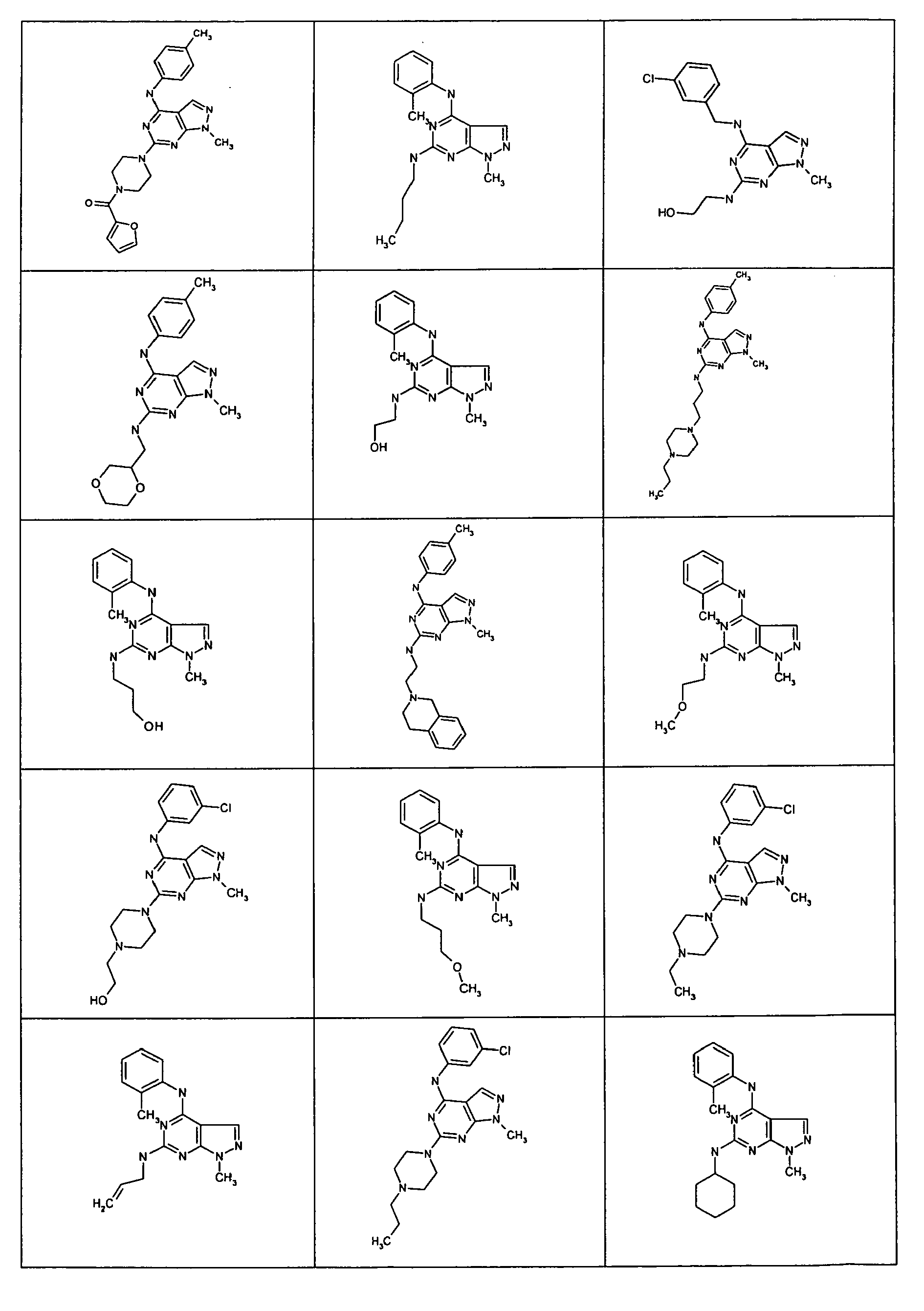

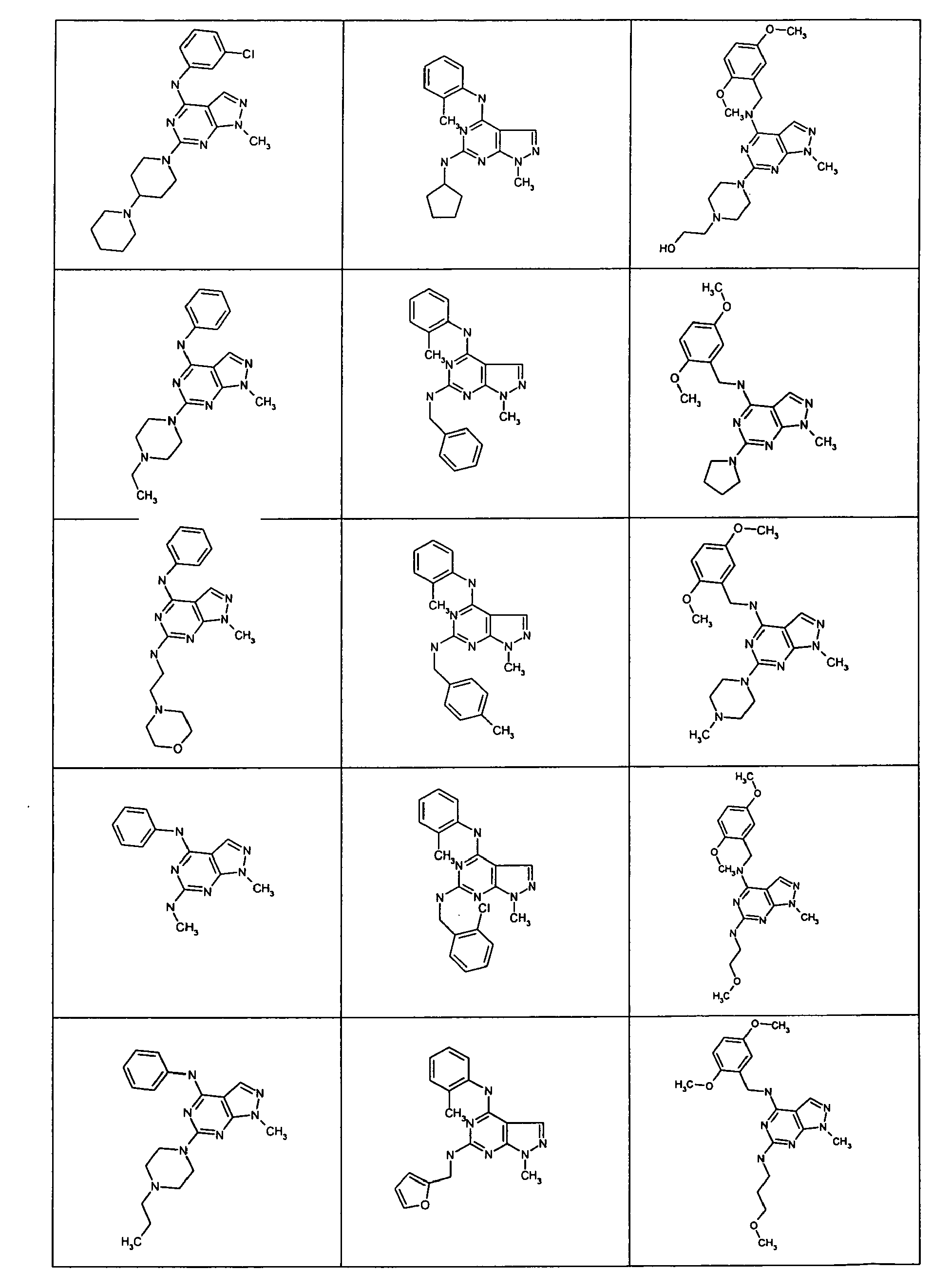

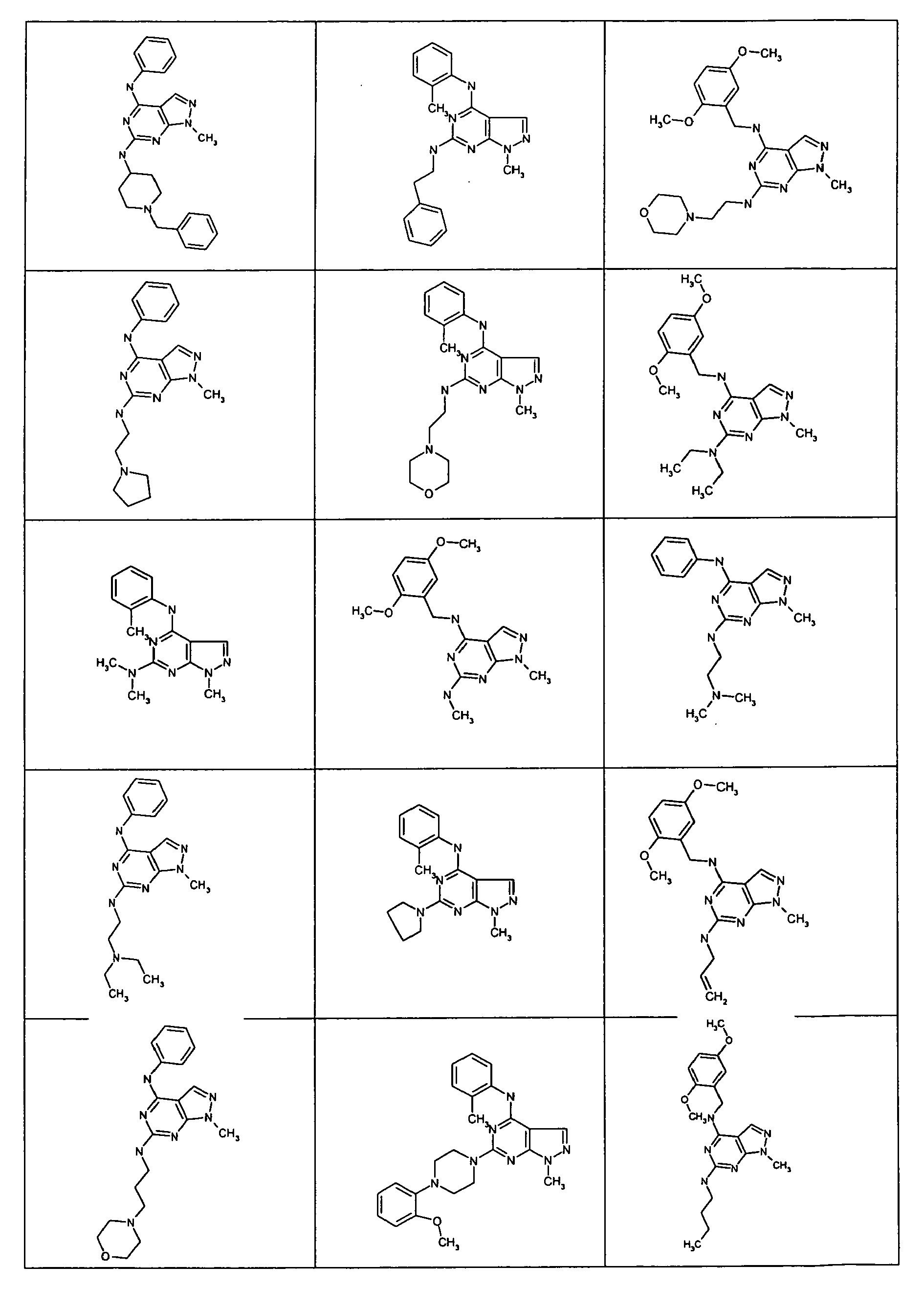

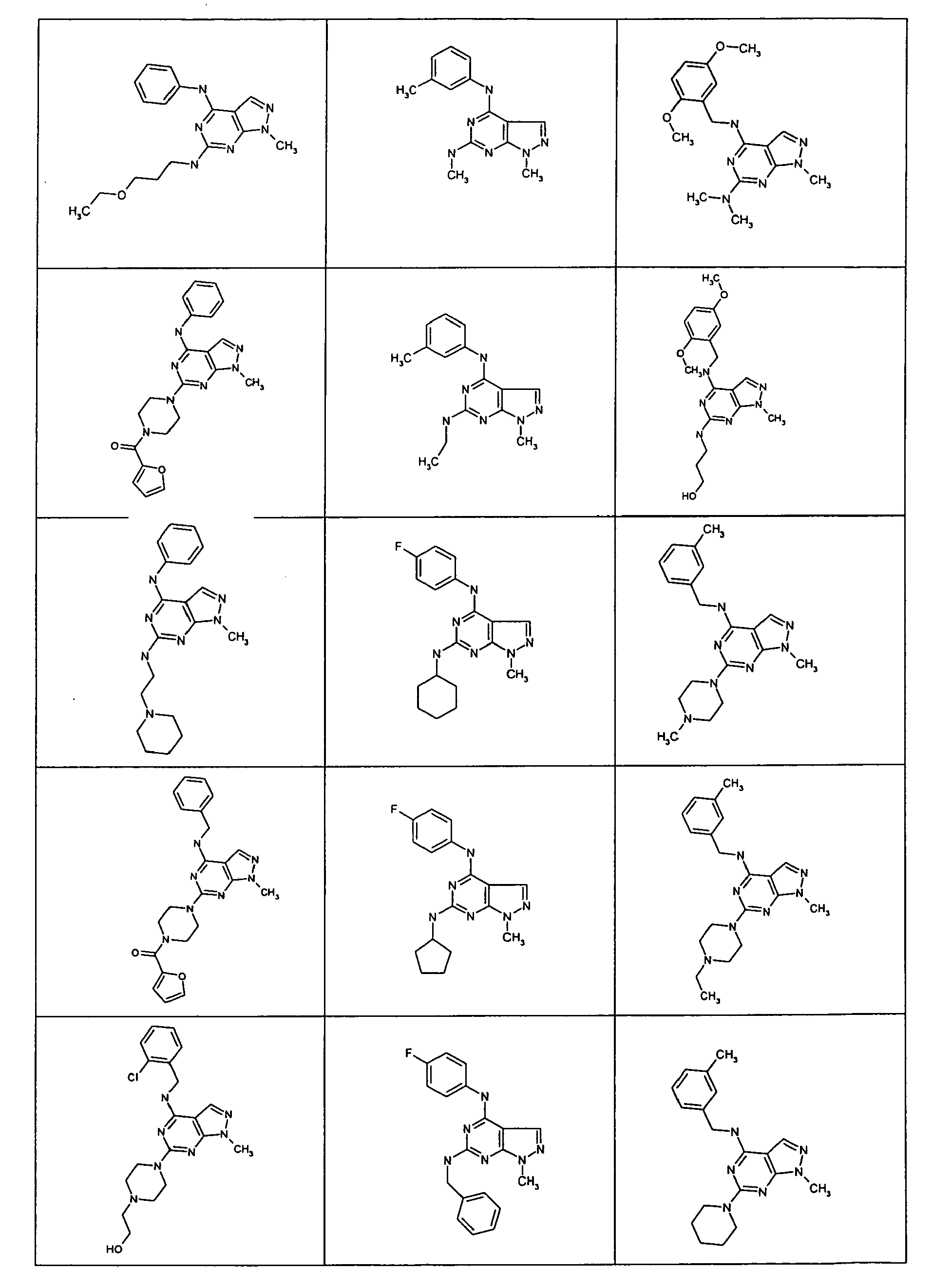

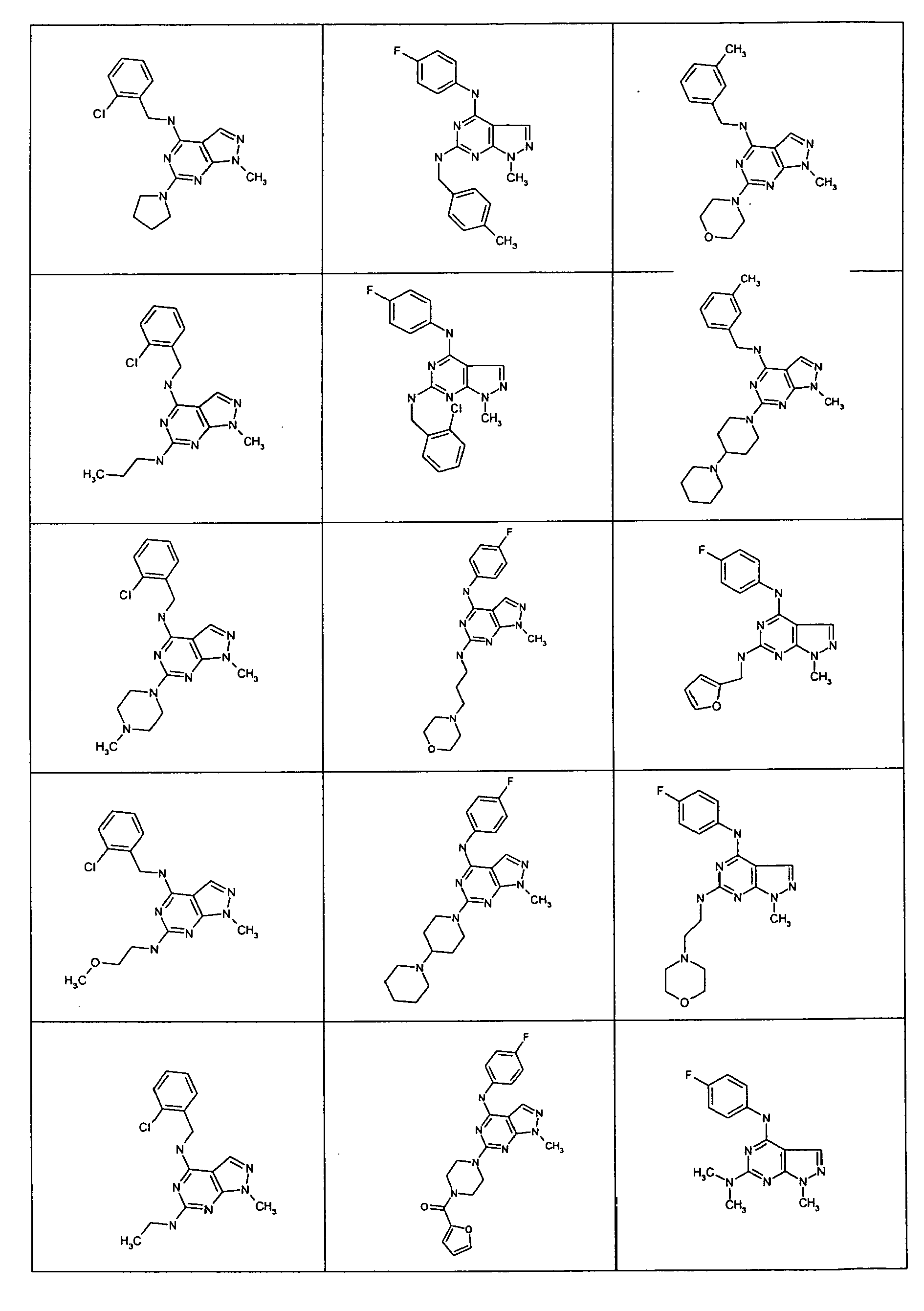

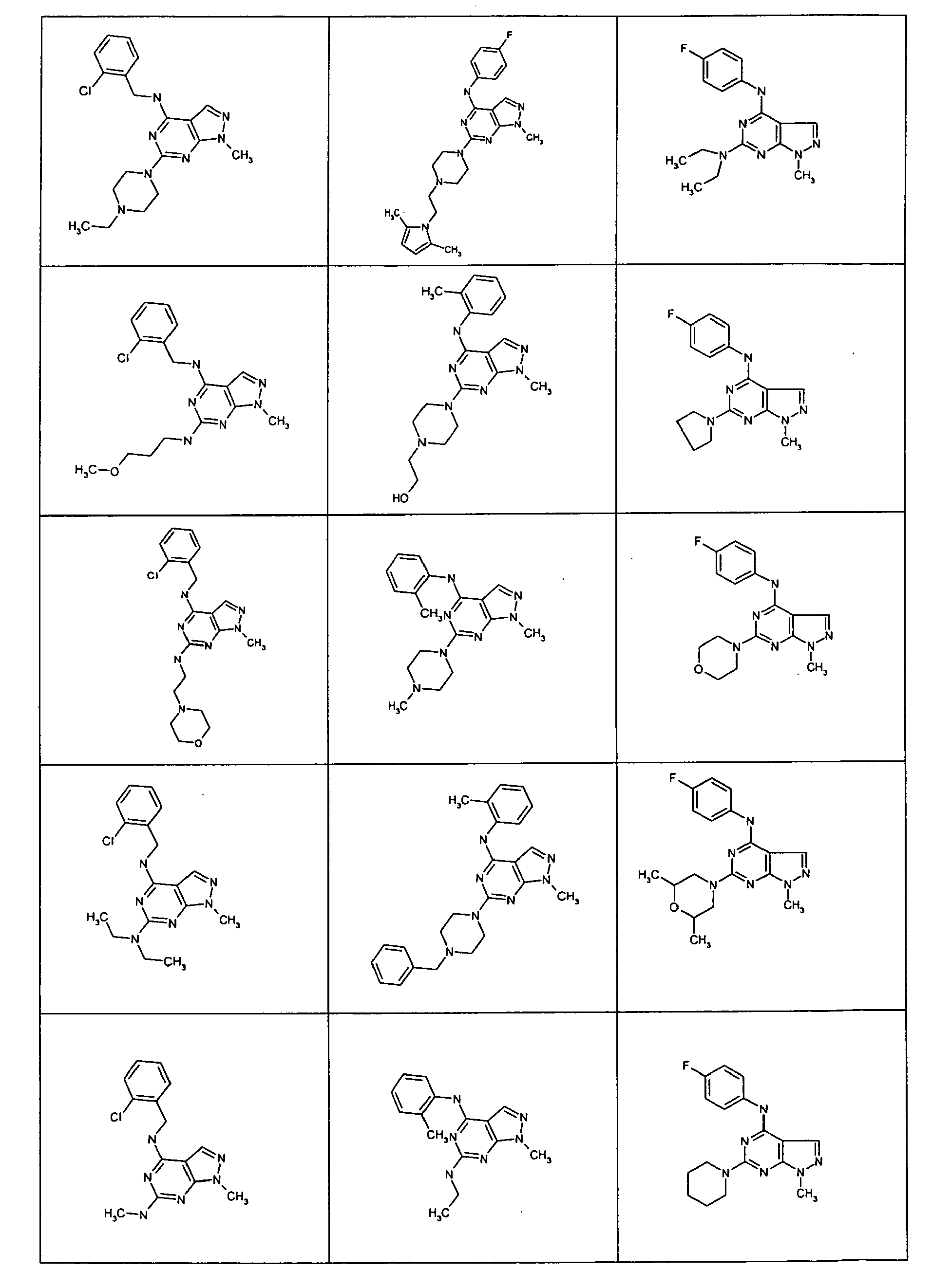

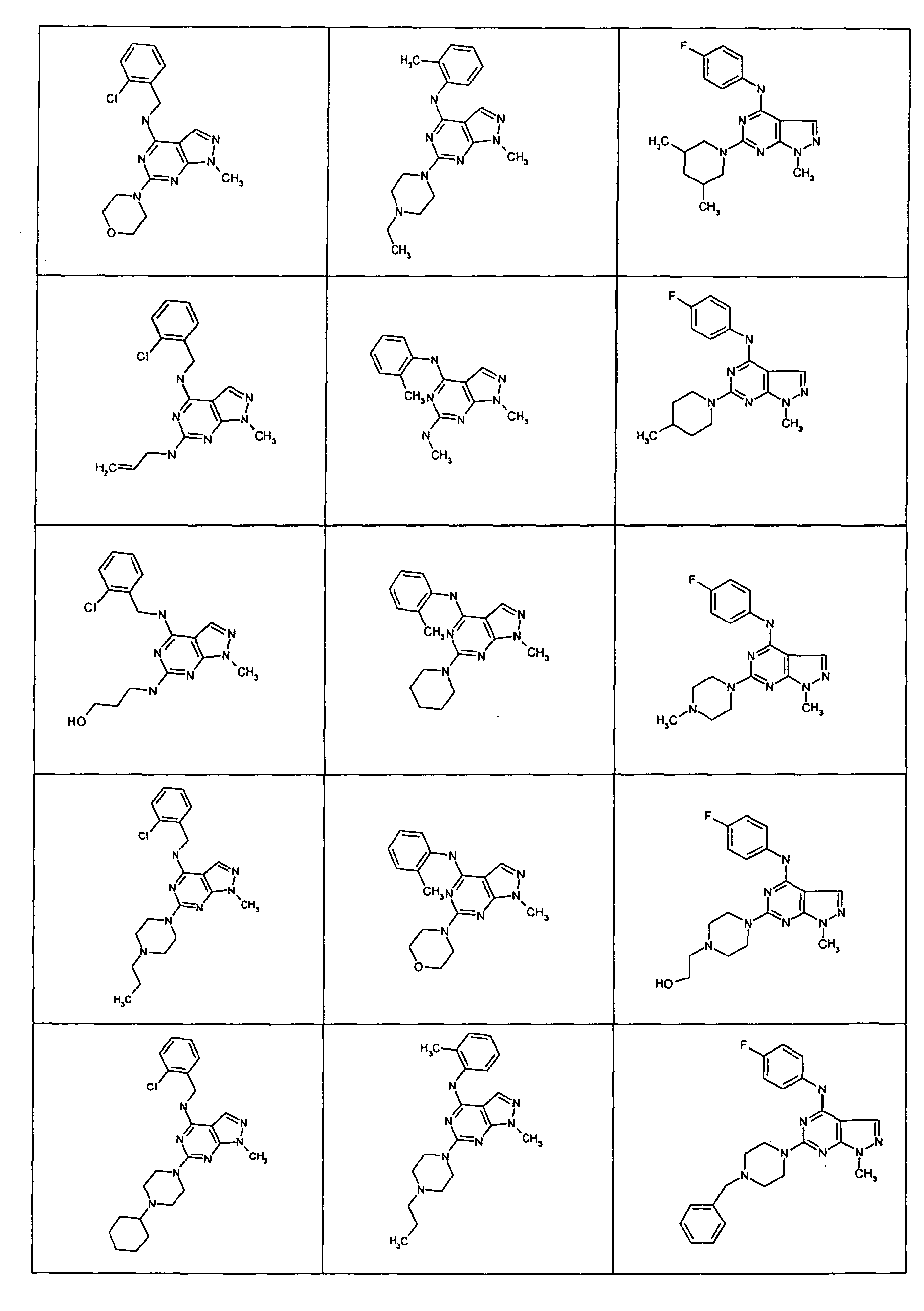

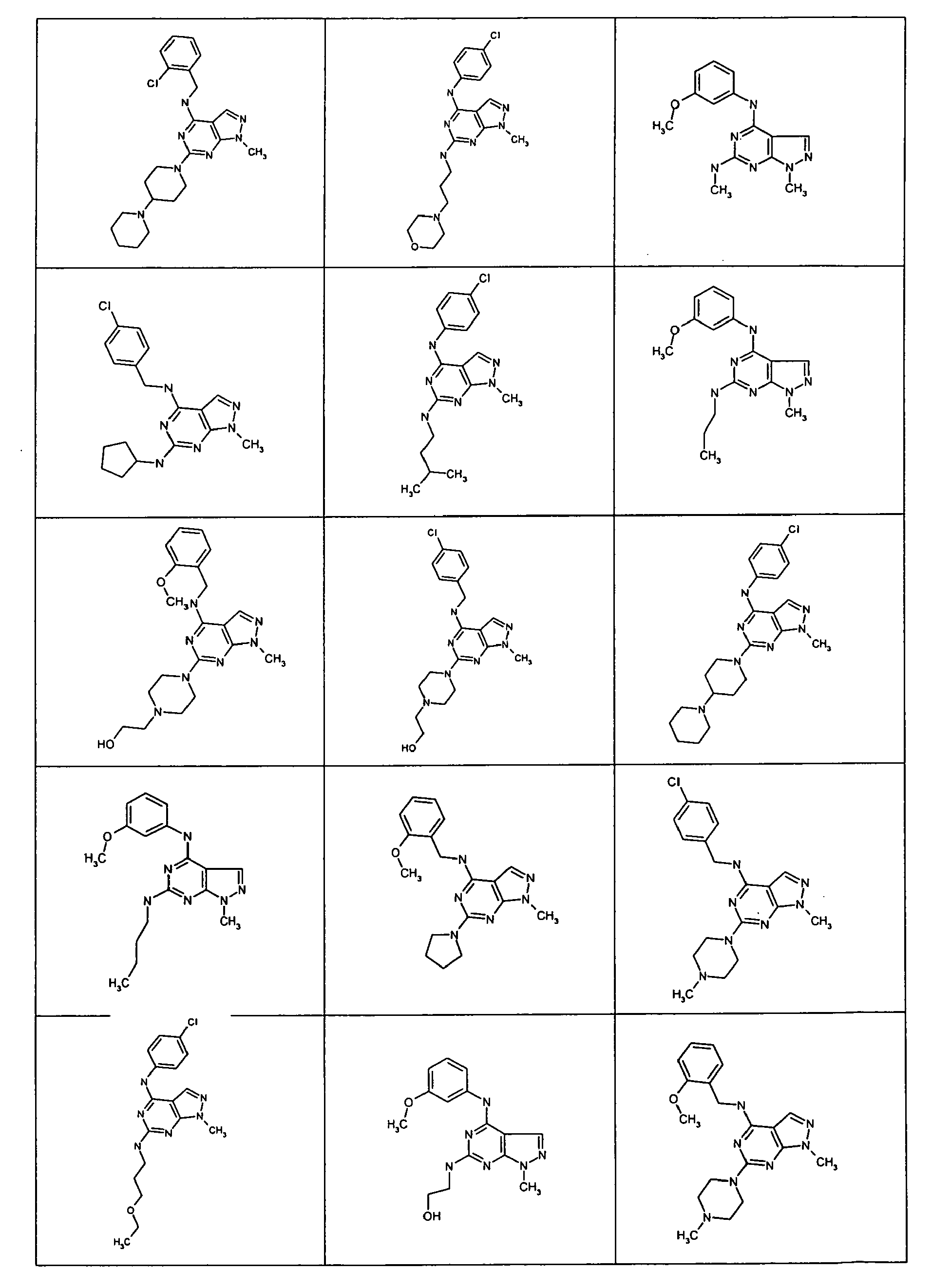

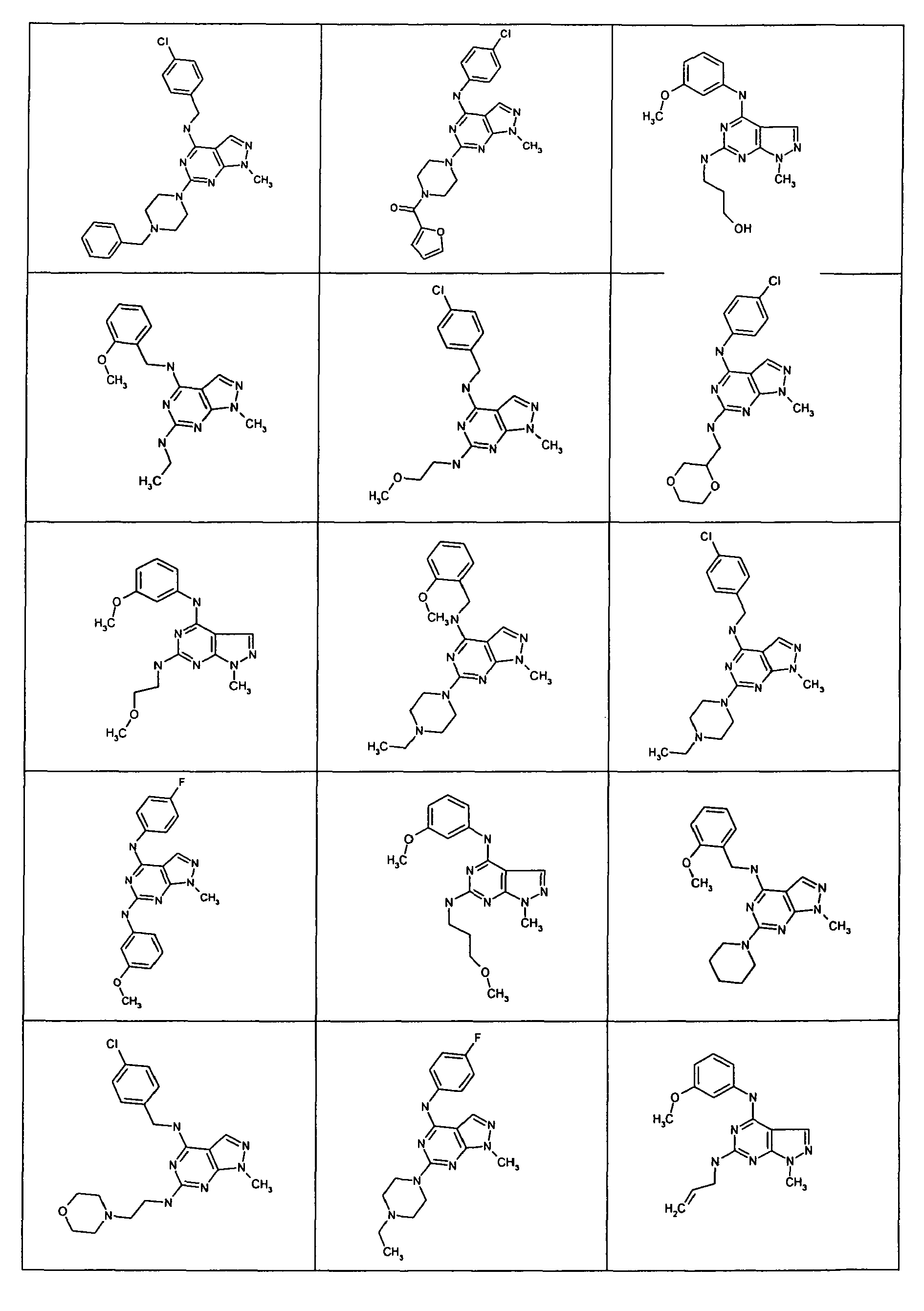

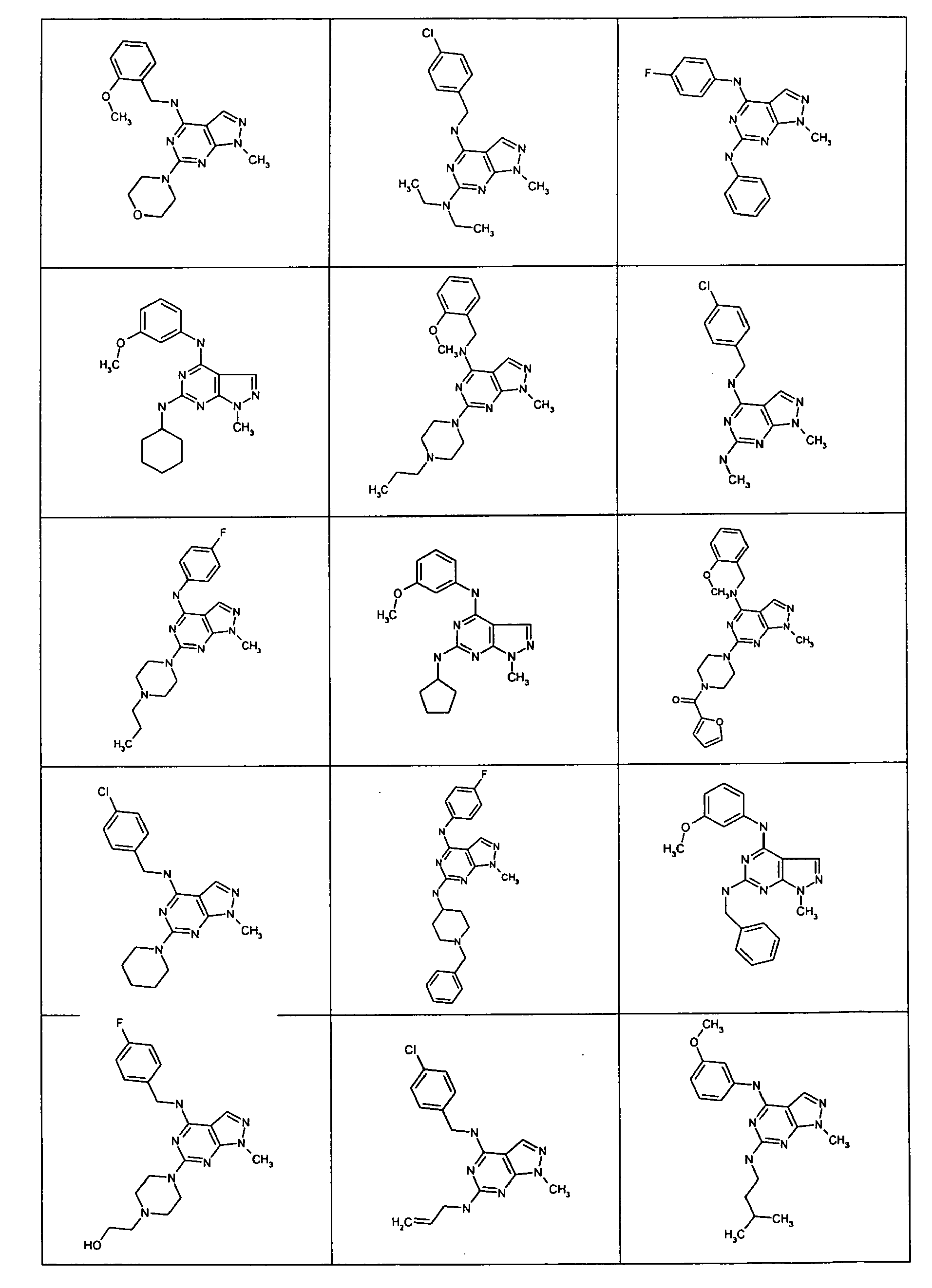

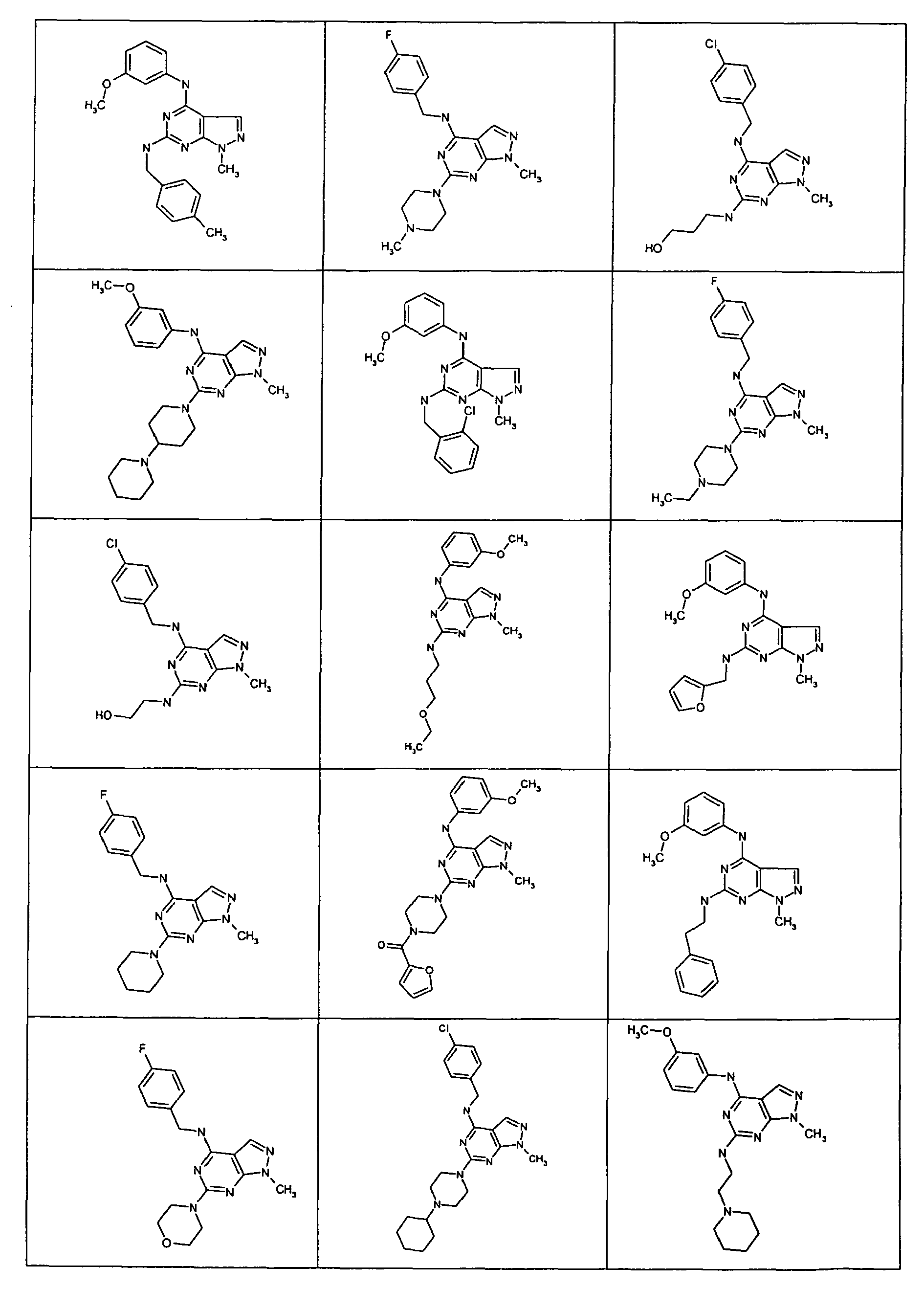

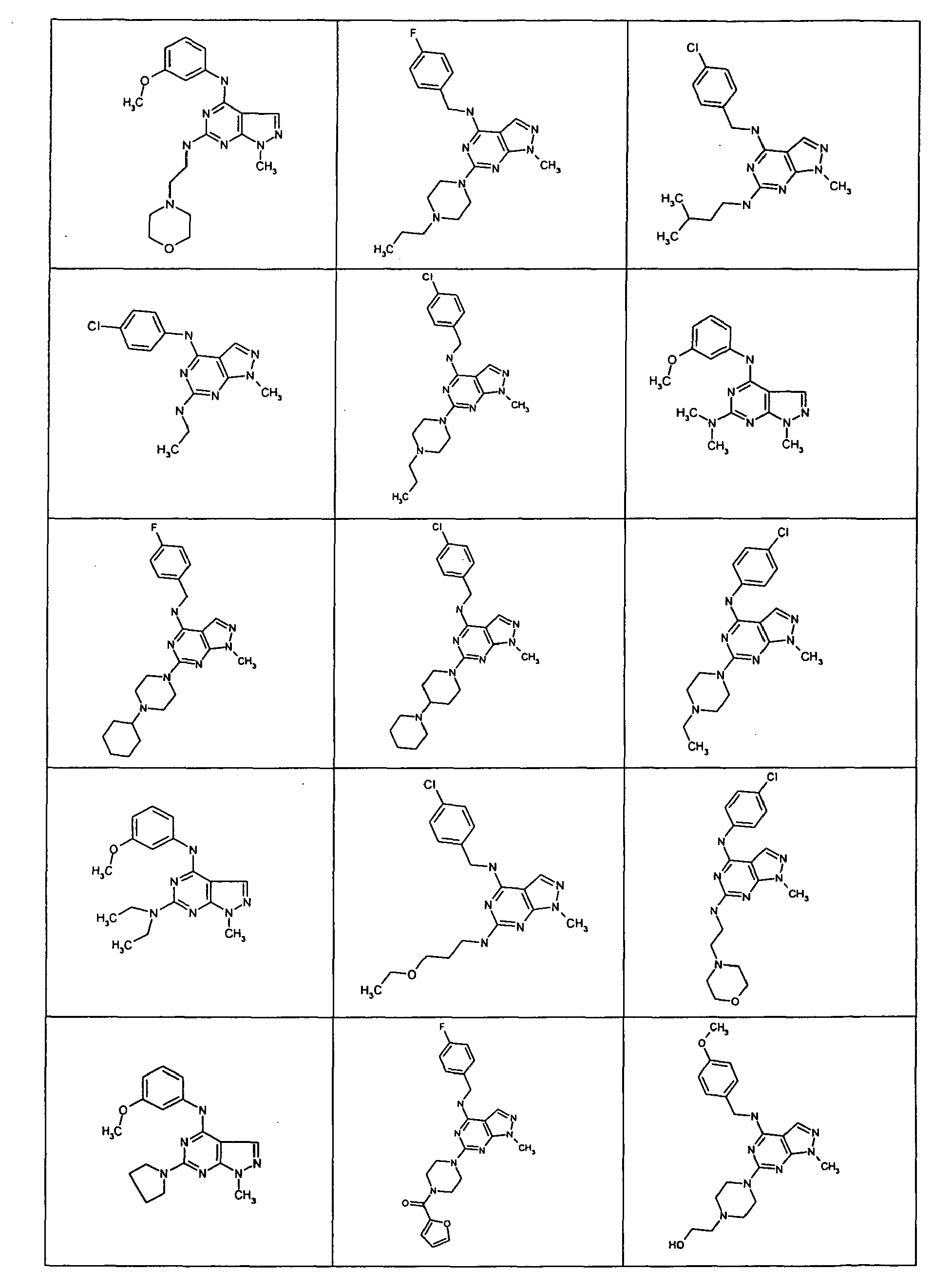

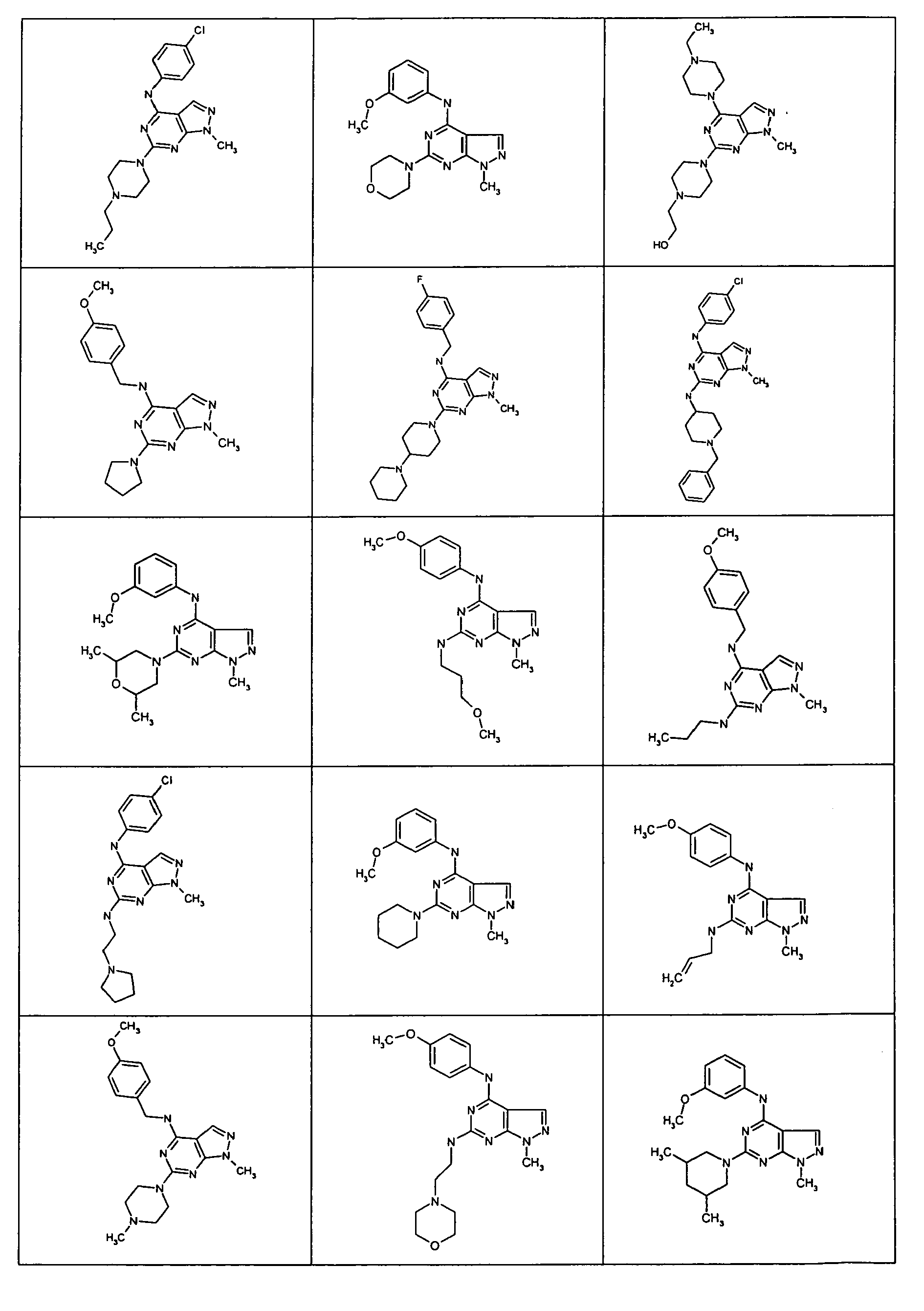

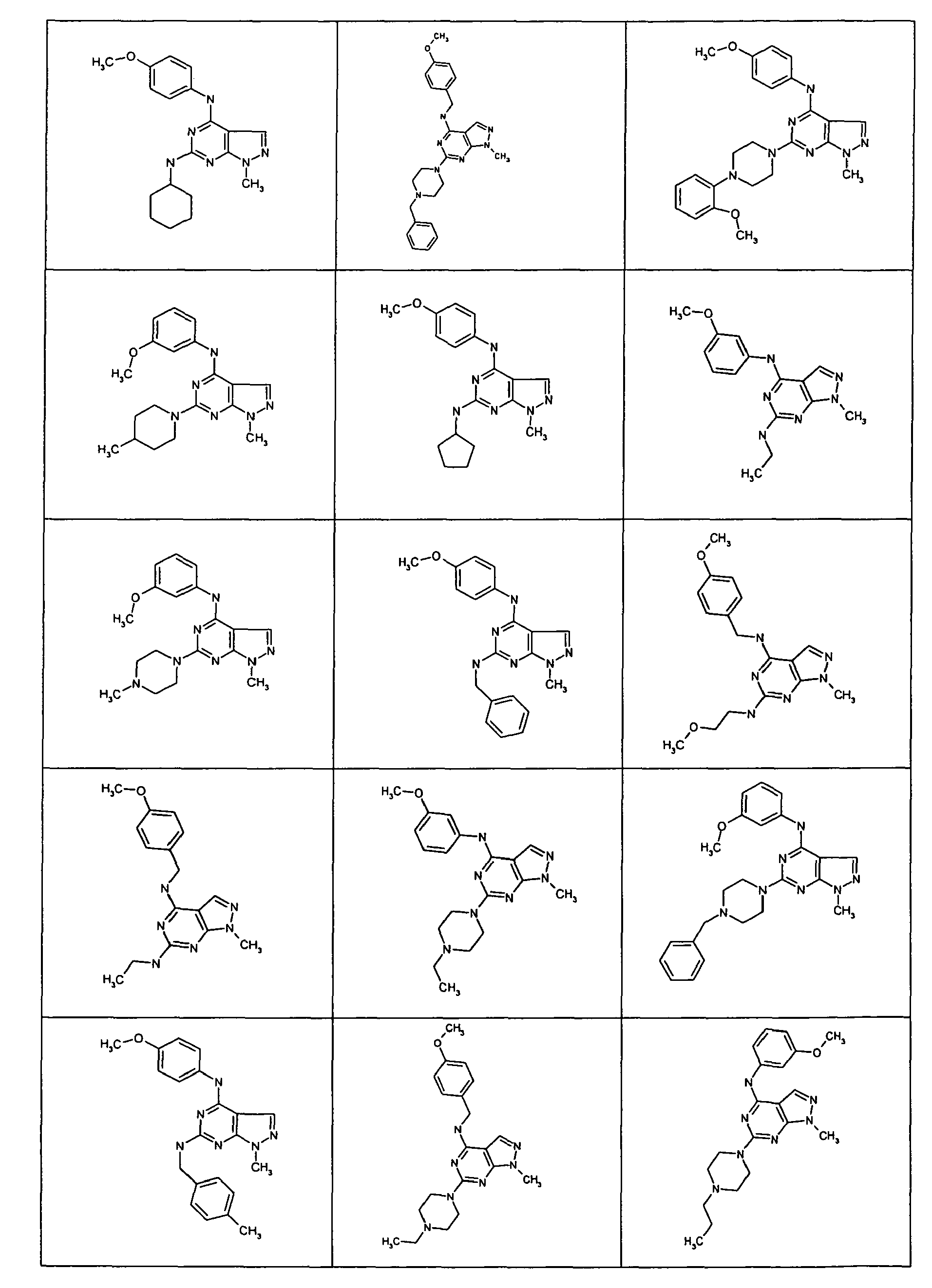

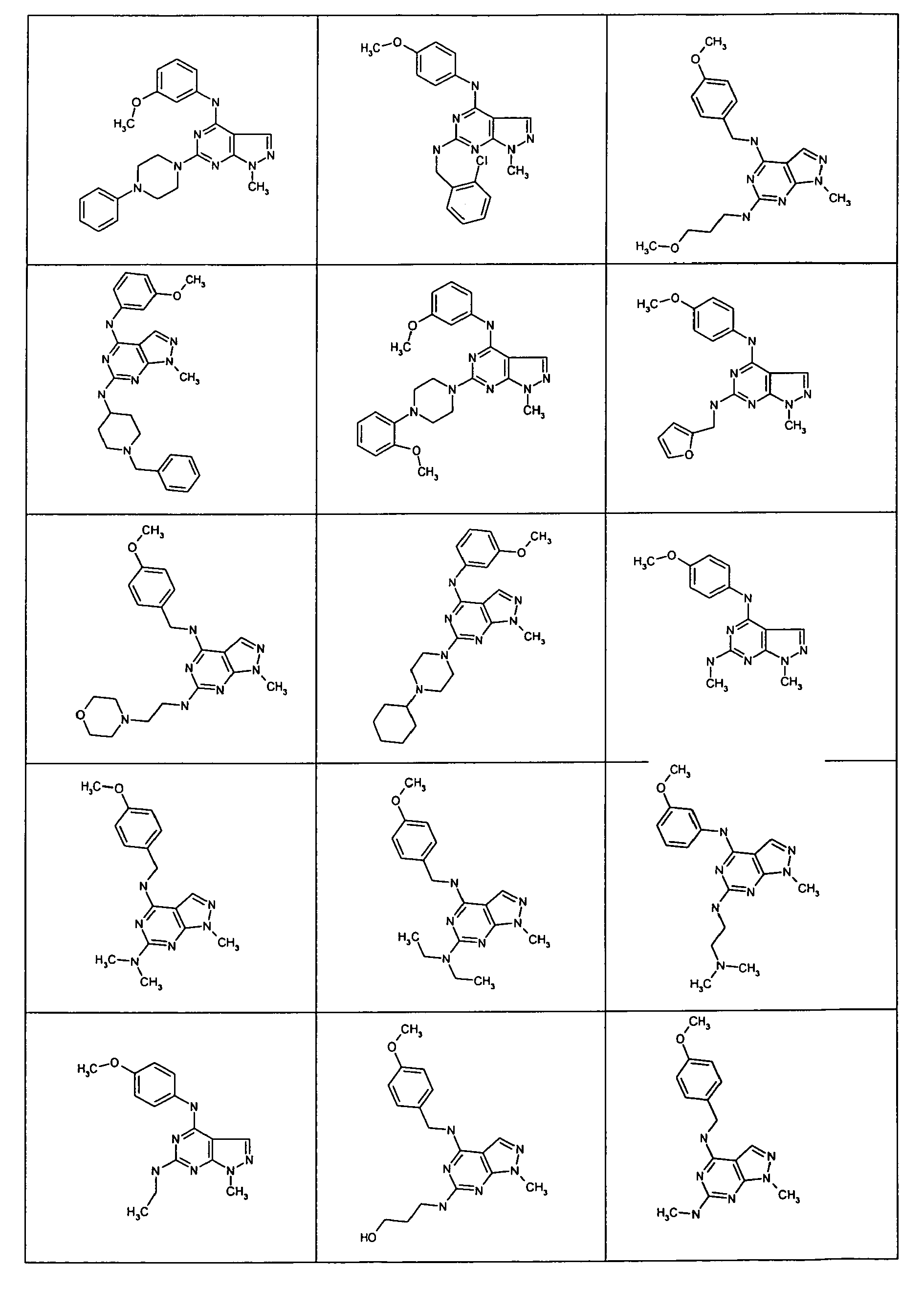

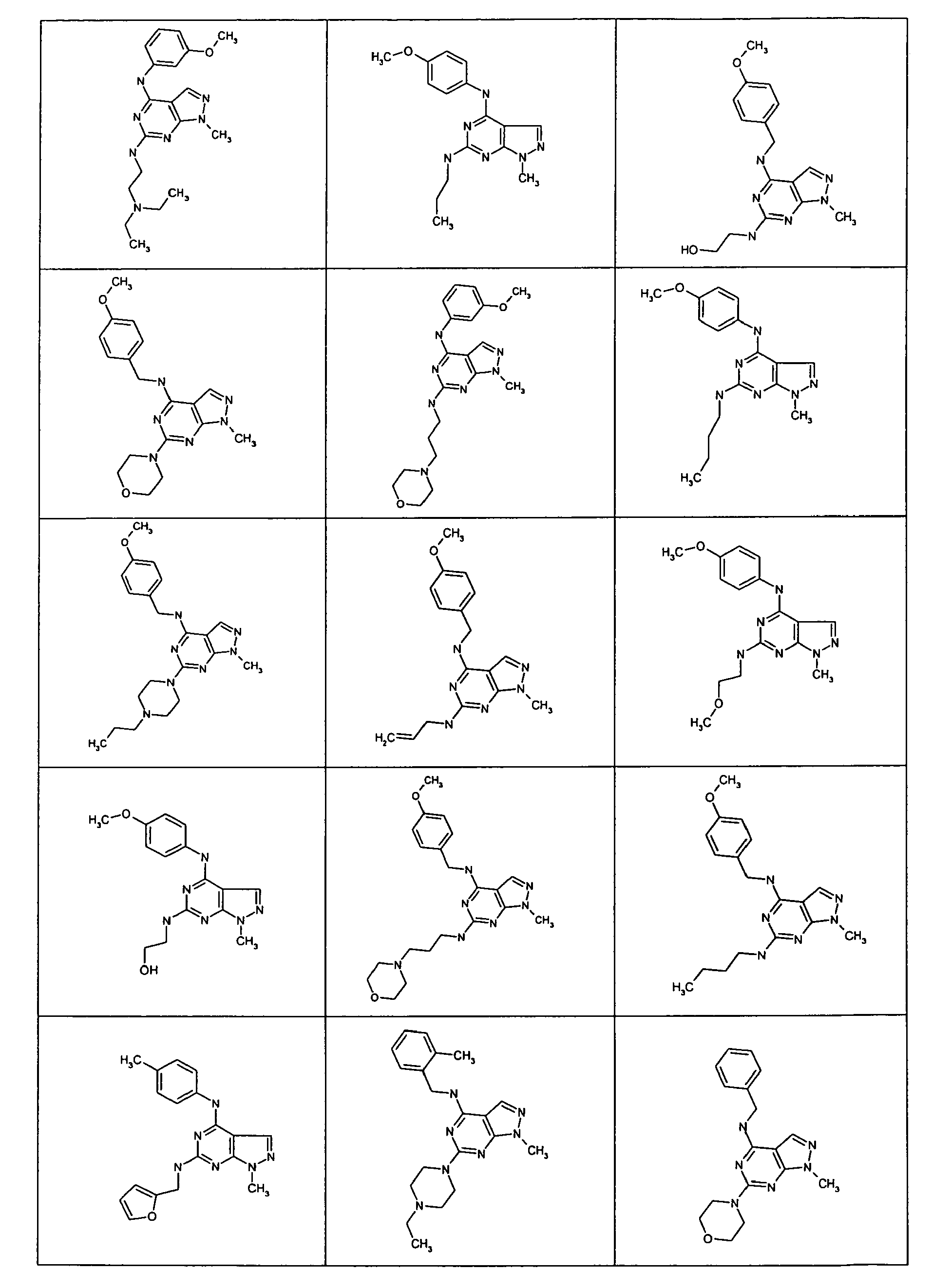

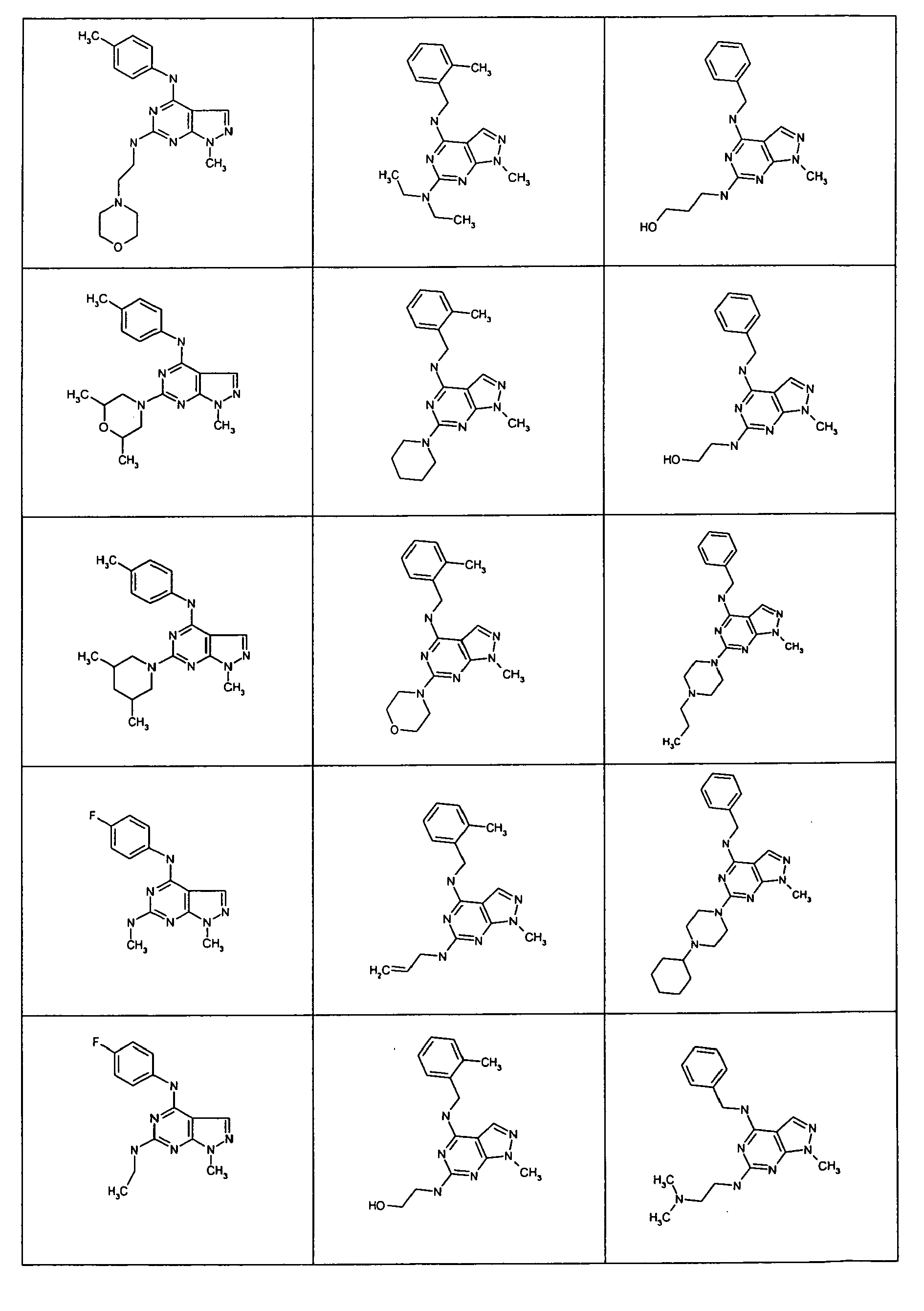

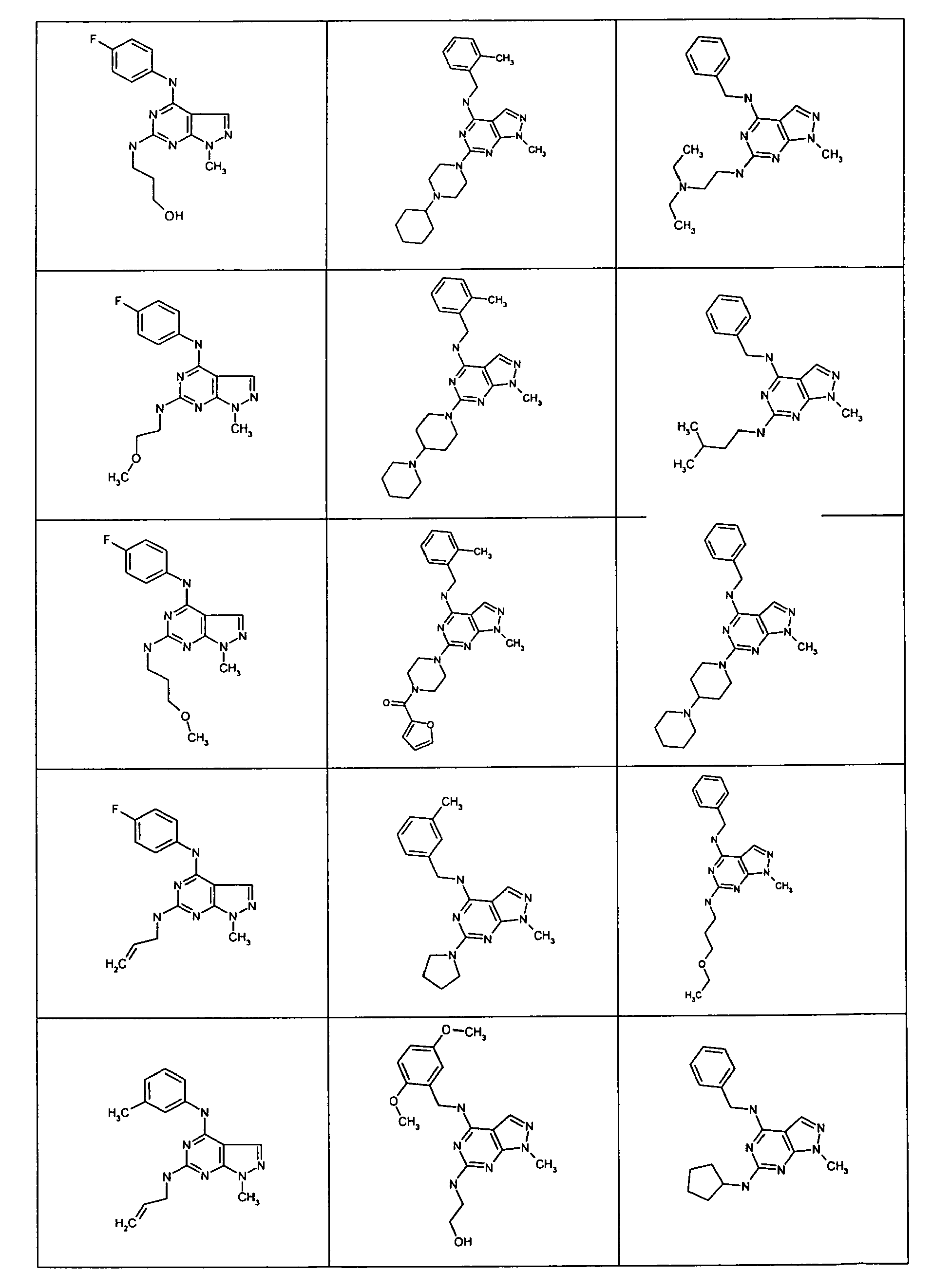

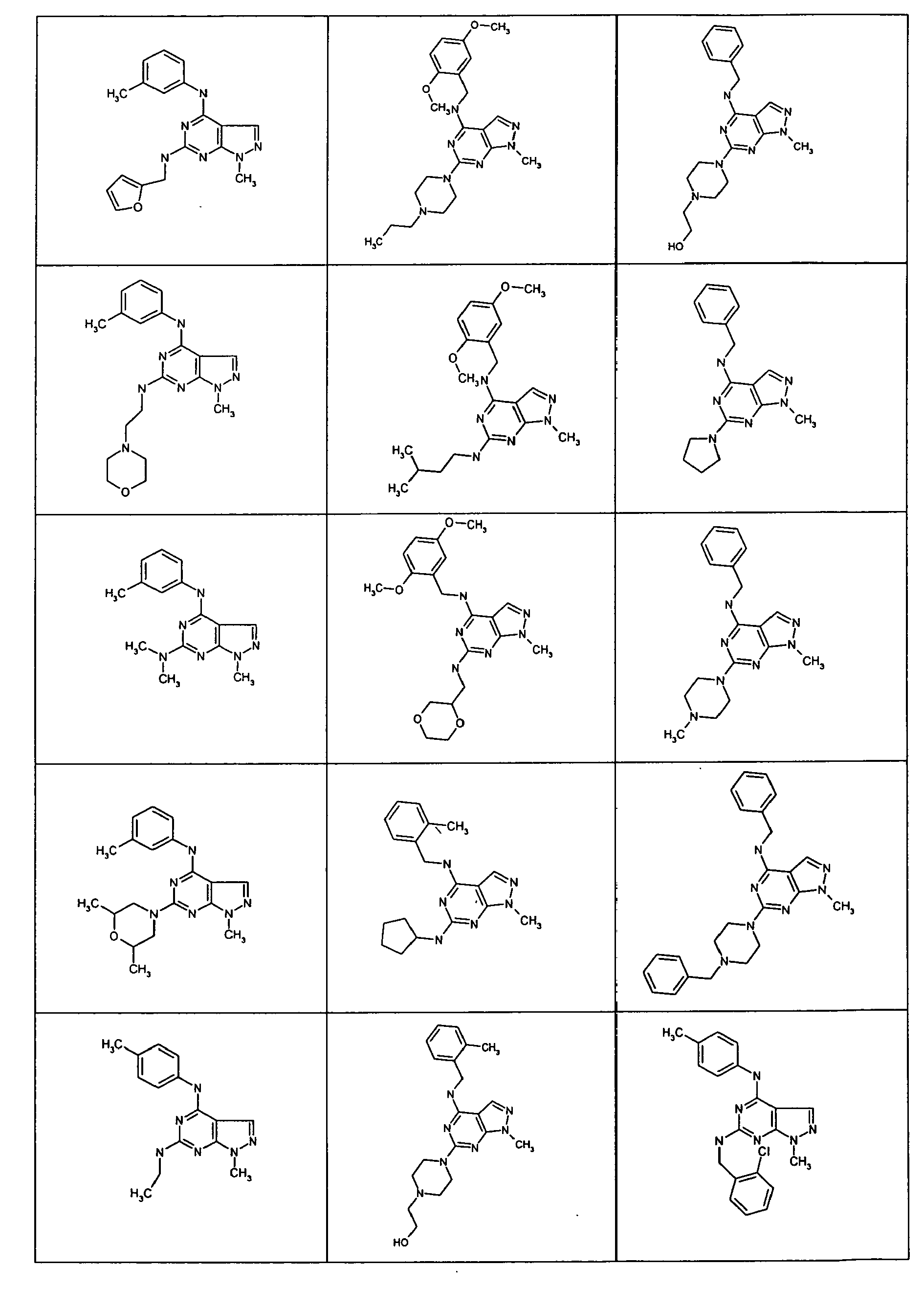

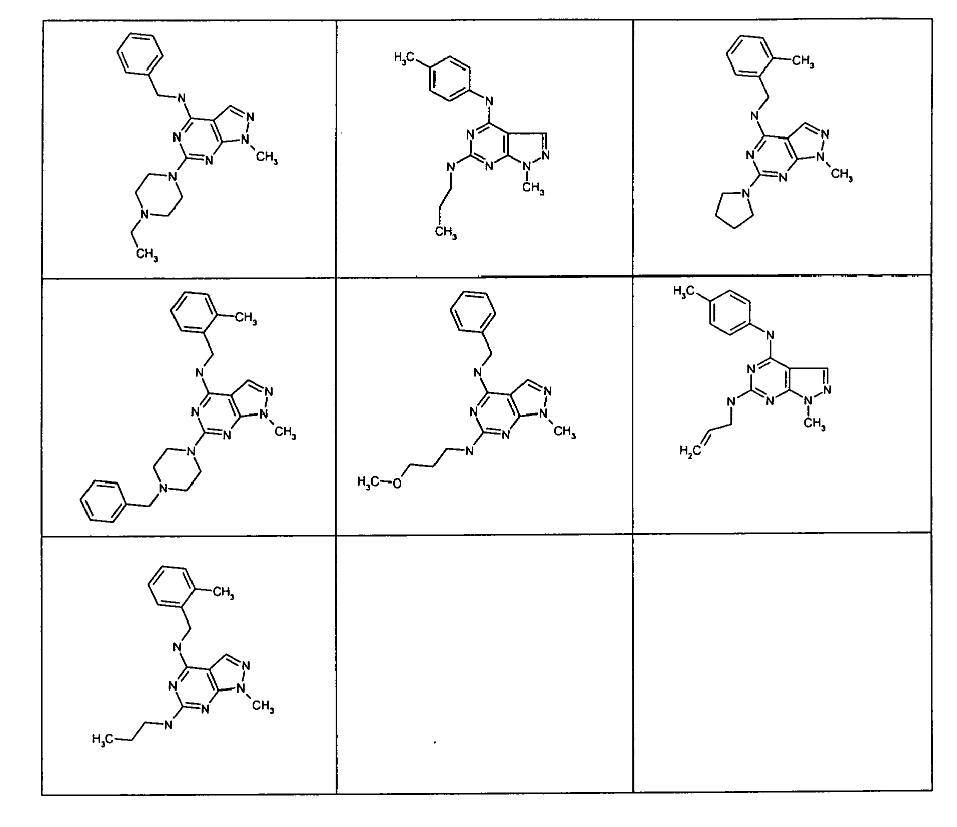

### yeast growth assay EphB4

The yeast growth assay was performed as described in "Method for the identification and/or validation of receptor tyrosine kinase inhibitors" WO2005/040413, section "Modes for Carrying out the Invention", Experiment 1 and Experiment 2: Yeast cells carrying plasmids with the RTK of interest were grown in glucose containing medium (repressing expression). Cultures were diluted into galactose containing medium, which induces expression of the kinase. Expression of the kinase inhibits growth of the yeast cells, which allows for positive selection of kinase inhibitors restoring growth of cells. Results are summarized in table X and Y.

### in vitro enzmatic assay EphB4

EphB4 inhibition activity of compounds was measured in an in vitro enzymatic kinase assay using the ADP Hunter assay kit (DiscoveRx Corp.), which measures accumulation of ADP produced by the kinase enzyme activity. EphB4 kinase (2ug/mL) is preincubated with different concentrations of compounds for 15 minutes at room temperature in kinase buffer (HEPES 15mM, MgCl₂ 10mM, EDTA 0.5mM, NaCl 20mM, Tween-20 0.02%, pH 7.4). ATP (300uM) is added and the mixture is incubated for 30 minutes at room temperature for enzyme activation. The peptide substrate poly (Glu4-Tyr) 800uM is added and the mixture is incubated for 60 minutes at 30°C. An enzyme- coupled reaction produces a red-shifted fluorescent signal that is directly proportional to the amount of ADP in solution. Fluorescence emission is measured at 590nm with excitation at 535nm.

### Cellular autophosphorylation assay EphB4 (ELISA)

CHO (chinese hamster ovary) cells were stably transfected with myc-tagged full length human EphB4. A cell line expressing EphB4 of which the phosphorylation was inducible with ephrinB2/Fc was identified and called CHO-EphB4. CHO-EphB4 cells were seeded into 96-well plates and cultured overnight in HAM-F12 medium supplemented with 10% fetal calf serum (FCS). Cells were washed once with PBS and different concentrations of compounds in HAM-F12 medium (final concentration of DMSO in the assay was 1%) were added. Cells were incubated at 37°C for 15 minutes. Ligand ephrinB2/Fc (R&D Systems, 1ug/mL final), which had been pre-clustered with anti-IgG Fc antibody (Jackson ImmunoResearch) for 45 minutes at room temperature, was added and cells were further incubated at 37°C for 45 minutes. Cells were lysed in 30uL lysis buffer per well (20mM Tris pH8, 150mM NaCl, 10% glycerol, 1% Triton X-100, 1mM Na₃VO₄, 1mM EDTA pH8, 1mM EGTA pH8, 1x protease inhibitor cocktail Sigma). ELISA 96-well plates (Nunc, Maxisorp) were coated with 100uL/well anti-c-myc antibody (Sigma) overnight at 4°C, blocked with 300uL/well 5% milk in TBS/0.005% Tween 20 (TBST) for 1 to 3 hours at room temperature, and incubated with 25uL/well cell lysate overnight at 4°C. ELISA plates were washed twice with TBST and incubated with 100uL/well antiphosphotyrosine HRP-coupled antibody (Upstate) diluted 1:1000 in 5% milk in TBST for 1.5 hour at room temperature and developed with 100uL/well BM BluePOD substrate (Roche). Absorbance was measured at 450nm.

**Table X Inhibition of EphB4 in different cellular assays as well as in cell-free enzymatic assays.**

| Structure | Yeast growth assay EphB4 | in vitro enzymatic assay EphB4 IC50 [uM] | ELISA EphB4 IC50 [uM] |
|---|---|---|---|
| 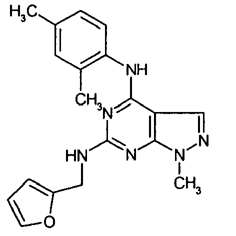 | + | ++ | n/a |
| 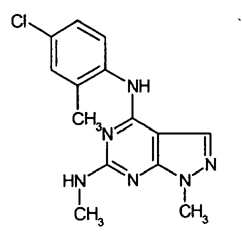 | + | ++ | + |
| 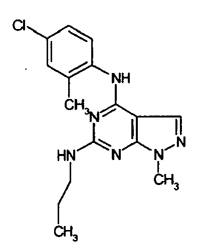 | + | ++ | n/a |
| 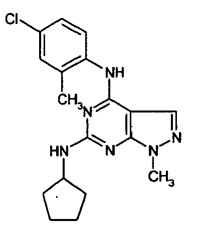 | (+) | ++ | ++ |
| 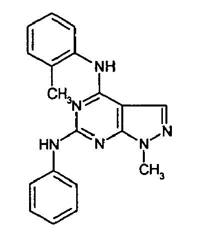 | + | ++ | n/a |
| 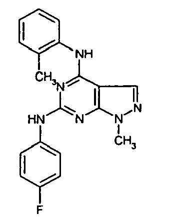 | + | ++ | n/a |
| 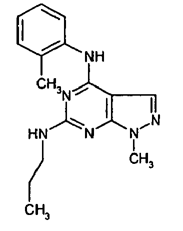 | + | +++ | n/a |
| 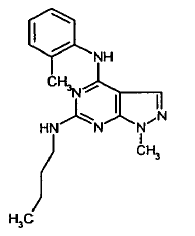 | + | +++ | +++ |
| 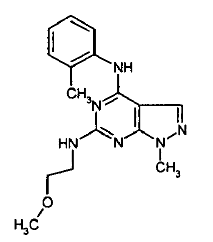 | + | +++ | n/a |
| 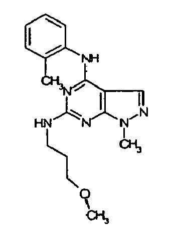 | ++ | +++ | n/a |
| 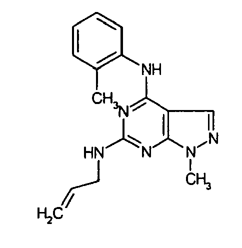 | + | +++ | n/a |
| 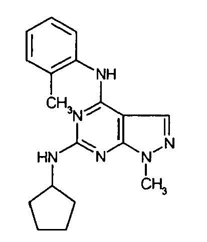 | + | +++ | n/a |
| 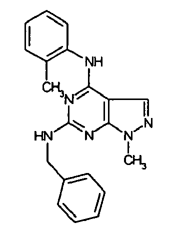 | + | ++ | n/a |
| 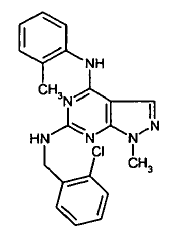 | + | ++ | +++ |
| 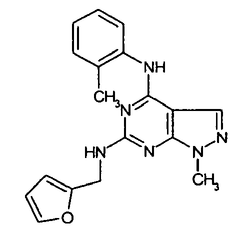 | ++ | +++ | ++ |
| 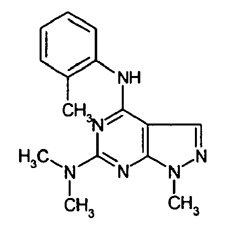 | ++ | ++ | n/a |
| 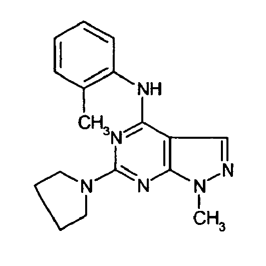 | ++ | ++ | n/a |
| 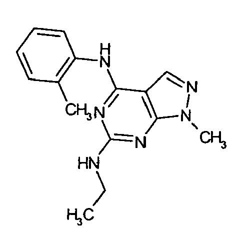 | ++ | +++ | +++ |
| 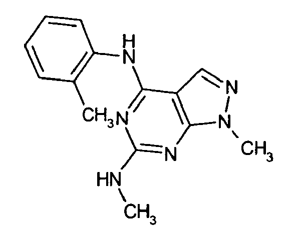 | ++ | +++ | n/a |
| 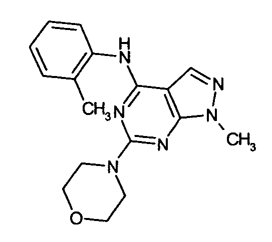 | + | ++ | n/a |
| 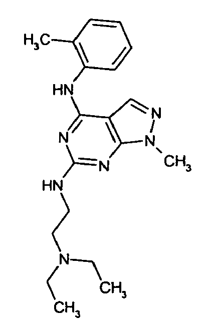 | + | +++ | n/a |
| 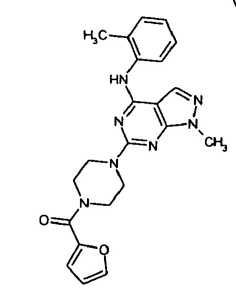 | + | ++ | n/a |
| 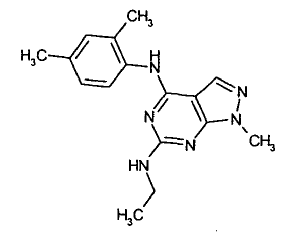 | + | ++ | n/a |
| 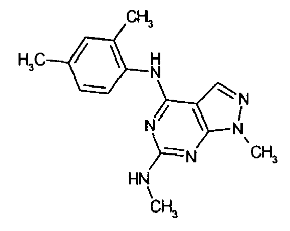 | + | ++ | n/a |

| | | | |
|---|---|---|---|
| Yeast growth assay EphB4: (+) <2% effect; '+ 2-10% effect; '++ 10-30% effect In vitro enzymatic EphB4 assay IC50 [uM]: + IC50 >10uM; '++ IC50 1-10uM; '+++ IC50 <1uM cellular autophosphorylation EphB4 assay IC50 [uM]: + IC50 >10uM; '++ IC50 1-10uM; '+++ IC50 <1uM; n/a not applied | | | |

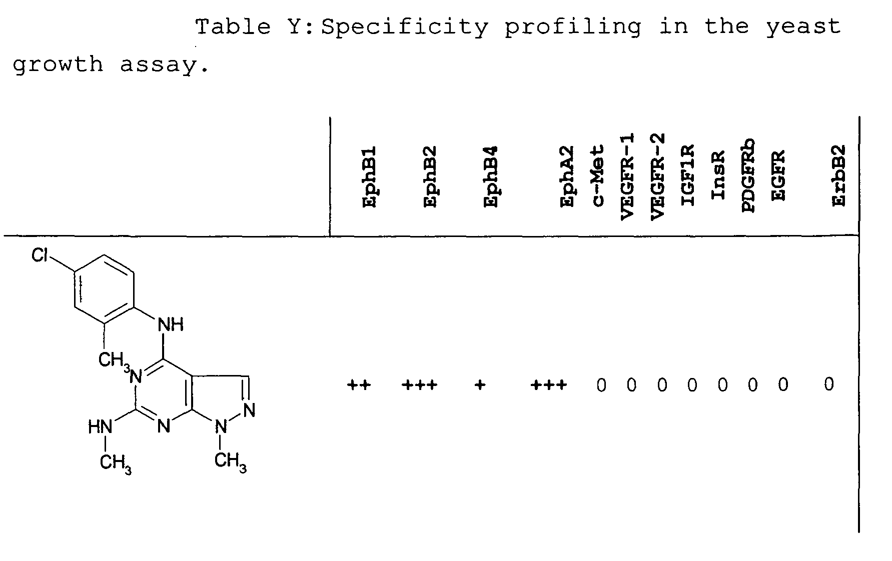

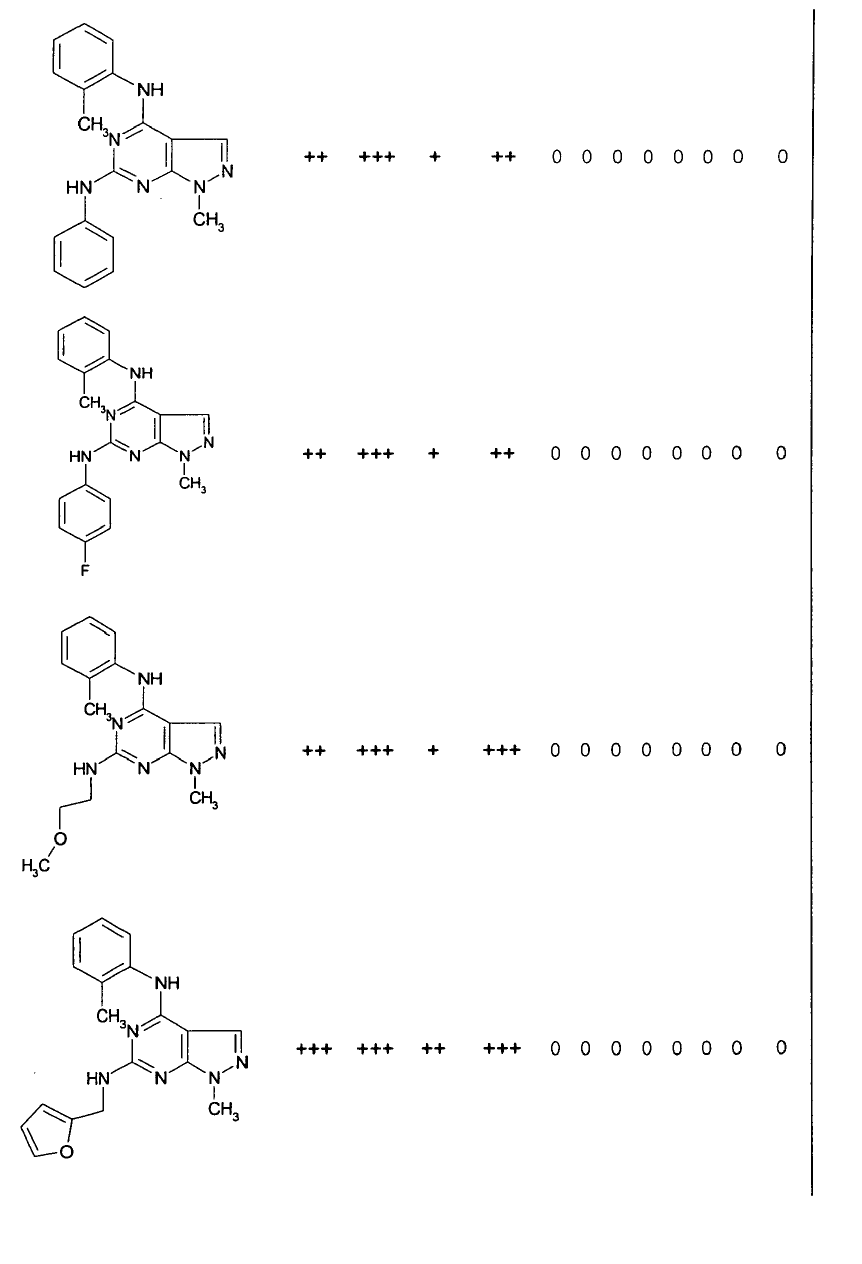

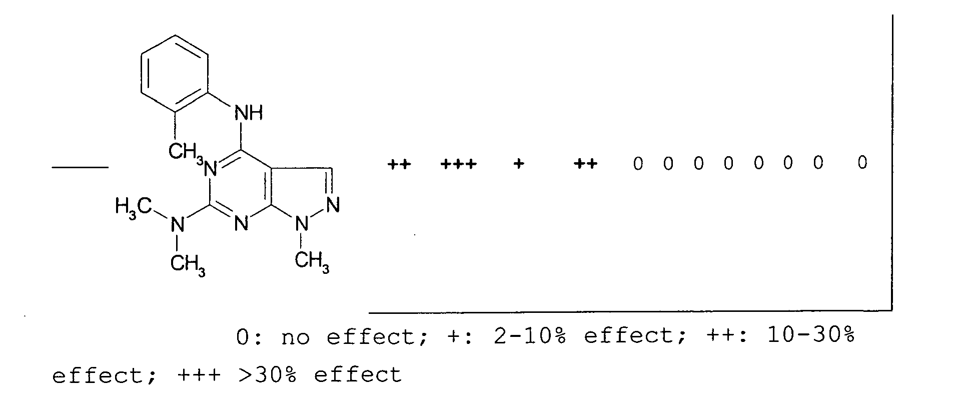

## Claims

1. A compound of the formula 1. 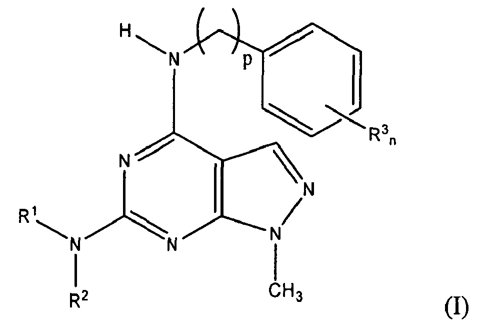 wherein
R1 represents unsubstituted or substituted alkyl, unsubstituted or substituted cycloalkyl, unsubstituted or substituted aryl, unsubstituted or substituted heterocyclyl and
R2 represents hydrogen or a substituent as defined for R1; or
R1 and R2 represents together with the nitrogen to which theay are bound unsubstituted or substituted heterocyclyl;
R3 represents unsubstituted or substituted lower alkyl, unsubstituted or substituted cycloalkyl, unsubstituted or substituted lower alkoxy, halo, cyano
n represents 0, 1, 2 or 3
p represents 0 or 1
or a (preferably pharmaceutically acceptable) salt thereof.

2. A compound according to claim 1 of the formula I, wherein
R1 and R2 together with the nitrogen to which they are bound represent unsubstituted or substituted monocyclic heterocyclyl having 5 to 7 ring atoms, the substituents being selected from the group consisting of unsubstituted or substituted lower alkyl, the substituents being selected from the group of hydroxy, halogen or aryl; unsubstituted or substituted cycloalkyl, the substituents being selected from the group of hydroxy, halogen. lower alkyl or aryl; unsubstituted heterocyclyl; substituted or unsubstituted heterocyclylcarbonyl, the substitutens being selected from the group of hydroxy, halogen, lower alkyl or aryl or
R1 represents unsubstituted or substituted lower alkyl, the substituents being selected from the group consisting of lower alkoxy, lower alkylamino, lower dialkylamino, halo, cyano, cylcoalkyl, unsubstituted heterocyclyl; unsubstituted or substituted cycloalkyl the substituents being selected from the group consisting of lower alkoxy, lower alkylamino, lower dialkylamino, halo, cyano,; unsubstituted or substituted phenyl, the substituents being selected from the group consisting of lower alkyl, lower alkoxy, lower alkylamino, lower dialkylamino, halo, cyano, cycloalkyl; unsubstituted lower alkenyl; unsubstituted or substituted heterocyclyl, the substituents being selected from the group consisting of lower alkoxy, lower alkylamino, lower dialkylamino, halo, cyano, cylcoalkyl, unsubstituted heterocyclyl and
R2 represents hydrogen.

3. A compound according to claim 1 or 2 of the formula I, wherein R3 represents unsubstituted lower alkyl, unsubstituted lower alkoxy, halo, cyano.

4. A compound of the formula I according to any of the preceeding claims, wherein n represents 1.

5. A compound of the formula I according to any of the preceeding claims, wherein at least one R3 is in the ortho-position.

6. A compound of the formula I according to any of the preceeding claims, wherein p represents 0.

7. A compound of the formula I according to claim 1, selected from the group of compounds consisting of N6-(furan-2-ylmethyl)-1-methyl-N4-o-tolyl-1H-pyrazolo[3,4-d]pyrimidine-4,6-diamine; N6-(2-methoxyethyl)-1-methyl-N4-o-tolyl-1H-pyrazolo[3,4-d]pyrimidine-4,6-diamine ; 1-methyl-N6-propyl-N4-o-tolyl-1H-pyrazolo[3,4-d]pyrimidine-4,6-diamine ; N6-butyl-1-methyl-N4-o-tolyl-1H-pyrazolo[3,4-d]pyrimidine-4,6-diamine ; N6,1-dimethyl-N4-o-tolyl-1H-pyrazolo[3,4-d]pyrimidine-4,6-diamine ; N6-ethyl-1-methyl-N4-o-tolyl-1H-pyrazolo[3,4-d]pyrimidine-4,6-diamine ; N6-(4-fluorophenyl)-1-methyl-N4-o-tolyl-1H-pyrazolo[3,4-d]pyrimidine-4,6-diamine ; N6-(2-chlorobenzyl)-1-methyl-N4-o-tolyl-1H-pyrazolo[3,4-d]pyrimidine-4,6-diamine ; in each case in free form or in salt form.

8. A pharmaceutical preparation comprising a compound as defined in any of claims 1 to 7, in free form or in pharmaceutically acceptable salt form, and a pharmaceutically acceptable carrier.

9. A compound as defined in any of claims 1 to 7 of the formula I, in free form or in pharmaceutically acceptable salt form, for use in the diagnostic or therapeutic treatment of the animal or human body.

10. A compound as defined in any of claims 1 to 7, in free form or in pharmaceutically acceptable salt form, for use in the treatment of a protein kinase modulation responsive disease.

11. The use of a compound as defined in any of claims 1 to 7, in free form or in pharmaceutically acceptable salt form, in the treatment of a protein kinase modulation responsive disease or for the manufacture of a pharmaceutical preparation useful in the treatment of a protein kinase modulation responsive disease.

12. The use according to claim 11, where the protein kinase modulation responsive disease is one or more diseases selected from the group consisting of diseases, that respond to the inhibition of one or more protein tyrosine kinases selected from abl kinase, especially v-abl or c-abl kinase, kinases from the family of the src kinases, especially c-src kinase, b-raf (V599E) and/or especially RET-receptor kinase or Ephrin receptor kinases, or mutated forms thereof.

13. The use according to claim 12 or claim 11, wherein the disease treated is one or more diseases selected from the group consisting of a proliferative disease, e.g. leukemia, especially chronic myelogenous leukemia (CML) or ALL, hyperplasia, fibrosis, such as cirrhosis of the liver, angiogenesis, psoriasis, atherosclerosis, especially arterial or post-transplantational atherosclerosis, smooth muscle proliferation in the blood vessels, such as stenosis or restenosis following angioplasty, tumor or cancer diseases, especially a benign or especially malignant tumor or cancer disease, more preferably solid tumors, e.g. carcinoma of the brain, kidney, liver, adrenal gland, bladder, breast, stomach, ovaries, colon, rectum, prostate, pancreas, lung, cervix, vagina, endometrium, thyroid, sarcoma, glioblastomas, multiple myeloma or gastrointestinal cancer, colorectal adenoma, melanoma, or a tumor of the neck and head, e.g. squameous carcinoma of the head and neck, mesangial cell-proliferative diseases, malignant pleural meso- therioma, lymphoma, multiple myeloma, neoplasias, especially of epithelial character, e.g. in the case of mammary carcinoma; an epidermal hyperproliferation (other than cancer), especially psoriasis; prostate hyperplasia; Kaposi' s sarcoma, thrombosis, scleroderma; a disease of the immune system; a disease of the central or peripheral nervous system where signal transmission by at least one protein (preferably tyrosine) kinase, especially selected from those protein tyrosine kinases mentioned as preferred, is involved, retinopathies, such as diabetic retinopathy, neovascular glaucoma or macula degeneration, obesity, haemangio- blastoma, haemangioma, diabetic nephropathy; malignant nephrosclerosis; inflammatory diseases, such as rheumatoid or rheumatic inflammatory diseases, especially arthritis, such as rheumatoid arthritis, other chronic inflammatory disorders, such as chronic asthma, endometriosis, Crohn' s disease; Hodgkin' s disease; glomerulonephritis; inflammatory bowel disease; thrombotic microangiopathic syndromes; transplant rejections, glomerulopathy; injuries of the nerve tissue; wound healing, age spots, contact dermatitis; restenosis, e.g. stent-induced restenosis; and protein kinase, such as Eph receptor kinase, modulation responsive conditions, diseases or disorders, where the stimulation or the promotion of neural regeneration (neuronal regeneration; neuroregeneration), such as axon regeneration, or the inhibition or the reversal of neural degeneration (neuronal degeneration; neurodegeneration) is desired, e. g. spinal cord injury, hypoxic conditions, traumatic brain injury, infarct, stroke, multiple sclerosis or other neurodegenerative conditions, diseases or disorders.

14. A method for the treatment of a protein kinase modulation, especially inhibition, responsive disease, especially one or more diseases named in claim 13, comprising administering an effective amount of a compound as defined in any of claims 1 to 7, in free form or in pharmaceutically acceptable salt form, to an animal or human in need of such treatment.

15. A process for the manufacture of a compound as defined in any of claims 1 to 7, in free form or in salt form, comprising the steps of
a) reacting a compound of the formula II 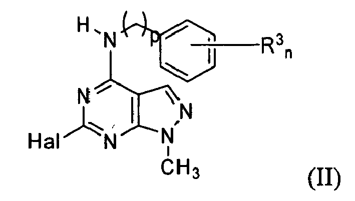 wherein R3, n, p are as defined for a compound of the formula I according to claim 1, Hal is halo, especially bromo or chloro, with a compound of the formula (III)
(R¹)(R²)NH (III)
wherein R1 and R2 are as defined for a compound of the formula I according to claim 1;
b) optionally removing any protecting groups present;
c) optionally transforming a compound of the formula I into a different compound of the formula I, transforming a salt of a compound of the formula I into the free compound or a different salt, transforming a free compound of the formula I into a salt thereof, and/or
d) optionally separating a mixture of isomers of a compound of the formula I into the individual isomers.
